# EUROPEAN PATENT SPECIFICATION

(11) **EP 2 694 646 B1**
(45) Date of publication and mention of the grant of the patent: **20.07.2016**
(21) Application number: 12714300.6
(22) Date of filing: 05.04.2012
(51) Int. Cl.: C12N 9/00, C12N 9/04, C12P 7/16

(54) **PRODUCTION OF BUTANOL BY FERMENTATION**
HERSTELLUNG VON BUTANOL DURCH FERMENTATION
PRODUCTION DE BUTAN-1-OL PAR FERMENTATION

(30) Priority: 05.04.2011 EP 11161243
(43) Date of publication of application: 12.02.2014
(73) Proprietor: Leibniz-Institut für Pflanzengenetik und Kulturpflanzenforschung, 06466 Gatersleben (DE)
(72) Inventor: KUNZE, Gotthard, 06466 Gatersleben (DE); HÄHNEL, Urs, 06484 Quedlinburg (DE); RIECHEN, Jan Hendrik, 38820 Halberstadt (DE)
(74) Representative: Taruttis, Stefan Georg
(86) International application number: PCT/EP2012/056380
(87) International publication number: WO 2012/136826

(56) References cited:
- EP-A1- 1 918 379
- WO-A1-2008/097064
- WO-A1-2008/121701
- WO-A1-2011/019894
- WO-A2-2008/006038
- WO-A2-2008/143704
- US-A1- 2010 167 365

## Description

The present invention relates to a genetically manipulated yeast which is suitable for use in the production of n-butanol by fermentation of an organic substrate, and to a process for producing n-butanol using the genetically manipulated yeast in a fermentation process, which is preferably semi-anaerobic or anaerobic. As a substrate, biologic waste, e.g. excrements from animals, household sewage, waste containing protein, fats, alkanes, especially n-alkanes, alcohols, especially ethanol, starch, lignocellulose and/or tannines, preferably in an aqueous composition can be used. Preferably, the substrate contains sugar, e.g. a pentose and/or a hexose sugar which is comprised of monomeric, oligomeric or polymeric sugar or sugar alcohol moieties, e.g. starch, and/or alcohol, e.g. ethanol.

### State of the art

US 2007/0092957 A1 describes micro-organisms, especially bacteria which are genetically manipulated to contain an acetolactate synthase, catalyzing the conversion of pyruvate to acetolactate, an acetohydroxy acid isomerase reductase for conversion of acetolactate to 2,3-dihydroxy isovalerate, an acetohydroxy acid dehydratase for conversion of 2,3-dihydroxy isovalerate to α-ketoisovalerate, a branched chain - ketoacid decarboxylase for conversion of α-ketoisovalerate to isobutyraldehyde, and a branched chain - alcohol dehydrogenase for conversion of isobutyraldehyde to isobutanol.

Ezeji et al in Current Opinion in Biotechnology 220 - 227 (2007) describe the fermentation of butanol and ethanol by Clostridium acetobutylicum, including the genetic manipulation of the bacterium by overexpression of alcohol / aldehyde - dehydrogenase and downregulation of CoA-transferase, both resulting in a decrease in butanol production.

WO 2008/097064 A1 describes Saccharomyces cerevisiae containing the Coenzyme A - transferase encoding gene of Clostridium for butanol production.

WO 2009/103533 A1 describes the genetic manipulation of Saccharomyces cerevisiae by overexpression of enzymes catalyzing the conversion steps from pyruvate to acetolactate, to 2,3-dihydroxyisovalerate, to 2-ketoisovalerate, to isobutyraldehyde, for the production of isobutanol.

WO 2009/013158 describes Saccharomyces cerevisiae for producing butanol which is genetically manipulated to contain a heterologous gene encoding an enzyme for conversion of acetyl-CoA to acetoacetyl-CoA, e.g. an acetyl-CoA acetyl transferase or thiolase, an enzyme converting acetoacetyl-CoA to 3-hydroxy butyryl-CoA, e.g. 3-hydroxy butyryl-CoA dehydrogenase, an enzyme converting 3-hydroxy butyryl-CoA to crotonyl-CoA, e.g. 3-hydroxy butyryl-CoA dehydratase, an enzyme converting crotonyl-CoA to butyryl-CoA, e.g. butyryl-CoA dehydrogenase, an enzyme converting butyryl-CoA to butyraldehyde, e.g. alcohol / aldehyde dehydrogenase, and an enzyme for converting butyraldehyde to n-butanol, e.g. an NAD(P)H - dependent butanol dehydrogenase, e.g. obtained from Clostridium.

WO 2011/019894 A1 describes recombinant cytosolic dihydroxyacid dehydratase enzymes, converting 2,3-dihydroxyisovalerate to ketoisovalerate, for use in microbial synthesis of isobutanol.

WO 2008/143704 A2 for the production of n-butanol describes the inactivation of native pathways for conversion of pyruvate or acetyl-CoA, especially in Clostridium.

### Objects of the invention

It is an object of the invention to provide for an alternative non-conventional yeast with high thermo- and osmotolerance, wide substrate spectrum and excellent growth behaviour which is capable of producing butanol by fermentation, especially at higher concentrations and/or with a lower concentration of by-products than prior art micro-organisms, and to provide a production process for n-butanol using the yeast.

### General description of the invention

The invention achieves these objects by providing a non-conventional, non-pathogenic yeast, e.g. of the genus Hansenula, e.g. H. anomala, of the genus Pichia, e.g. Pichia stipidis, of the genus Kluyveromyces, Yarrowia, Trichosporon or Brettanomyces, especially a yeast of the genus Arxula, especially Arxula adeninivorans (syn. Blastobotrys adeninivorans), and a process using the yeast, for producing n-butanol according to the claims, which yeast has been genetically manipulated to contain a set of genes comprising or consisting of the genes encoding the pathway enzymes for the conversion of acetyl-CoA to butyryl-CoA from a heterologous micro-organism, especially from Clostridium, preferably from Clostridium acetobutylicum, e.g. genes encoding an acetyl-CoA-acetyl transferase (also termed β-ketothiolase, preferably E.C. 2.3.1.9), a β-hydroxybutyryl-CoA dehydrogenase (e.g. E.C. 1.1.1.35), a crotonase (e.g. E.C. 4.2.1.17), and a butyryl-CoA dehydrogenase (e.g. E.C. 1.3.8.1, and optionally a butyryl aldehyde dehydrogenase (also termed butyraldehyde dehydrogenase).

In embodiments in which the yeast of the invention is e.g. of the genus Arxula, especially Arxula adeninivorans, the heterologous genes introduced into the yeast can consist of genes encoding the pathway enzymes for the conversion of acetyl-CoA to butyryl-CoA. The reason for the optional genetic manipulation of the yeast by introduction of heterologous genes consisting of those genes encoding the pathway enzymes for the conversion of acetyl-CoA to butyryl-CoA is, as has been discovered during the preparation of the present invention that Arxula contains an endogenous butyraldehyde dehydrogenase and an endogenous butanol dehydrogenase, which catalyzes the conversion of butyryl-CoA to n-butanol.

The yeast of the invention, and the process of the invention using the yeast are characterized in that the yeast in addition to being genetically manipulated by introduction of heterologous genes encoding the metabolic pathway enzymes for conversion of acetyl-CoA to butyryl-CoA, the yeast's metabolic pathway to or of the peroxisomal β-oxidation is impaired, preferably inactivated. The metabolic pathway in the yeast to or of the peroxisomal β-oxidation is impaired, by the yeast being genetically manipulated by impairing or inactivating, e.g. mutation or deletion of the genes encoding at least one of the metabolic pathway enzymes according to claim 1 of the metabolic pathway enzymes catalyzing the metabolism towards or from or within β-oxidation. This impairment or inactivation of the metabolic pathway to β-oxidation and/or impairment or inactivation of β-oxidation, e.g. the yeast can be genetically manipulated by impairment and/or inactivation of the gene encoding the peroxisomal acyl-carnitine permease.

Further alternatively or additionally, the yeast can be genetically manipulated by impairment and/or inactivation of the gene encoding the peroxisomal acyl-carnitine transferase which catalyzes the transfer of the butyryl moiety from butyryl-carnitine to peroxisomal butyryl-CoA.

Further, additionally or alternatively, the yeast can be genetically manipulated by impairment and/or inactivation of the gene encoding the peroxisomal acyl-CoA-oxidase which catalyzes the oxidation of butyryl-CoA. The oxidation of butyryl-CoA within the peroxisome is currently regarded as the metabolic step towards β-oxidation of butyryl-CoA, and therefore impairment or inactivation of at least one of the genes encoding an enzyme that participates in the pathway from cytosolic butyryl-CoA towards β-oxidation is regarded as the cause for the increase in n-butanol yield in the fermentation.

Additionally, the yeast of the invention can be genetically manipulated for impairment or interruption of the pathway of glycerol synthesis, e.g. by impairing or inactivating at least one of the genes encoding an enzyme that participates in the synthesis of glycerol from dihydroxyacetone-3-phosphate, e.g. by impairing, preferably by inactivating the glycerol-3-phosphate dehydrogenase gene.

Generally, impairment or inactivation of a metabolic enzyme can be by impairment or inactivation of the gene encoding the enzyme, preferably by mutation of the gene, e.g. interruption, e.g. by insertion of one or more nucleotides, or partial or complete deletion of the gene encoding the enzyme.

The genetically manipulated yeast of the invention has the advantage of being tolerant to n-butanol, allowing comparatively high concentrations of n-butanol being produced in the fermentation medium. Further, the impairment, i.e. a reduction in flux of the metabolic pathway from cytosolic butyryl-CoA to butyryl carnitine, and further the impairment of the metabolic flux to peroxisomal butyryl carnitine, butyryl-CoA followed by β-oxidation, i.e. from butyryl-CoA to acetyl-CoA, increases the flux from butyryl-CoA originating from crotonyl-CoA towards butyraldehyde, respectively, and results in an increase in n-butanol production.

For impairment or inactivation of the transfer of butyryl-CoA or of butyryl-carnitine, respectively, to the peroxisome, it is preferred to inactivate or to delete the gene encoding peroxisomal acyl-carnitine transferase, and/or to inactivate or to delete the gene encoding the peroxisomal acyl-carnitine permease specific for acyl-carnitine and butyryl-carnitine, and/or to inactivate or to delete the gene encoding the peroxisomal acyl-carnitine transferase, e.g. the peroxisomal butyryl-carnitine transferase catalyzing the reaction from butyryl-carnitine to butyryl-CoA.

For impairment or inactivation of β-oxidation, it is preferred to inactivate the gene encoding the acyl-CoA oxidase. Generally, inactivation of an endogenous metabolic enzyme is by inactivation of the endogenous gene encoding the metabolic enzyme, preferably by mutation of the gene, which mutation is e.g. an insertion of at least one nucleotide into the gene, preferably the at least partial or the complete deletion of the gene.

For the optional additional impairment or inactivation of the conversion of dihydroxyaceton-3-phosphate to glycerol, it is preferred to delete the gene encoding glycerol-3-phosphate dehydrogenase. Generally, CoA designates coenzyme A.

The genetic manipulation of the yeast can be achieved by impairment or inactivation, preferably interruption of the yeast's metabolic pathway to peroxisomal ß-oxidation or of the peroxisomal ß-oxidation pathway and results in an increased yield of n-butanol production. This genetic manipulation can e.g. be a mutation which inactivates gene function, e.g. a mutation, preferably a deletion or another inactivation of at least one gene of this metabolic pathway.

Optionally additionally, at least one of the genes encoding metabolic pathway enzymes catalyzing the metabolism from acetyl-CoA to the citric acid cycle or to the glyoxylate cycle, respectively, is impaired or inactivated, e.g. by impairment or inactivation of the endogenous yeast gene encoding the isocitrate lyase (e.g. E.C.4.1.3.1).

Optinally additionally, the genes encoding metabolic pathway enzymes catalyzing the metabolism from pyruvate to acetaldehyde are impaired or inactivated, e.g. by impairment or inactivation of the endogenous yeast gene encoding the pyruvate decarboxylase (e.g. E.C. 4.1.1.1).

Preferably additionally, the genes encoding metabolic pathway enzymes catalyzing the conversion of acetyl-CoA to acetate and CoA are impaired or inactivated, e.g. by impairment or inactivation of the endogenous yeast genes encoding an acetyl-CoA hydrolase, e.g. E.C. 3.1.2.1 (optionally also having an acetyl-CoA transferase activity, E.C. 2.8.3). Preferably, both the genes encoding the mitochondrial acetyl-CoA hydrolase and the cytosolic acetyl-CoA hydrolase are impaired or inactivated. For example, it has been found that Arxula contains a mitochondrial acetyl-CoA hydrolase (AACH1) and a cytosolic acetyl-CoA hydrolase (AACH2). A preferred cytosolic acetyl-CoA hydrolase gene that is inactivated or impaired is the gene encoding the succinyl-:3 ketoacid-CoA transferase catalyzing the transfer of CoA from succinyl-CoA to acetoacetate.

In a preferred embodiment, the yeast is Arxula, and the genetic manipulation, e.g. introduction of the metabolic pathway enzymes catalyzing the conversion of acetyl-CoA to n-butanol, consists of the introduction of the genes encoding the pathway enzymes catalyzing the conversion of acetyl-CoA to butyryl-CoA, i.e. of the genes encoding an acetyl-CoA-acetyl transferase, a β-hydroxybutyryl-CoA dehydrogenase, a crotonase and a butyryl-CoA dehydrogenase from Clostridium, preferably additionally a butyryl aldehyde dehydrogenase from Clostridium. The introduction of genes encoding these enzymes has the advantage of utilising the endogenous pathway enzymes catalyzing the conversion of butyryl-CoA to n-butanol, i.e. the butyraldehyde dehydrogenase and butanol-dehydrogenase encoded by Arxula. As the endogenous butyryl aldehyde dehydrogenase from yeast, especially from Arxula, can have a comparatively low activity, it is preferred that the yeast additionally contains a heterologous gene encoding a butyryl aldehyde dehydrogenase, e.g. from Clostridium, preferably the gene encoding the AdhE2p.

Optionally, in a yeast of the invention, both the heterologous genes introduced into the yeast and, if applicable, the endogenous gene encoding butyraldehyde dehydrogenase and the endogenous gene encoding butanol dehydrogenase have a promoter with increased activity, e.g. a constitutive promoter with increased activity. Preferably, both the heterologous genes introduced into the yeast and, if applicable, the endogenous gene encoding butyraldehyde dehydrogenase and the endogenous gene encoding butanol dehydrogenase have a promoter with increased activity, preferably an inducible promoter, e.g. a nitrate and/or nitrite inducible promoter. A preferred promoter, which is inducible by nitrate and/or nitrite is selected from the group comprising the promoters of *AYNR1, AYNR1', AYNI1, AYNI1'* and *AYNT1, AYNT1',* which originate from the nitrate reductase gene *(AYNR1),* the nitrite reductase gene *(AYNI1),* and from the nitrate transporter gene *(AYNT1)* of Arxula adeninivorans, as well as truncated promoters of these. Alternatively, the promoter can be any promoter active in the yeast, preferably a high activity promoter, constitutive or inducible, e.g. the TEF promoter (promoter of the elongation factor 1α gene).

Preferably, the yeast contains an additional gene encoding a butyryl-CoA dehydrogenase, e.g. the butyryl-CoA dehydrogenase gene of Clostridium acetobutylicum, preferably a butyryl-CoA dehydrogenase which is only dependent on NAD(P)H, e.g. the butyryl-CoA dehydrogenase of Treponema denticola, also termed trans-enoyl-CoA-reductase (TER), preferably a butyryl-CoA dehydrogenase gene that is codon-optimized for the yeast. The butyryl-CoA dehydrogenase from Clostridium is dependent on cofactors ETF A and B (ETF = electron transfer flavoprotein, subunits A and B) including ferredoxin, whereas the butyryl-CoA dehydrogenase (TER, e.g. nucleic acid sequence of SEQ ID NO: 19, encoding the amino acid sequence of SEQ ID NO: 20) of Treponema denticola has the advantage of being independent of ferredoxin, the reaction only requiring NADH as the cofactor.

Preferably, the acetyl-CoA-acetyltransferase gene (THL) is the homologous yeast gene, e.g. yeast does not contain the acetyl-CoA-acetyltransferase gene from Clostridium, which e.g. is the β-ketothiolase gene. More preferably, the acetyl-CoA-acetyltransferase gene is an optimized variant, preferably a codon-optimized variant, of the yeast acetyl-CoA-acetyltransferase gene (nucleic acid sequence of SEQ ID NO: 21, encoding the amino acid sequence of SEQ ID NO: 22), e.g. derived by evolution-optimization from the endogenous yeast acetyl-CoA-acetyltransferase gene.

Generally preferred, the yeast genes which are impaired or inactivated encode an enzyme comprising or having an amino acid sequence having a homology of at least 90%, preferably of at least 95%, more preferred of at least 98%, most preferred of at least 99% to 100% to the amino acid sequence encoded by the Arxula gene or of the gene specified herein. Alternatively, the yeast genes which are impaired or inactivated have a nucleic acid sequence having a homology of at least 80% or of at least 90%, preferably of at least 95%, more preferred of at least 98%, most preferred of at least 99% to 100% to the nucleic acid sequence of the Arxula gene or of the gene specified herein. For impairment or inactivation of the yeast gene, the yeast can contain, e.g. following transformation, a nucleic acid construct containing a section, e.g. a mutated, e.g. a truncated coding and/or non-coding gene section, e.g. of 10 to 90%, of 20 to 80% of the original gene sequence, between a 5' flanking region (FR5) and a 3' flanking region (3FR), both having homology to the genomic sequences adjacent or within the gene to be inactivated. Optionally, the mutated gene section can be arranged between the FR5 and the FR3 with an additional selection marker, e.g. positioned within the mutated gene section or in 5' or 3' to the mutated gene section. In this embodiment, the yeast having an impaired or inactivated gene can contain a nucleic acid construct having a mutated, e.g. a truncated coding and/or non-coding gene section, e.g. of 10 to 90%, of 20 to 80% of the original gene sequence and a selection marker gene, between a 5' flanking region (FR5) and a 3' flanking region (3FR), with both flanking regions having homology to the genomic sequences adjacent or within the gene to be inactivated. Further optionally, the nucleic acid construct can comprise a section of the gene to be impaired or inactivated, e.g. a section of 10 to 90%, of 20 to 80% of the original gene sequence, in 5' to the FR5 and/or in 3' to the FR3. Accordingly, the nucleic acid constructs can be used in a process in the production of a yeast of the invention, e.g. for transformation of the yeast by the nucleic acid construct. For the purposes of the disclosure, a mutated gene section also includes a reverse complementary nucleic acid sequence.

The yeast genes to be inactivated or impaired can be selected from the following genes, which in Arxula, using amplified sections of genomic DNA of Arxula that in addition to the gene comprise flanking regions (FR) in the construction of nucleic acid constructs comprising between the flanking regions a selection gene, which selection gene preferably is a gene complementing an auxotrophy of the yeast. In these constructs, the yeast gene to be inactivated or impaired is at least partially inactivated, e.g. mutated, preferably at least partially deleted. Following transformation of the yeast with a construct used for inactivating the metabolic yeast gene, the construct by recombination of the flanking regions integrates into the yeast genome, replacing the metabolic yeast gene by the construct. For integration, homologous recombination, e.g. two crossing-over events, can be used as presently exemplified by the flanking regions of genomic yeast origin. Preferably, homologous, preferably identical flanking regions are used, e.g. of 0.5 to 2 kb, preferably of approx. 1 kb length. Accordingly, a yeast of the invention can contain a nucleic acid construct which replaces the genomic gene copy of the metabolic gene to be inactivated, e.g. by the selection gene, leaving an at least partially interrupted or at least partially deleted metabolic yeast gene. Accordingly, a yeast of the invention preferably is devoid of a functional gene of at least one of the metabolic genes mentioned herein, reducing or deleting the metabolic flow to and from the respective metabolic pathway. Preferred metabolic genes, amplified genomic sections and exemplary constructs are given on the example of Arxula adeninivorans. Using enzyme homology, the skilled person is capable of identifying the respective gene sequences in other yeasts, using conventional cloning procedures, e.g. based on hybridization of the coding nucleic acid sequences or on the basis of amino acid homology to the encoded amino acid sequence of the metabolic gene to be inactivated:
- The pyruvate decarboxylase 1 gene (APDC1) having the nucleic acid sequence of SEQ ID NO: 23, encoding its amino acid sequence of SEQ ID NO: 24,
- an amplified genomic nucleic acid section of Arxula containing the pyruvate decarboxylase 1 (ORF = nt 1262-3019) as obtained by PCR is given in SEQ ID NO: 25, the amplified section containing the pyruvate decarboxylase gene between a 5' flanking region (FR5, nt 1-965) and a 3' flanking region (3FR, nt 3061-4005),
- a construct used for inactivating the pyruvate decarboxylase gene 1, the construct containing a selection marker (ATRP, an expression cassette for the tryptophan synthesis gene from Arxula complementing the respective auxotrophy, ORF = nt 1607-2323, promoter nt 1037-1600) flanked by the genomic FR5 (nt 1-955) and FR3 (nt 2497-3441) sections, wherein the pyruvate decarboxylase gene is at least partially interrupted or deleted is given in SEQ ID NO: 26, the amino acid sequence of the selection gene (generally also referred to as a marker gene) is SEQ ID NO: 27,
- the isocitrate lyase gene (AICL1)), having the nucleic acid sequence of SEQ ID NO: 28, encoding its amino acid sequence of SEQ ID NO: 29,
- an amplified genomic nucleic acid section of Arxula containing the isocitrate lyase gene (ORF = nt 989-2629) as obtained by PCR between flanking sequences FR5 (nt 1-907) and FR3 (nt 3044-3945) is given in SEQ ID NO: 30, and
- a construct used for inactivating the isocitrate lyase gene, the construct containing a selection marker (ATRP, an expression cassette for the tryptophan synthesis gene from Arxula, ORF = nt 1547-2263, promoter nt 977-1540) flanked by the genomic FR5 (nt 1-895) and FR3 (nt 2431-3329) sections, wherein the isocitrate lyase gene is at least partially interrupted or deleted is given in SEQ ID NO: 31, with the amino acid sequence of the marker given as SEQ ID NO: 32,
- the mitochondrial acetyl CoA-hydrolase (acetyl CoA-hydrolase 1) (AACH1) having the nucleic acid sequence of SEQ ID NO: 33, encoding its amino acid sequence of SEQ ID NO: 34,
- an amplified genomic nucleic acid section of Arxula containing the acetyl CoA-hydrolase gene (ORF = nt 1332-2891) between flanking sequences FR5 (nt 1-975) and FR3 (nt 3041-4148) is given in SEQ ID NO: 35, and
- a construct used for inactivating the mitochondrial acetyl CoA-hydrolase containing a selection marker (ATRP, an expression cassette for the tryptophan synthesis gene from Arxula, ORF = nt 1630-2346, promoter nt 1060-1623) flanked by the genomic FR5 (nt 1-978) and FR3 (nt 2514-3530) sections, wherein the acetyl CoA-hydrolase gene is at least partially interrupted or deleted is given in SEQ ID NO: 36, with the amino acid sequence of the marker given as SEQ ID NO: 37,
- the cytoplasmatic acetyl-CoA-hydrolase (acetyl CoA-hydrolase 2) (AACH2) having the nucleic acid sequence of SEQ ID NO: 38, encoding its amino acid sequence of SEQ ID NO: 39,
- an amplified genomic nucleic acid section of Arxula containing the acetyl CoA-hydrolase 2 (ORF = nt 1029-2660) between flanking sequences FR5 (nt 1-1001) and FR3(nt 2678-3583) is given in SEQ ID NO: 40, and
- a construct used for inactivating the cytoplasmatic acetyl CoA-hydrolase gene with a selection marker containing a selection marker (ATRP, an expression cassette for the expression cassette for the tryptophan synthesis gene from Arxula, ORF = nt 1543-2259, promoter nt 973-1536) flanked by the genomic FR5 (nt 1-890) and FR3 (nt 2433-3382) sections, wherein the cytoplasmatic acetyl CoA-hydrolase gene is at least partially interrupted or deleted is given in SEQ ID NO: 41, with the amino acid sequence of the marker given as SEQ ID NO: 42,
- the acyl-carnitine transferase (1) (AACT1) having the nucleic acid sequence of SEQ ID NO: 43, encoding its amino acid sequence of SEQ ID NO: 44,
- an amplified genomic nucleic acid section of Arxula containing this acyl-carnitine transferase 1 (ORF = nt 1541-3859) between homologous flanking sequences FR5 (nt 1-989) and FR3 (nt 3937-4969) is given in SEQ ID NO: 45, and
- a construct used for inactivating the acyl-carnitine transferase 1 gene with a selection marker (ATRP, an expression cassette for the tryptophan synthesis gene from Arxula, given in 3' to 5': ORF = nt 1832-1116, promoter nt 2402-1839) flanked by the genomic FR5 (nt 1-944) and FR3 (nt 2480-3515) sections, wherein the acyl-carnitine transferase gene is at least partially interrupted or deleted is given in SEQ ID NO: 46, with the amino acid sequence of the marker given as SEQ ID NO: 47,
- the acyl carnitine transferase 2 gene (AACT2) having the nucleic acid sequence of SEQ ID NO: 48, encoding its amino acid sequence of SEQ ID NO: 49,
- an amplified genomic nucleic acid section of Arxula containing this acyl-carnitine transferase 2 (ORF = nt 1024-2877) between homologous flanking sequences FR5 (nt 1-953) and FR3 (nt 2903-3894) is given in SEQ ID NO: 50, and
- a construct used for inactivating the acyl-carnitine transferase 2 gene with a selection marker (ATRP, an expression cassette for the tryptophan synthesis gene from Arxula, in 3' to 5': ORF = nt 1842-1126, promoter nt 2412-1849) flanked by the genomic FR5 (nt 1-953) and FR3 (nt 2495-3486) sections, wherein the acyl-carnitine transferase 2 gene is at least partially interrupted or deleted is given in SEQ ID NO: 51, with the amino acid sequence of the marker given as SEQ ID NO: 52,
- the ORF, currently identified as a carnitine-acylcarnitine permease 1 (AACP1), having the nucleic acid sequence of SEQ ID NO: 53, encoding its amino acid sequence of SEQ ID NO: 54,
- an amplified genomic nucleic acid section of Arxula containing this carnitine-acylcarnitine permease 1 (ORF = 971-1912) between homologous flanking sequences FR5 (nt 1-963) and FR3 (nt 1968-2692), having the nucleic acid sequence of SEQ ID NO: 55,
- a construct used for inactivating this carnitine-acylcarnitine permease 1 gene with a selection marker (ATRP, an expression cassette for the tryptophan synthesis gene from Arxula, in 3' to 5': ORF = nt 1852-1136, promoter nt 2422-1859) between homologous flanking sequences FR5 (nt 1-963) and FR3 (nt) 2487-3211), wherein the carnitine-acylcarnitine permease 1 gene is at least partially interrupted or deleted is given in SEQ ID NO: 56, with the amino acid sequence of the marker given as SEQ ID NO: 57,
- the carnitine-acylcarnitine permease 2 (AACP2) having the nucleic acid sequence of SEQ ID NO: 58, encoding its amino acid sequence of SEQ ID NO: 59,
- an amplified genomic nucleic acid section of Arxula containing this carnitine-acylcarnitine permease 2 (ORF = nt 1665..2615) between homologous flanking sequences FR5 (nt 1-877) and FR3 (nt 2882-3914) is given in SEQ ID NO: 60, and
- a construct used for inactivating this acyl-carnitine permease gene 2 with a selection marker (ATRP, an expression cassette for the tryptophan synthesis gene from Arxula, ORF = nt 1524-2246, promoter nt 960-1523) between homologous flanking sequences FR5 (nt 1-877) and FR3 (nt 2414-3411) wherein the acyl-carnitine permease gene 2 gene is at least partially interrupted or deleted is given in SEQ ID NO: 61, with the amino acid sequence of the marker given as SEQ ID NO: 62.

In the above constructs used for inactivating the respective metabolic pathway gene, nucleic acid sections between indicated sections can be fractions from a bacterial shuttle vector. Unless indicated otherwise, nucleic acid sequences are given in 5' to 3'. Generally, the nucleic acid sequences given contain the yeast nucleic acid sections in 5' to 3'. In the sequences SEQ ID NO: 46, SEQ ID NO: 51 and SEQ ID NO: 56, the cassette containing the selection gene ATRP is oriented in reverse to the flanking yeast nucleic acid sections in agreement with the respective preceding sequences relating to the same yeast gene. SEQ ID NO: 25, SEQ ID NO: 30, SEQ ID NO: 35, SEQ ID NO: 40, SEQ ID NO: 45, SEQ ID NO: 50 and SEQ ID NO: 60 are given in accordance with the genome sequence of Arxula adeninivorans, and therefore can be in 3' to 5'.

The production process comprises the steps of
providing a genetically manipulated yeast of the invention,
providing a substrate in an aqueous composition in a fermentation vessel under control of pH and temperature,
preferably with controlled introduction of air, e.g. sparging with air, preferably controlled by the dissolved oxygen concentration, and with optional agitation, and isolating or separating n-butanol, optionally in combination with acetone and/or ethanol, from the fermentation broth resulting from the cultivation of the yeast in the substrate composition.

Preferably, the fermentation process comprises a first aerobic phase in an aqueous medium composition containing substrate without inductor, e.g. without nitrate and without nitrite in the case of a yeast containing the butanol pathway enzyme encoding genes under the control of a nitrate and/or nitrite inducible promoter, e.g. containing monomeric or polymeric amino acids and/or ammonia as a nitrogen source, and a second semi-anaerobic or anaerobic phase which is commenced by addition of the inductor, e.g. nitrate and/or nitrite. The second phase of the fermentation process can be carried out as a fed-batch fermentation or as a continuous fermentation by addition of substrate. Preferably, n-butanol, ethanol and/or acetone are isolated continuously from the fermentation broth, e.g. by pervaporation through a membrane, under reduced pressure, i.e. under vacuum, by stripping using a gaseous or liquid stripping medium, or by a combination of these processes. Isolation of n-butanol, ethanol and/or acetone from the fermentation broth can occur in the fermentation vessel, or from a withdrawal pipe of the vessel with optional recirculation of the fermentation broth after isolation of n-butanol, ethanol and/or acetone.

The fermentation and cultivation process advantageously is carried out at a cultivation temperature of 10 to 70 °C, preferably 25 to 50 °C, using a genetically modified Arxula adeninivorans, because the elevated temperature allows an effective isolation of n-butanol, ethanol and/or acetone from the fermentation broth. The elevated cultivation temperature can be used, because the genetically modified Arxula is capable of growth and of butanol synthesis at these temperatures.

### Detailed description of the invention

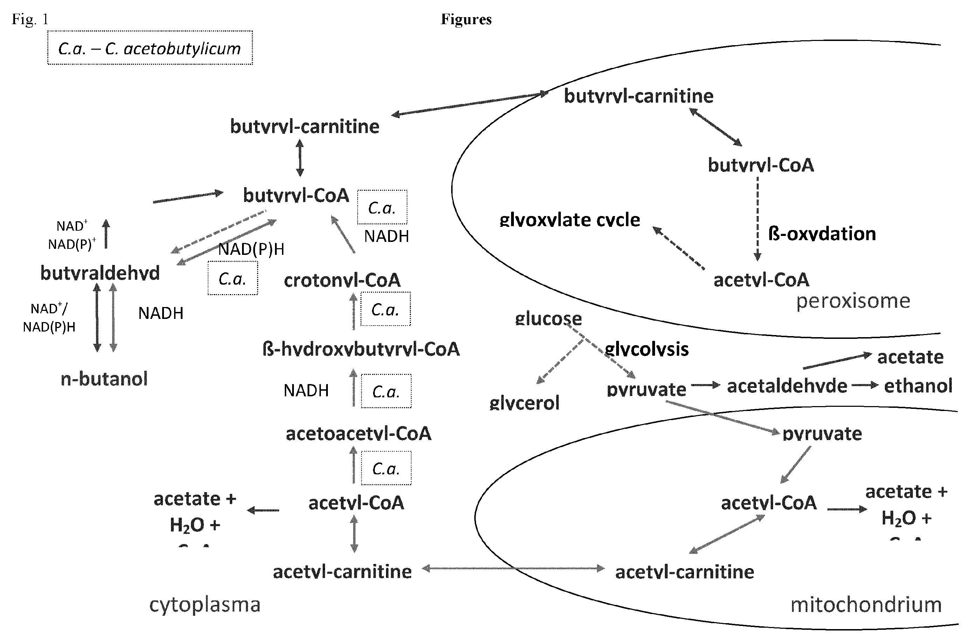

The invention is now described in greater detail with reference to the figures by way of examples. The figures schematically show in
- Figure 1: An overview of the metabolism as catalyzed by homologous and heterologous pathway enzymes expressed in the yeast which are essential for the butanol synthesis.

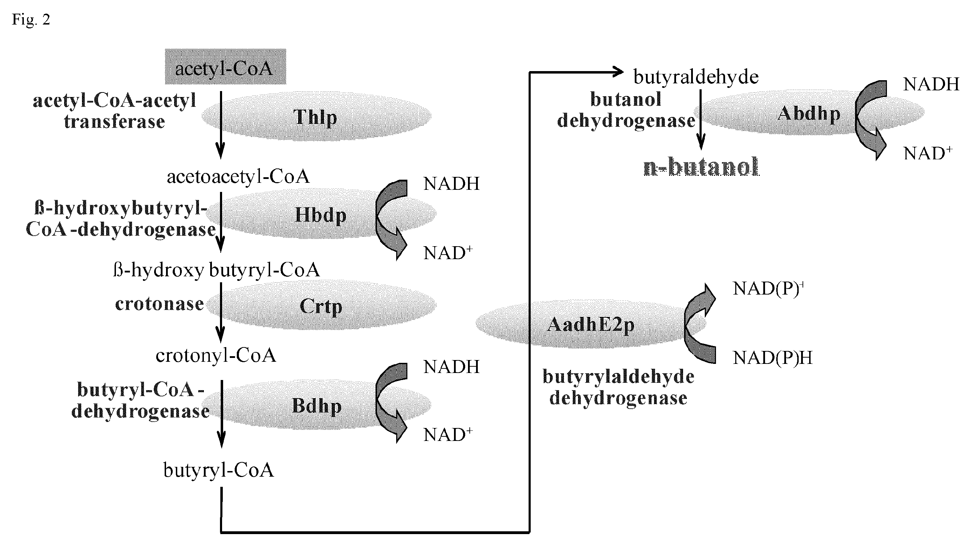

- Figure 2: Butanol metabolism from acetyl-CoA to butanol after insertion of the expression modules containing nucleic acid sequences encoding the Clostridium acetobutylicum enzymes acetyl-CoA-acetyl transferase (Thlp), ß-hydroxybutyryl-CoA-dehydrogenase (Hbdp), crotonase (Crtp), and butyryl-CoA-dehydrogenase (Bdhp), including the homologous genes encoding butyraldehyde dehydrogenase (AadhE2p) and butanol dehydrogenase (Abdhp) of Arxula adeninivorans.

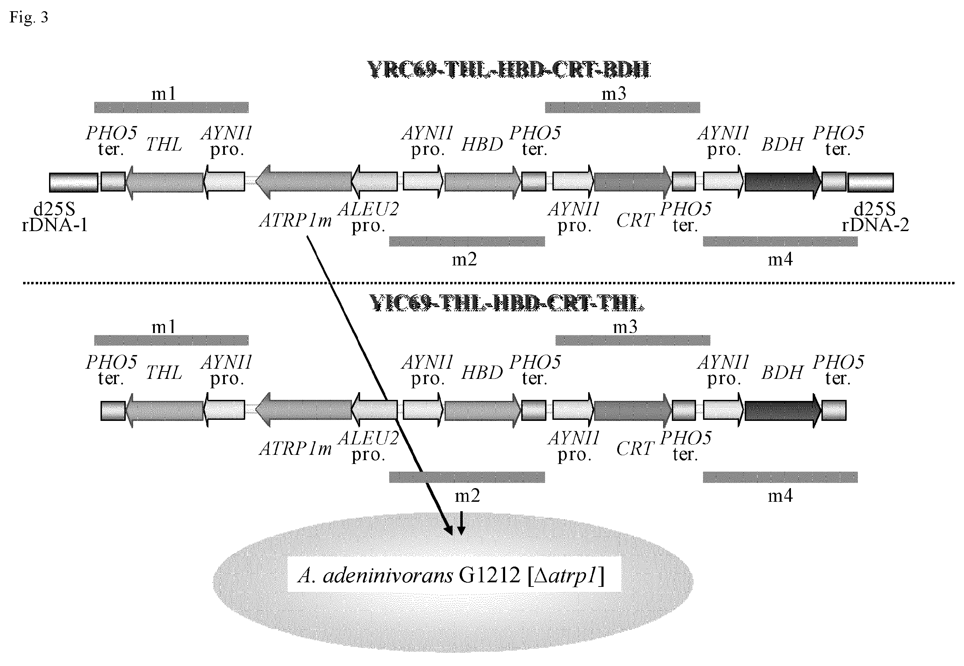

- Figure 3: Nucleic acid constructs YRC69-THL-HBD-CRT-BDH and YIC69-THL-HBD-CRT-BDH, each containing expression modules encoding the Clostridium acetobutylicum enzymes Thlp, Hbdp, Crtp, and Bdhp used for expression of these pathway enzymes participating in or constituting the metabolic pathway from acetyl-CoA to butyryl-CoA in Arxula adeninivorans (strain G1212).

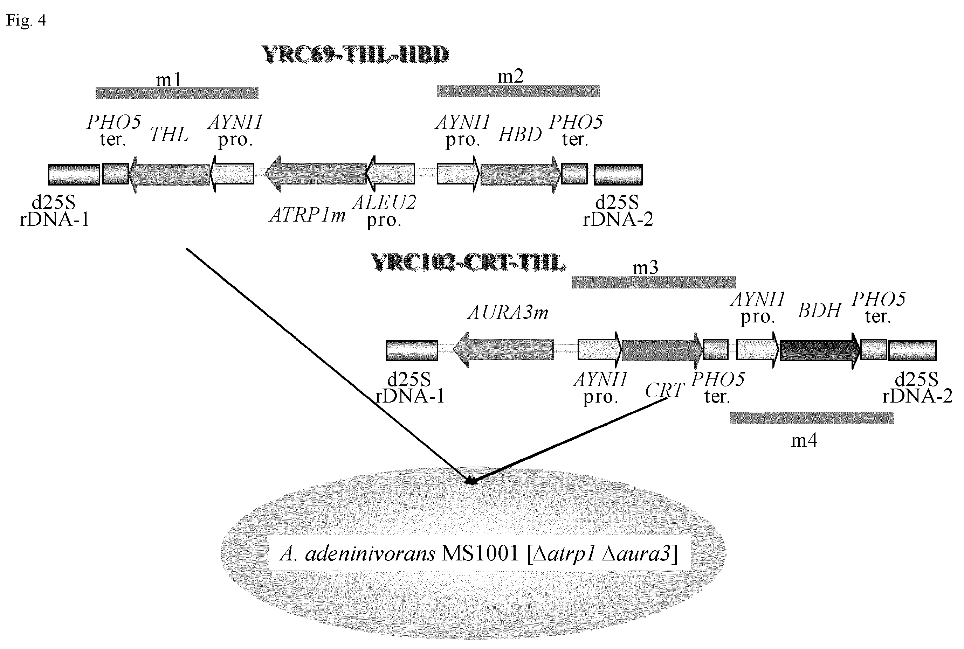

- Figure 4: Nucleic acid constructs YRC69-THL-HBD - YRC102-CRT-BDH and YIC69-THL-HBD - YIC102-CRT-BDH, each containing expression modules encoding the Clostridium acetobutylicum enzymes Thlp, Hbdp, and Crtp, Bdhp, respectively, for expression of these pathway enzymes participating in or constituting the metabolic pathway from acetyl-CoA to butyryl-CoA in Arxula adeninivorans (strain MS1001).

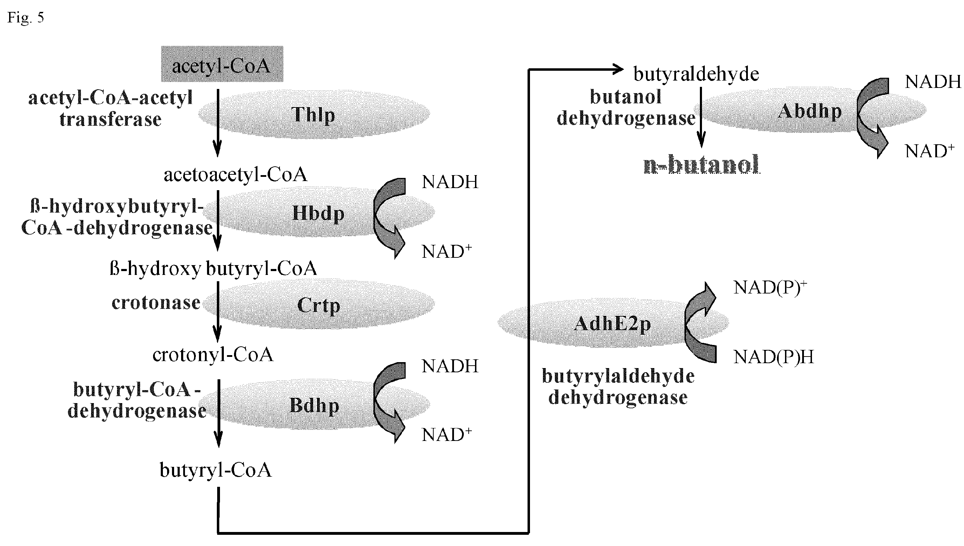

- Figure 5: An overview of butanol metabolism from acetyl-CoA to butanol after insertion of the expression modules encoding the Clostridium acetobutylicum enzymes acetyl-CoA-acetyl transferase (Thlp), ß-hydroxybutyryl-CoA-dehydrogenase (Hbdp), crotonase (Crtp), butyryl-CoA-dehydrogenase (Bdhp) and butyraldehyde dehydrogenase (AdhE2p) including the homologous gene encoding butanol dehydrogenase (Abdhp) of Arxula adeninivorans.

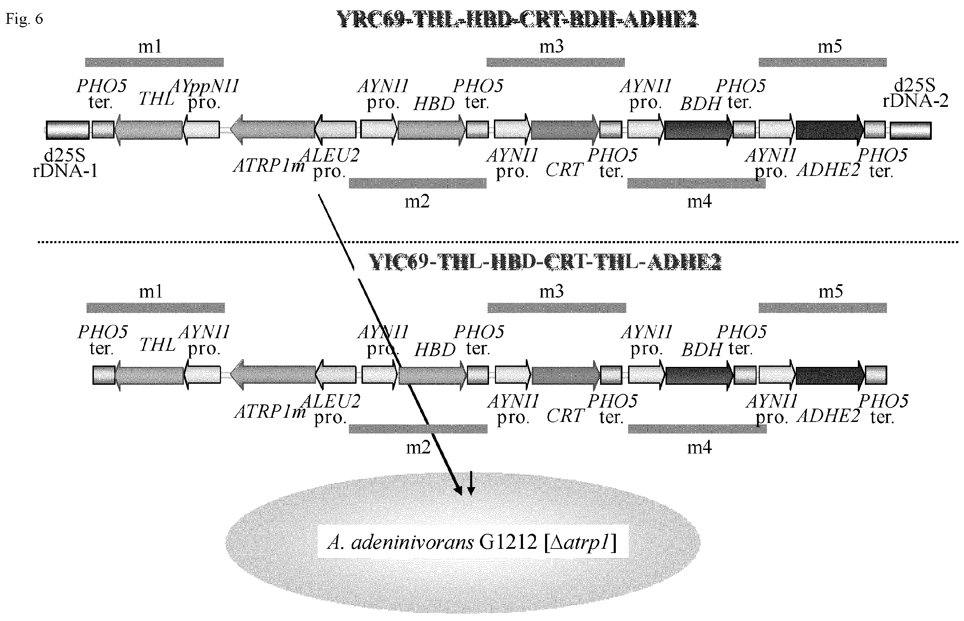

- Figure 6: Nucleic acid constructs YRC69-THL-HBD-CRT-BDH-ADHE2 and YIC69-THL-HBD-CRT-BDH-ADHE2, each containing expression modules encoding the Clostridium acetobutylicum enzymes acetyl-CoA-acetyl transferase (Thlp), ß-hydroxybutyryl-CoA-dehydrogenase (Hbdp), crotonase (Crtp), butyryl-CoA-dehydrogenase (Bdhp) and butyraldehyde dehydrogenase (AdhE2p), respectively, for the metabolic pathway from acetyl-CoA to butyraldehyde in Arxula adeninivorans (strain G1212).

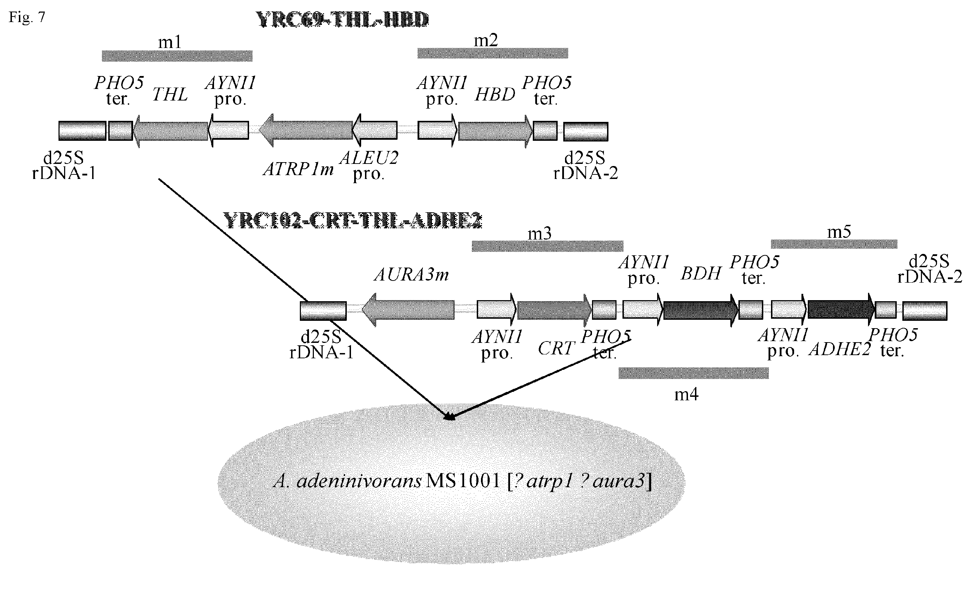

- Figure 7: Nucleic acid constructs YRC69-THL-HBD - YRC102-CRT-BDH-ADHE2 and YIC69-THL-HBD - YIC102-CRT-BDH-ADHE2, containing expression modules encoding the Clostridium acetobutylicum enzymes acetyl-CoA-acetyl transferase (Thlp), ß-hydroxybutyryl-CoA-dehydrogenase (Hbdp), crotonase (Crtp), butyryl-CoA-dehydrogenase (Bdhp) and butyraldehyde dehydrogenase (AdhE2p), respectively, for constituting the metabolic pathway from acetyl-CoA to butyraldehyde in Arxula adeninivorans (strain MS1001).

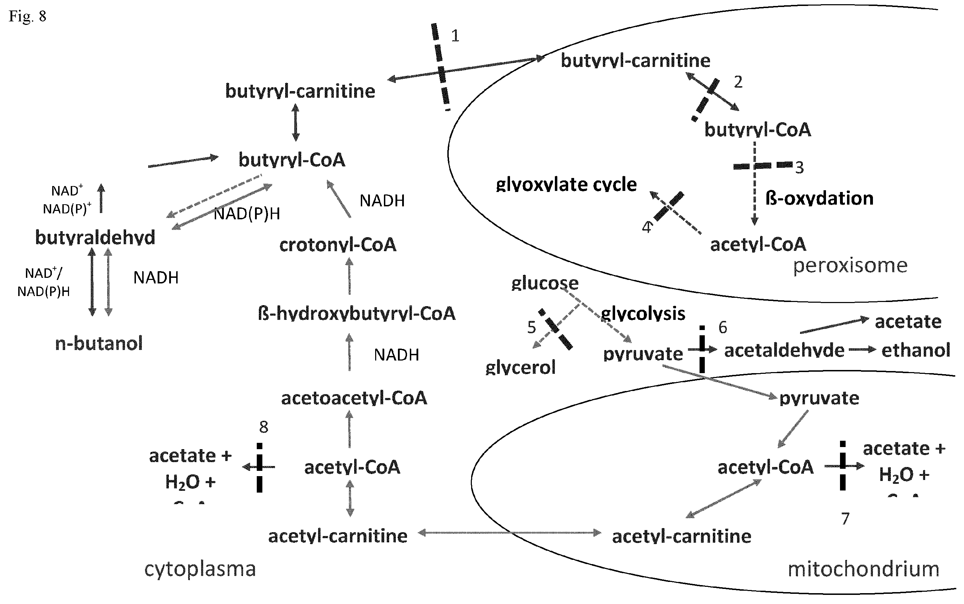

- Figure 8: An overview of the metabolism as catalyzed by homologous and heterologous pathway enzymes expressed in the yeast which are essential for butanol synthesis including inactivation of the (1) peroxisomal acyl-carnitine permease - Aacpp, (2) the acyl-carnitine transferase - Aactp, (3) the acyl-CoA oxidase - Aacop, (4) isocitrate lyase - Aiclp, (5) the glycerol-3-phosphate dehydrogenase 4 - Agpdlp, (6) the pyruvate decarboxylase - Apdcp, (7) the mitochondrial acetyl CoA hydrolase 1 - Aach1p and/or (8) the cytosolic acetyl CoA hydrolase 2 - Aach2p to improve the butanol level.

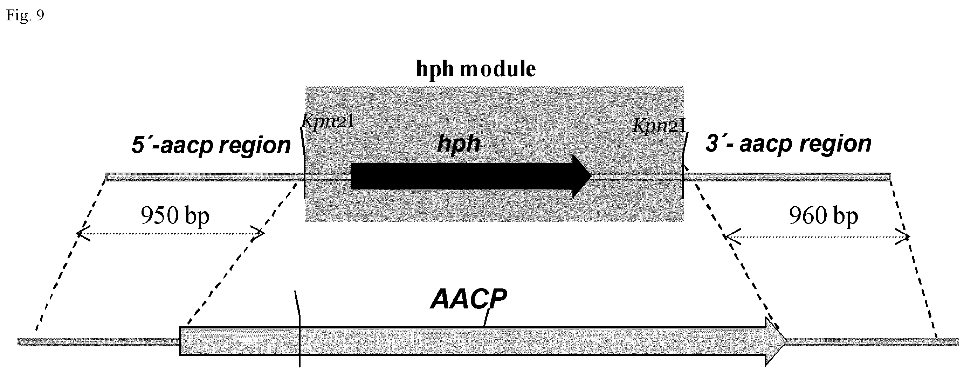

- Figure 9: Construction procedure for an Arxula adeninivorans aacp gene disruption mutant.

Preferably, the acetyl-CoA-acetyl transferase (Thlp) has the amino acid sequence of SEQ ID NO: 1,
the β-hydroxy butyryl-CoA dehydrogenase (Hbdp) has the amino acid sequence of SEQ ID NO: 2,
the crotonase (3-hydroxybutyryl-CoA dehydratase, Crtp) has the amino acid sequence of SEQ ID NO: 3,
the butyryl-CoA dehydrogenase (Bdhp) has the amino acid sequence of SEQ ID NO: 4. These sequences SEQ ID NO: 1 to SEQ ID NO: 4 originate from Clostridium.

Preferably, the butyraldehyde dehydrogenase (AadhE2p) and the butanol dehydrogenase (Abdhp) are the endogenous enzymes of Arxula. As an alternative or in addition to the endogenous AadhE2p of Arxula, the heterologous butyraldehyde dehydrogenase (AdhE2) having SEQ ID NO: 5 from Clostridium acetobutylicum can be expressed in the yeast of the invention.

More preferably, the amino acid sequences of acetyl-CoA- acetyl transferase (Thlp), of β-hydroxybutyryl-CoA dehydrogenase (Hbdp), of crotonase (Crtp), of butyryl-CoA dehydrogenase (Bdhp) and of butyraldehyde dehydrogenase (AdhE2p) are encoded by nucleic acid sequences which are codon-optimized for Arxula, e.g. by a coding sequence having a codon usage of the codons as preferred in Arxula for optimum translation. Preferably, the acetyl-CoA- acetyl transferase is encoded by codon-optimized SEQ ID NO: 6, the β-hydroxy butyryl-CoA dehydrogenase is encoded by codon-optimized SEQ ID NO: 7, the crotonase is encoded by codon-optimized SEQ ID NO: 8, the butyryl-CoA dehydrogenase is encoded by codon-optimized SEQ ID NO: 9, and the heterologous butyraldehyde dehydrogenase is encoded by codon-optimized SEQ ID NO: 10. Generally, a coding sequence in contained in an expression module, wherein e.g. the coding sequence is functionally arranged in 3' to a promoter, and in 5' to a terminator.

The preferred promoters for regulating expression of the genes are independently selected from the group of promoters which are inducible by nitrate and/or nitrite, preferably from promoters of *AYNR1* (SEQ ID NO: 11), *AYNR1' (SEQ* ID NO: 12), *AYNI1* (SEQ ID NO: 13), *AYNI1'* (SEQ ID NO*:* 14) und *AYNT1* (SEQ ID NO: 15), *AYNT1'* (SEQ ID NO: 16) which originate from the nitrate reductase gene *(AYNR1),* nitrite reductase gene *(AYNI1),* and from the nitrate transporter gene *(AYNT1)* of Arxula adeninivorans, as well as truncated promoters of these, preferably a truncated variant of *AYNR1* termed *AYNR1-2* (SEQ ID NO: 17) and/or a truncated variant of *AYNR1'* termed *AYNR1' -2* (SEQ ID NO: 18).

### Example 1: Generation of a yeast which is genetically manipulated to contain coding sequences for the metabolic pathway to n-butanol

An Arxula adenininvorans strain having at least one auxotrophic marker was transformed with nucleic acid constructs containing a complementing gene as a selection marker. The Arxula adeninivorans contained an impaired metabolic pathway converting acyl-carnitine to peroxisomal acetyl-CoA. This yeast was obtained by interrupting the acyl-carnitine transferase gene, or alternatively one of the genes from the gene encoding the peroxisomal acyl-carnitine permease, the gene encoding the acyl-CoA oxidase, and the gene encoding the glycerol-3-phosphate dehydrogenase. Gene interruption was obtained by genetic manipulation of Arxula as described in Steinborn G, Wartmann T, Gellissen G, Kunze G (2007), Construction of an Arxula adeninivorans host-vector system based on trp1 complementation. J. Biotechnol. 127: 392-401.

According to a further preferred embodiment, wherein each sequence encoding an enzyme is contained in an expression module under the control of a nitrate and/or nitrite inducible promoter with a terminator element represented by the PHO5 terminator. As depicted, the expression modules encoding acetyl-CoA- acetyl transferase (Thlp, SEQ ID NO: 1), the β-hydroxy butyryl-CoA dehydrogenase (Hbdp, SEQ ID NO: 2), the crotonase (Crtp, SEQ ID NO: 3), the butyryl-CoA dehydrogenase (Bdhp, SEQ ID NO: 4) and butyraldehyde dehydrogenase (AdhE2p, SEQ ID NO: 5) can be contained on one common nucleic acid construct. Optionally, the butyraldehyde dehydrogenase gene of Arxula (AadhE2), and/or the butanol dehydrogenase gene of Arxula (ABDH) in addition to the genomic gene copy is contained on the nucleic acid construct for genetically manipulating the yeast, especially for integrating an expression module having an inducible promoter, as exemplified by the nitrate and/or nitrite inducible promoter *AYNI1* (SEQ ID NO: 13) for each coding sequence, for simultaneous and increased induction of the enzyme.

The Xplor^{®}2 transformation/expression platform was used for integration of the nucleic acid constructs into the Arxula adeninivorans genomic DNA (as e.g. described by Boer E, Piontek M, Kunze G (2009) Xplor®2 - an optimized transformation/expression system for recombinant protein production in the yeast A. adeninivorans. Appl Microbiol Biotechnol 84:583-594). The nucleic acid constructs are Yeast rDNA Integrative Expression Cassettes (YRCs), that contain homologous DNA sections at both termini, which are represented by the exemplary 25S rDNA sections d25S rDNA-1 and d25S rDNA-2 (YRC). The embodiments of nucleic acid constructs which are Yeast Integrative Expression Cassettes (YIC) do not contain the terminal homologous rDNA sections. Both the YRC and YIC contain a selection marker module, e.g. an expression cassette containing a gene for complementing an auxotrophy of the recipient yeast, as shown by the exemplary complementing gene ATRP1m serving as a selection marker under the control of the promoter ALEU2 which complements the tryptophan auxotrophy of a recipient yeast, e.g. Arxula adeninivorans G1212 [aleu2 Δatrp1::ALEU2] or MS1001 [aleu2 Δatrp1::ALEU2 aleu2 Δaura3::ALEU2]. In addition, the expression modules encoding Thlp, Hbdp, Crtp, Bdhp, and alternatively AdhE2p are localised on YRC and YIC (Figs. 3 and 6).

Figures 4 and 7 show an alternative cloning procedure for generating a genetically manipulated yeast of the invention. In this procedure, the expression modules encoding the pathway enzymes are contained on two separate YRC' s, each of which has a complementing gene as a selection marker, and the recipient yeast Arxula adeninivorans MS 1001 [aleu2 Δatrp1::ALEU2 aleu2 Δaura3::ALEU2] has the respective auxotrophy which is complemented by the respective selection marker. The first YRC (YRC69-THL-HBD) contains the expression modules encoding Thlp and Hbdp, each under the control of a nitrate and/or nitrite inducible promoter, and comprises the selection marker ATRP1m encoding sequence for complementing the atrp1 auxotrophy of the recipient Arxula adeninivorans MS1001 [aleu2 Δatrp1::ALEU2 aleu2 Δaura3::ALEU2], and the second YRC (YRC102-CTR-THL) contains the expression modules for CRT, BDH, and optionally for ADHE2, which can be an alternative to or an addition to the endogenous gene of Arxula, as well as the selection marker AURA3m for complementing the aura3 auxotrophy of the recipient yeast Arxula adeninivorans MS1001 [aleu2 Δatrp1::ALEU2 aleu2 Δaura3::ALEU2].

As shown for the YRC's of Figures 3,4,6,7, the nucleic acid constructs can contain up to five expression modules for the metabolic pathway enzymes for n-butanol plus the respective selection marker. These nucleic acid constructs are flanked at both termini by homologous sections for homologous recombination and integration, e.g. d25S rDNA sections d25S rDNA -1 and d25S rDNA -2. In contrast YIC's, prefer the non-homologous recombination for their integration in the genomic DNA.

Following transformation of the auxotrophic Arxula strains G1212 and/or MS1001 according to standard procedures, selection of transformants was by cultivation on yeast minimal medium as described by Tanaka A, Ohnishi N, Fukui S (1967) Studies on the formation of vitamins and their function in hydrocarbon fermentation. Production of vitamin B6 by Candida albicans in hydrocarbon medium. J Ferment Technol 45:617-623.

Figures 2, 5 and 8 give an overview of the metabolic pathway provided by the yeast and the heterologous genes with the impairments of the metabolism.

### Example 2: Arxula adeninivorans strain optimization by gene disruption

Impairment of the metabolic flux from butanol synthesis towards peroxisomal catabolism is achieved by inactivating the peroxisomal acyl-carnitine permease 1 (Aacpp), the acyl-carnitine transferase 2 (Aactp), the acyl-CoA oxidase 3 (Aacop), and/or the glycerol-3-phosphate dehydrogenase 4 (Agpd1p). For this purpose, the Arxula adeninivorans genes AACP, AACT, AACO and/or AGPD1 were mutated by gene disruption. Since Arxula adeninivorans MS1001 is a hygromycin B sensitive strain, all gene disruption mutants were initially selected as hygromycin B resistant transformants by using an expression cassette containing the TEF1 promoter - hph gene (from E. coli) - PHO5 terminator which was flanked by ca. 950 bp Δacpp, Δaact, Δaaco and Δagpd1 fragments. E.g., the Δaacp fragment with 950 bp 5'-aacp region - hph expression module - 960 bp 3'-aacp region was amplified by PCR and used to transform Arxula adeninivorans MS1001 for sequential disruption of the chromosomal AACP copy. For selection of the respective gene disruption mutants, the transformants were firstly selected for hygromycin resistance, and secondly selected for aacp deletion by amplification of the inserted 5'-aacp region - hph expression cassette - 3'-aacp region and Southern hybridization with the AACP fragment as labelled probe (Fig. 4).

Δaact, Δaaco and Δagpd1 gene disruption mutants were constructed using the same strategy. Using transformation by constructs containing a selection (marker) gene expression cassette between flanking regions of the metabolism gene to be inactivated, e.g. nucleic acid constructs according to SEQ ID NO: 26 for the inactivation of the pyruvate decarboxylase gene (APDC1), SEQ ID NO: 31 for the inactivation of the isocitrate lyase gene (AICL1), SEQ ID NO: 36 for the inactivation of the acetyl-CoA hydrolase 1 gene (AACH1), SEQ ID NO: 41 for the inactivation of the acetyl-CoA hydrolase 2 gene (AACH2), SEQ ID NO: 46 for the inactivation of the acyl-carnitine transferase 1 gene (AACT1), SEQ ID NO: 51 for the inactivation of the acyl-carnitine transferase 2 gene (AACT2), SEQ ID NO: 56 for the inactivation of the carnitine- acylcarnitine permease 1 gene (AACP1), SEQ ID NO: 61 for the inactivation of the carnitine- acylcarnitine permease 2 gene (AACP2), the respective genes could be inactivated, e.g. deleted. The resulting transformants contained the selection marker gene cassette between the flanking regions with only remnants of the original structural genomic gene.

### Example 3: Production of n-butanol from a starch substrate by fermentation

An Arxula strain that was obtained by the cloning procedure according to Example 1 was cultivated in an aqueous composition of starch, containing ammonia as a buffering substance in the absence of nitrate and absence of nitrite at 25-48°C under aerobic conditions in an agitated 3 L fermenter. Subsequent to an initial cultivation phase in the absence of inductor, nitrate was added and an aqueous starch composition containing 10-50 g/L starch was added for fed-batch fermentation. N-butanol was separated from the fermentation broth by standard methods. Fermentation of a yeast strain obtained according to Example 2 gave higher production of n-butanol, especially a higher yield of n-butanol per initial substrate. Analysis of the fermentation broth by gas chromatography with mass spectrometric detection showed production of up to 1 g/L n-butanol, preferably up to 20 g/L n-butanol, more preferably at least 50 g/L n-butanol.

## Claims

1. Genetically manipulated yeast containing heterologous nucleic acid sequences encoding pathway enzymes for the synthesis of n-butanol from a substrate, **characterized in that** the heterologous pathway enzymes comprise an acetyl-CoA-acetyl transferase (Thlp), a β-hydroxy butyryl-CoA dehydrogenase (Hbdp), a crotonase (Crtp) and a butyryl-CoA dehydrogenase (Bdhp) and each encoding nucleic acid sequence is arranged in an expression module, in which yeast the metabolic pathway from acyl-carnitine to β-oxidation is impaired or interrupted by mutation of at least one of the endogenous genes encoding at least one of the peroxisomal acyl-carnitine transferase, the peroxisomal acyl-carnitine permease, and/or the acyl-CoA oxidase, and/or that the metabolic pathway from dihydroxyacetone phosphate to glycerol is impaired or interrupted by mutation of the gene encoding the glycerol-3-phosphate dehydrogenase.

2. Genetically manipulated yeast according to claim 1, **characterized in that** the heterologous pathway enzymes consist of an acetyl-CoA-acetyl transferase (Thlp), a β-hydroxy butyryl-CoA dehydrogenase (Hbdp), a crotonase (Crtp), and a butyryl-CoA dehydrogenase (Bdhp).

3. Genetically manipulated yeast according to claim 1 or 2, **characterized in that** each expression module the heterologous nucleic acid sequences encoding pathway enzymes for the synthesis of butanol are each independently arranged under the control of a promoter which is inducible by nitrate and/or nitrite, a heat inducible promoter, and/or a promoter inducible by the substrate

4. Genetically manipulated yeast according to one of the preceding claims, **characterized in that** the yeast is of the genus Arxula.

5. Genetically manipulated yeast according to one of the preceding claims, **characterized in that** the yeast contains a heterologous nucleic acid sequence encoding a butyraldehyde dehydrogenase (AdhE2p).

6. Genetically manipulated yeast according to one of the preceding claims, **characterized in that** at least one of the genes catalyzing the metabolism from acetyl-CoA to the citric acid cycle or glyoxylate cycle is impaired or inactivated.

7. Genetically manipulated yeast according to one of the preceding claims, **characterized in that** at least one of the genes catalyzing the metabolism from pyruvate to acetaldehyde are impaired or inactivated.

8. Genetically manipulated yeast according to one of the preceding claims, **characterized in that** the genes encoding a metabolic pathway enzyme catalyzing the metabolism from acetyl-CoA to acetate and CoA are impaired or inactivated.

9. Genetically manipulated yeast according to one of the preceding claims, **characterized in that** each expression module containing a nucleic acid sequence encoding a pathway enzyme for the synthesis of n-butanol contains the same promoter.

10. Genetically manipulated yeast according to one of the preceding claims, **characterized in that** the acetyl-CoA-acetyl transferase has the amino acid sequence of SEQ ID NO: 1, the β-hydroxy butyryl-CoA dehydrogenase has the amino acid sequence of SEQ ID NO: 2, the crotonase has the amino acid sequence of SEQ ID NO: 3, the butyryl-CoA dehydrogenase has the amino acid sequence of SEQ ID NO: 4, and the optional butyraldehyde dehydrogenase (AdhE2p) has the amino acid sequence of SEQ ID NO: 5.

11. Genetically manipulated yeast according to one of the preceding claims, **characterized in that** the butyraldehyde dehydrogenase and the butanol dehydrogenase are endogenous enzymes.

12. Genetically manipulated yeast according to one of the preceding claims, **characterized in that** the acetyl-CoA-acetyl transferase is encoded by SEQ ID NO: 6, the β-hydroxy butyryl-CoA dehydrogenase is encoded by SEQ ID NO: 7, the crotonase is encoded by SEQ ID NO: 8, the butyryl-CoA dehydrogenase is encoded by SEQ ID NO: 9, and the optional additional butyraldehyde dehydrogenase is encoded by SEQ ID NO: 10.

13. Genetically manipulated yeast according to one of the preceding claims, **characterized in that** the butyryl-CoA-dehydrogenase has a sequence encoding an enzyme having a homology of at least 90% to 100% to the amino acid sequence encoded by SEQ ID NO: 19.

14. Genetically manipulated yeast according to one of the preceding claims, **characterized in that** the acetyl-CoA-acetyltransferase has a sequence encoding an enzyme having a homology of at least 90% to 100% to the amino acid sequence encoded by SEQ ID NO: 21.

15. Genetically manipulated yeast according to one of the preceding claims, **characterized in that** at least one gene encoding a metabolic enzyme is inactivated, which gene encodes an amino acid sequence having an amino acid sequence homology of at least 90% to one of the sequences contained in the group consisting of SEQ ID NO: 24 having pyruvate decarboxylase activity, SEQ ID NO: 29 having isocitrate lyase activity, SEQ ID NO: 34 having acetyl-CoA hydrolase activity, SEQ ID NO: 39 having acetyl-CoA hydrolase activity, SEQ ID NO: 44 having acyl-carnitine transferase activity, SEQ ID NO: 49 having acyl-carnitine transferase activity, SEQ ID NO: 54 having carnitine-acylcarnitine permease acitivity and SEQ ID NO: 59 having carnitine-acylcarnitine permease acitivity.

16. Process for producing n-butanol from a substrate by cultivation of a yeast in an aqueous fermentation broth containing the substrate, **characterized in that** the yeast is a yeast according to one of the preceding claims, and n-butanol is isolated from the fermentation broth.

17. Process according to claim 16, **characterized in that** the cultivation and fermentation process is carried out at a temperature between 10 to 70°C, preferably between 25 to 50°C.

18. Process according to one of claims 16 to 17, **characterized in that** the cultivation comprises a first aerobic phase in a fermentation broth in the absence of inductor, and a subsequent second phase is commenced by addition of inductor.

19. Process according to one of claims 16 to 18, **characterized in that** the cultivation and fermentation processes are carried out as continuous processes.

20. Process according to one of claims 16 to 19, **characterized in that** the substrate is selected from aqueous mixtures containing excrements from animals, household sewage, waste containing protein, fats, alkanes, n-alkanes, alcohols, ethanol, starch, lignocelluloses, tannins, pentose and/or hexose sugars which are comprised of monomeric, oligomeric or polymeric sugar moieties and/or of sugar alcohol moieties and mixtures thereof.

## Patentansprüche

1. Genetisch manipulierte Hefe, die heterologe Nukleinsäuresequenzen enthält, die Stoffwechselwegenzyme für die Synthese von n-Butanol aus einem Substrat kodieren, **dadurch gekennzeichnet, dass** die heterologen Stoffwechselwegenzyme eine Acetyl-CoA-Acetyl-Transferase (Thlp), eine β-Hydroxybutyryl-CoA-Dehydrogenase (Hbdp), eine Crotonase (Crtp), und eine Butyryl-CoA-Dehydrogenase (Bdhp) umfassen und jede kodierende Nukleinsäuresequenz in einem Expressionsmodul angeordnet ist, wobei in der Hefe der Stoffwechselweg von Acylcarnitin zur Betaoxidation durch Mutation wenigstens eines der endogenen Gene eingeschränkt oder unterbrochen ist, die wenigstens eines von peroxisomaler Acetylcarnitin-Transferase, der peroxisomalen Acylcarnitin-Permease und/oder der Acetyl-CoA-Oxidase kodieren, und/oder dass der Stoffwechselweg vom Dihydroxyacetonphosphat zum Glycerin durch Mutation des Gens beeinträchtigt oder unterbrochen ist, das die Glycerin-3-phosphat-Dehydrogenase kodiert.

2. Genetisch manipulierte Hefe nach Anspruch 1, **dadurch gekennzeichnet, dass** die heterologen Stoffwechselwegenzyme aus einer Acetyl-CoA-Acetyl-Transferase (Thlp), einer β-Hydroxybutyryl-CoA- Dehydrogenase (Hbdp), einer Crotonase (Crtp) und einer Butyryl-CoA-Dehydrogenase (Bdhp) bestehen.

3. Genetisch manipulierte Hefe nach Anspruch 1 oder 2, **dadurch gekennzeichnet, dass** in jedem Expressionsmodul die heterologen Nukleinsäuresequenzen, die die Stoffwechselwegenzyme für die Synthese von Butanol kodieren, jeweils unabhängig unter der Kontrolle eines Promotors angeordnet sind, der durch Nitrat und/oder Nitrit induzierbar ist, einem durch Wärme induzierbaren Promotor und/oder einem durch ein Substrat induzierbaren Promotor.

4. Genetisch manipulierte Hefe nach einem der voranstehenden Ansprüche, **dadurch gekennzeichnet, dass** die Hefe von der Art Arxula ist.

5. Genetisch manipulierte Hefe nach einem der voranstehenden Ansprüche, **dadurch gekennzeichnet, dass** die Hefe eine heterologe Nukleinsäuresequenz enthält, die eine Butyraldehyd-Dehydrogenase (AdhE2p) kodiert.

6. Genetisch manipulierte Hefe nach einem der voranstehenden Ansprüche, **dadurch gekennzeichnet, dass** wenigstens eines der Gene, die den Stoffwechsel von Acetyl-CoA zum Zitronensäurezyklus oder Glyoxylatzyklus katalysieren, beeinträchtigt oder inaktiviert ist.

7. Genetisch manipulierte Hefe nach einem der voranstehenden Ansprüche, **dadurch gekennzeichnet, dass** wenigstens eines der Gene, die den Stoffwechsel vom Pyruvat zum Acetaldehyd katalysieren, beeinträchtigt oder inaktiviert ist.

8. Genetisch manipulierte Hefe nach einem der voranstehenden Ansprüche, **dadurch gekennzeichnet, dass** die Gene, die ein Stoffwechselwegenzym kodieren, die den Stoffwechsel von Acetyl-CoA zu Acetat und CoA katalysieren, beeinträchtigt oder inaktiviert ist.

9. Genetisch manipulierte Hefe nach einem der voranstehenden Ansprüche, **dadurch gekennzeichnet, dass** in jedem Expressionsmodul, das eine Nukleinsäuresequenz, die ein Stoffwechselwegenzym für die Synthese von n-Butanol kodiert, denselben Promotor enthält.

10. Genetisch manipulierte Hefe nach einem der voranstehenden Ansprüche, **dadurch gekennzeichnet, dass** die Acetyl-CoA-Acetyl-Transferase die Aminosäuresequenz von SEQ ID NO: 1 hat, die β-Hydroxy-butyryl-CoA-Dehydrogenase die Aminosäuresequenz von SEQ ID NO: 2 hat, die Crotonase die Aminosäuresequenz von SEQ ID NO: 3 hat, die Butyryl-CoA-Dehydrogenase die Aminosäuresequenz von SEQ ID NO: 4 hat und die optionale Butyraldehyd-Dehydrogenase (AdhE2p) die Aminosäuresequenz von SEQ ID NO: 5 hat.

11. Genetisch manipulierte Hefe nach einem der voranstehenden Ansprüche, **dadurch gekennzeichnet, dass** die Butyraldehyd-Dehydrogenase und die Butanol-Dehydrogenase endogene Enzyme sind.

12. Genetisch manipulierte Hefe nach einem der voranstehenden Ansprüche, **dadurch gekennzeichnet, dass** die Acetyl-CoA-Acetyl-Transferase durch SEQ ID NO: 6 kodiert ist, die β-Hydroxy-butyryl-CoA-Dehydrogenase durch SEQ ID NO: 7 kodiert ist, die Crotonase durch SEQ ID NO: 8 kodiert ist, die Butyryl-CoA-Dehydrogenase durch SEQ ID NO: 9 kodiert ist und die optionale zusätzliche Butyraldehyd-Dehydrogenase durch SEQ ID NO: 10 kodiert ist.

13. Genetisch manipulierte Hefe nach einem der voranstehenden Ansprüche, **dadurch gekennzeichnet, dass** die Butyryl-CoA-Dehydrogenase eine Sequenz aufweist, die ein Enzym mit einer Homologie von wenigstens 90% bis 100% zu der Aminosäuresequenz kodiert, die durch SEQ ID NO: 19 kodiert wird.

14. Genetisch manipulierte Hefe nach einem der voranstehenden Ansprüche, **dadurch gekennzeichnet, dass** die Acetyl-CoA-Acetyl-Transferase eine Sequenz aufweist, die ein Enzym mit einer Homologie von wenigstens 90% bis 100% zu der Aminosäuresequenz kodiert, die durch SEQ ID NO: 21 kodiert wird.

15. Genetisch manipulierte Hefe nach einem der voranstehenden Ansprüche, **dadurch gekennzeichnet, dass** wenigstens ein Gen, das ein Stoffwechselenzym kodiert, inaktiviert ist, wobei das Gen eine Aminosäuresequenz kodiert, die eine Aminosäuresequenzhomologie von wenigstens 90% zu einer der Sequenzen aufweist, die in der Gruppe enthalten sind, die aus SEQ ID NO: 24 mit Pyruvat-Decarboxylaseaktivität, SEQ ID NO: 29 mit Isocitratlyaseaktivität, SEQ ID NO: 34 mit Acetyl-CoA-Hydrolaseaktivität, SEQ ID NO: 39 mit Acetyl-CoA-Hydrolaseaktivität, SEQ ID NO: 44 mit Acylcarnitin-Transferaseaktivität, SEQ ID NO: 49 mit Acylcarnitin-Transferaseaktivität, SEQ ID NO: 54 mit Carnitin-Acylcarnitin-Permeaseaktivität und SEQ ID NO: 59 mit Carnitin-Acylcarnitin-Permeaseaktivität besteht.

16. Verfahren zur Herstellung von Butanol aus einem Substrat durch Kultivieren einer Hefe in einer wässrigen Fermentationsbrühe, die das Substrat enthält, **dadurch gekennzeichnet, dass** die Hefe eine nach einem der voranstehenden Ansprüche ist und dass n-Butanol aus der Fermentationsbrühe isoliert wird.

17. Verfahren nach Anspruch 16, **dadurch gekennzeichnet, dass** das Kultivierungs- und Fermentationsverfahren bei einer Temperatur zwischen 10 bis 70 °C ausgeführt wird, vorzugsweise zwischen 25 bis 50 °C.

18. Verfahren nach einem der Ansprüche 16 bis 17, **dadurch gekennzeichnet, dass** das Kultivieren eine erste aerobe Phase in einer Fermentationsbrühe in Abwesenheit von Induktor umfasst und eine anschließende zweite Phase, die durch Zugabe des Induktors gestartet wird.

19. Verfahren nach einem der Ansprüche 16 bis 18, **dadurch gekennzeichnet, dass** die Kultivierungs- und Fermentationsverfahren als kontinuierliche Verfahren durchgeführt werden.

20. Verfahren nach einem der Ansprüche 16 bis 19, **dadurch gekennzeichnet, dass** das Substrat aus wässrigen Mischungen ausgewählt wird, die tierische Exkremente, Haushaltsabwässer, proteinhaltigen Abfall, Fette, Alkane, n-Alkane, Alkohole, Ethanol, Stärke, Lignocellulosen, Tannine, Pentose- und/oder Hexosezucker enthalten, die aus monomeren, oligomeren oder polymeren Zuckerresten bestehen und/oder aus Zuckeralkoholresten und Mischungen dieser.

## Revendications

1. Levure génétiquement manipulée contenant des séquences d'acides nucléiques hétérologues codantes pour enzymes du métabolisme pour effectuer la synthèse de n-butanol à partir d'un substrat, **caractérisée en ce que** les enzymes du métabolisme hétérologues comprennent une transférase acétyle-CoA-acétyle (Thlp), une déshydrogénase β-hydroxy butyryle-CoA (Hbdp), une crotonase (Crtp) et une déshydrogénase butyryle-CoA (Bdhp) et chaque séquence d'acides nucléiques codante est organisée en un module d'expression, dans laquelle levure la voie métabolique allant de l'acyle-carnitine vers la β-oxydation est altérée ou interrompue par mutation d'au moins l'un des gènes endogènes codants pour au moins l'une de la transférase acyle-cartinine peroxisomale, la perméase acyle-carnitine peroxisomale et/ou l'oxydase acyle-CoA, et/ou que la voie métabolique allant du phosphate de dihydroxyacétone au glycérol est altérée ou interrompue par mutation du gène codant pour la déshydrogénase de glycérol-3-phosphate.

2. Levure génétiquement manipulée selon la revendication 1, **caractérisée en ce que** les enzymes du métabolisme hétérologues se composent d'une transférase acétyle-CoA-acétyle (Thlp), une déshydrogénase β-hydroxy butyryle-CoA (Hbdp), d'une crotonase (Crtp) et d'un déshydrogénase butyryle-CoA (Bdhp).

3. Levure génétiquement manipulée selon les revendications 1 ou 2, **caractérisée en ce que** chaque module d'expression des séquences d'acides nucléiques hétérologues codantes pour les enzymes du métabolisme permettant la synthèse de butanol est chacun placé de façon indépendante sous le contrôle d'un promoteur qui est inductible par nitrate et/ou nitrite, d'un promoteur inductible par la chaleur et/ou d'un promoteur inductible par le substrat.

4. Levure génétiquement manipulée selon l'une des revendications précédentes, **caractérisée en ce que** la levure est du genre Arxula.

5. Levure génétiquement manipulée selon l'une des revendications précédentes, **caractérisé en ce que** la levure contient une séquence d'acides nucléiques hétérologues codant une déshydrogénase butyraldéhyde (AdhE2p).

6. Levure génétiquement manipulée selon l'une des revendications précédentes, **caractérisée en ce qu'**au moins l'un des gènes catalysant le métabolisme à partir de l'acétyle-CoA pour le cycle d'acide citrique ou le cycle du glyoxylate est altéré ou inactivé.

7. Levure génétiquement manipulée selon l'une des revendications précédentes, **caractérisée en ce qu'**au moins l'un des gènes catalysant le métabolisme à partir du pyruvate en acétaldéhyde est altéré ou inactivé.

8. Levure génétiquement manipulée selon l'une des revendications précédentes, **caractérisée en ce que** les gènes codant pour une enzyme métabolique catalysant le métabolisme à partir de l'acétyl-CoA en acétate et CoA sont altérés ou inactivés.

9. Levure génétiquement manipulée selon l'une des revendications précédentes, **caractérisée en ce que** chaque module d'expression contenant une séquence d'acides nucléiques codant pour une enzyme métabolique pour la synthèse de n-butanol contient le même promoteur.

10. Levure génétiquement manipulée selon l'une des revendications précédentes, **caractérisée en ce que** la transférase acétyle-CoA-acétyle présente la séquence d'acides aminés de la séquence SEQ ID NO: 1, la déshydrogénase β-hydroxy butyryle-CoA présente la séquence d'acides aminés de la séquence SEQ ID NO: 2, la crotonase présente la séquence d'acides aminés de la séquence SEQ ID NO: 3, la déshydrogénase butyryle-CoA présente la séquence d'acides aminés de la séquence SEQ ID NO: 4 et la déshydrogénase butyraldéhyde (AdhE2p) en option présente la séquence d'acides aminés de la séquence SEQ ID NO: 5.

11. Levure génétiquement manipulée selon l'une des revendications précédentes, **caractérisée en ce que** la déshydrogénase butyraldéhyde et la déshydrogénase butanol sont des enzymes endogènes.

12. Levure génétiquement manipulée selon l'une des revendications précédentes, **caractérisée en ce que** la transférase acétyle-CoA-acétyle est codée par la séquence SEQ ID NO: 6, la déshydrogénase β-hydroxy butyryle-CoA est codée par la séquence SEQ ID NO: 7, la crotonase est codée par la séquence SEQ ID NO: 8, la déshydrogénase butyryle-CoA est codée par la séquence SEQ ID NO: 9, la déshydrogénase butyraldéhyde supplémentaire éventuelle est codée par la séquence SEQ ID NO: 10.

13. Levure génétiquement manipulée selon l'une des revendications précédentes, **caractérisée en ce que** la déshydrogénase butyryle-CoA possède une séquence codante pour une enzyme ayant une homologie d'au moins 90% à 100% avec la séquence d'acides aminés codée par la séquence SEQ ID NO: 19.

14. Levure génétiquement manipulée selon l'une des revendications précédentes, **caractérisée en ce que** la transférase acétyle-CoA-acétyle possède une séquence codante pour une enzyme ayant une homologie d'au moins 90% à 100% avec la séquence d'acides aminés codée par la séquence SEQ ID NO: 21.

15. Levure génétiquement manipulée selon l'une des revendications précédentes, **caractérisée en ce qu'**au moins un gène codant pour une enzyme métabolique est inactivé, lequel gène code une séquence d'acides aminés ayant une homologie de séquence d'acides aminés d'au moins 90% avec l'une des séquences contenues dans le groupe composé des séquences SEQ ID NO: 24 ayant une activité décarboxylase pyruvate, SEQ ID NO: 29 ayant une activité lyase isocitrate, SEQ ID NO: 34 ayant une activité hydrolase acétyle-CoA, SEQ ID NO: 39 ayant une activité hydrolase acétyle-CoA, SEQ ID NO: 44 ayant une activité transférase acyle-carnitine, SEQ ID NO: 49 ayant une activité transférase acyle-carnitine, SEQ ID NO: 54 ayant une activité perméase carnitine-acylcarnitine et SEQ ID NO: 59 ayant une activité perméase carnitine-acylcarnitine.

16. Processus de production de n-butanol à partir d'un substrat par culture de levure dans un bouillon de fermentation aqueux contenant le substrat, **caractérisé en ce que** la levure est une levure selon l'une des revendications précédentes, et que le n-butanol est isolé du bouillon de fermentation.

17. Processus selon la revendication 16, **caractérisé en ce que** le processus de culture et de fermentation est effectué à une température comprise entre 10 à 70°C, de préférence entre 25 à 50°C.

18. Processus selon l'une quelconque des revendications 16 à 17, **caractérisé en ce que** la culture comprend une première phase aérobie dans un bouillon de fermentation en l'absence d'inducteur et une deuxième phase est commencée par addition d'inducteur.

19. Processus selon l'une quelconque des revendications 16 à 18, **caractérisé en ce que** les processus de culture et de fermentation sont effectués comme processus continus.

20. Processus selon l'une quelconque des revendications 16 à 19, **caractérisé en ce que** le substrat est sélectionné parmi les mélanges aqueux contenant des excréments d'animaux, les eaux usées domestiques, les déchets contenant des protéines, les matières grasses, les alcanes, les n-alcanes, les alcools, l'éthanol, l'amidon, les lignocelluloses, les tannins, les sucres pentose et/ou hexose qui se composent de groupements monomères, oligomères ou polymères et/ou de groupements d'alcool de sucre et de leurs mélanges.


### SEQUENCE LISTING

<110> ACS AGROCHEMISCHE SYSTEME GmbH Jaeckering Muehlen- und Naehrmittelwerke GmbH Leibniz-Institut fuer Pflanzengenetik und Kulturpflanzenfors chung
<120> Production of butanol by fermentation
<130> I1009PCT
<150> EP11161243
   <151> 2011-04-05
<160> 62
<170> BiSSAP 1.0
<210> 1
   <211> 392
   <212> PRT
   <213> Clostridium acetobutylicum
<220>
   <221> SOURCE
   <222> 1..392
   <223> /mol_type="protein" /organism="Clostridium acetobutylicum"
<400> 1
   Met Lys Glu Val Val Ile Ala Ser Ala Val Arg Thr Ala Ile Gly Ser
   1               5                   10                  15
   Tyr Gly Lys Ser Leu Lys Asp Val Pro Ala Val Asp Leu Gly Ala Thr
               20                  25                  30
   Ala Ile Lys Glu Ala Val Lys Lys Ala Gly Ile Lys Pro Glu Asp Val
           35                  40                  45
   Asn Glu Val Ile Leu Gly Asn Val Leu Gln Ala Gly Leu Gly Gln Asn
       50                  55                  60
   Pro Ala Arg Gln Ala Ser Phe Lys Ala Gly Leu Pro Val Glu Ile Pro
   65                  70                  75                  80
   Ala Met Thr Ile Asn Lys Val Cys Gly Ser Gly Leu Arg Thr Val Ser
                   85                  90                  95
   Leu Ala Ala Gln Ile Ile Lys Ala Gly Asp Ala Asp Val Ile Ile Ala
               100                 105                 110
   Gly Gly Met Glu Asn Met Ser Arg Ala Pro Tyr Leu Ala Asn Asn Ala
           115                 120                 125
   Arg Trp Gly Tyr Arg Met Gly Asn Ala Lys Phe Val Asp Glu Met Ile
       130                 135                 140
   Thr Asp Gly Leu Trp Asp Ala Phe Asn Asp Tyr His Met Gly Ile Thr
   145                 150                 155                 160
   Ala Glu Asn Ile Ala Glu Arg Trp Asn Ile Ser Arg Glu Glu Gln Asp
                   165                 170                 175
   Glu Phe Ala Leu Ala Ser Gln Lys Lys Ala Glu Glu Ala Ile Lys Ser
               180                 185                 190
   Gly Gln Phe Lys Asp Glu Ile Val Pro Val Val Ile Lys Gly Arg Lys
           195                 200                 205
   Gly Glu Thr Val Val Asp Thr Asp Glu His Pro Arg Phe Gly Ser Thr
       210                 215                 220
   Ile Glu Gly Leu Ala Lys Leu Lys Pro Ala Phe Lys Lys Asp Gly Thr
   225                 230                 235                 240
   Val Thr Ala Gly Asn Ala Ser Gly Leu Asn Asp Cys Ala Ala Val Leu
                   245                 250                 255
   Val Ile Met Ser Ala Glu Lys Ala Lys Glu Leu Gly Val Lys Pro Leu
               260                 265                 270
   Ala Lys Ile Val Ser Tyr Gly Ser Ala Gly Val Asp Pro Ala Ile Met
           275                 280                 285
   Gly Tyr Gly Pro Phe Tyr Ala Thr Lys Ala Ala Ile Glu Lys Ala Gly
       290                 295                 300
   Trp Thr Val Asp Glu Leu Asp Leu Ile Glu Ser Asn Glu Ala Phe Ala
   305                 310                 315                 320
   Ala Gln Ser Leu Ala Val Ala Lys Asp Leu Lys Phe Asp Met Asn Lys
                   325                 330                 335
   Val Asn Val Asn Gly Gly Ala Ile Ala Leu Gly His Pro Ile Gly Ala
               340                 345                 350
   Ser Gly Ala Arg Ile Leu Val Thr Leu Val His Ala Met Gln Lys Arg
           355                 360                 365
   Asp Ala Lys Lys Gly Leu Ala Thr Leu Cys Ile Gly Gly Gly Gln Gly
       370                 375                 380
   Thr Ala Ile Leu Leu Glu Lys Cys
   385                 390
<210> 2
   <211> 282
   <212> PRT
   <213> Clostridium acetobutylicum
<220>
   <221> SOURCE
   <222> 1..282
   <223> /mol_type="protein" /organism="Clostridium acetobutylicum"
<400> 2
   Met Lys Lys Val Cys Val Ile Gly Ala Gly Thr Met Gly Ser Gly Ile
   1               5                   10                  15
   Ala Gln Ala Phe Ala Ala Lys Gly Phe Glu Val Val Leu Arg Asp Ile
               20                  25                  30
   Lys Asp Glu Phe Val Asp Arg Gly Leu Asp Phe Ile Asn Lys Asn Leu
           35                  40                  45
   Ser Lys Leu Val Lys Lys Gly Lys Ile Glu Glu Ala Thr Lys Val Glu
       50                  55                  60
   Ile Leu Thr Arg Ile Ser Gly Thr Val Asp Leu Asn Met Ala Ala Asp
   65                  70                  75                  80
   Cys Asp Leu Val Ile Glu Ala Ala Val Glu Arg Met Asp Ile Lys Lys
                   85                  90                  95
   Gln Ile Phe Ala Asp Leu Asp Asn Ile Cys Lys Pro Glu Thr Ile Leu
               100                 105                 110
   Ala Ser Asn Thr Ser Ser Leu Ser Ile Thr Glu Val Ala Ser Ala Thr
           115                 120                 125
   Lys Arg Pro Asp Lys Val Ile Gly Met His Phe Phe Asn Pro Ala Pro
       130                 135                 140
   Val Met Lys Leu Val Glu Val Ile Arg Gly Ile Ala Thr Ser Gln Glu
   145                 150                 155                 160
   Thr Phe Asp Ala Val Lys Glu Thr Ser Ile Ala Ile Gly Lys Asp Pro
                   165                 170                 175
   Val Glu Val Ala Glu Ala Pro Gly Phe Val Val Asn Arg Ile Leu Ile
               180                 185                 190
   Pro Met Ile Asn Glu Ala Val Gly Ile Leu Ala Glu Gly Ile Ala Ser
           195                 200                 205
   Val Glu Asp Ile Asp Lys Ala Met Lys Leu Gly Ala Asn His Pro Met
       210                 215                 220
   Gly Pro Leu Glu Leu Gly Asp Phe Ile Gly Leu Asp Ile Cys Leu Ala
   225                 230                 235                 240
   Ile Met Asp Val Leu Tyr Ser Glu Thr Gly Asp Ser Lys Tyr Arg Pro
                   245                 250                 255
   His Thr Leu Leu Lys Lys Tyr Val Arg Ala Gly Trp Leu Gly Arg Lys
                   260                 265                 270
   Ser Gly Lys Gly Phe Tyr Asp Tyr Ser Lys
           275                 280
<210> 3
   <211> 261
   <212> PRT
   <213> Clostridium acetobutylicum
<220>
   <221> SOURCE
   <222> 1..261
   <223> /mol_type="protein" /organism="Clostridium acetobutylicum"
<400> 3
   Met Glu Leu Asn Asn Val Ile Leu Glu Lys Glu Gly Lys Val Ala Val
   1               5                   10                  15
   Val Thr Ile Asn Arg Pro Lys Ala Leu Asn Ala Leu Asn Ser Asp Thr
               20                  25                  30
   Leu Lys Glu Met Asp Tyr Val Ile Gly Glu Ile Glu Asn Asp Ser Glu
           35                  40                  45
   Val Leu Ala Val Ile Leu Thr Gly Ala Gly Glu Lys Ser Phe Val Ala
       50                  55                  60
   Gly Ala Asp Ile Ser Glu Met Lys Glu Met Asn Thr Ile Glu Gly Arg
   65                  70                  75                  80
   Lys Phe Gly Ile Leu Gly Asn Lys Val Phe Arg Arg Leu Glu Leu Leu
                   85                  90                  95
   Glu Lys Pro Val Ile Ala Ala Val Asn Gly Phe Ala Leu Gly Gly Gly
               100                 105                 110
   Cys Glu Ile Ala Met Ser Cys Asp Ile Arg Ile Ala Ser Ser Asn Ala
           115                 120                 125
   Arg Phe Gly Gln Pro Glu Val Gly Leu Gly Ile Thr Pro Gly Phe Gly
       130                 135                 140
   Gly Thr Gln Arg Leu Ser Arg Leu Val Gly Met Gly Met Ala Lys Gln
   145                 150                 155                 160
   Leu Ile Phe Thr Ala Gln Asn Ile Lys Ala Asp Glu Ala Leu Arg Ile
                   165                 170                 175
   Gly Leu Val Asn Lys Val Val Glu Pro Ser Glu Leu Met Asn Thr Ala
               180                 185                 190
   Lys Glu Ile Ala Asn Lys Ile Val Ser Asn Ala Pro Val Ala Val Lys
           195                 200                 205
   Leu Ser Lys Gln Ala Ile Asn Arg Gly Met Gln Cys Asp Ile Asp Thr
       210                 215                 220
   Ala Leu Ala Phe Glu Ser Glu Ala Phe Gly Glu Cys Phe Ser Thr Glu
   225                 230                 235                 240
   Asp Gln Lys Asp Ala Met Thr Ala Phe Ile Glu Lys Arg Lys Ile Glu
                   245                 250                 255
   Gly Phe Lys Asn Arg
               260
<210> 4
   <211> 379
   <212> PRT
   <213> Clostridium acetobutylicum
<220>
   <221> SOURCE
   <222> 1..379
   <223> /mol_type="protein" /organism="Clostridium acetobutylicum"
<400> 4
   Met Asp Phe Asn Leu Thr Arg Glu Gln Glu Leu Val Arg Gln Met Val
   1               5                   10                  15
   Arg Glu Phe Ala Glu Asn Glu Val Lys Pro Ile Ala Ala Glu Ile Asp
               20                  25                  30
   Glu Thr Glu Arg Phe Pro Met Glu Asn Val Lys Lys Met Gly Gln Tyr
           35                  40                  45
   Gly Met Met Gly Ile Pro Phe Ser Lys Glu Tyr Gly Gly Ala Gly Gly
       50                  55                  60
   Asp Val Leu Ser Tyr Ile Ile Ala Val Glu Glu Leu Ser Lys Val Cys
   65                  70                  75                  80
   Gly Thr Thr Gly Val Ile Leu Ser Ala His Thr Ser Leu Cys Ala Ser
                   85                  90                  95
   Leu Ile Asn Glu His Gly Thr Glu Glu Gln Lys Gln Lys Tyr Leu Val
               100                 105                 110
   Pro Leu Ala Lys Gly Glu Lys Ile Gly Ala Tyr Gly Leu Thr Glu Pro
           115                 120                 125
   Asn Ala Gly Thr Asp Ser Gly Ala Gln Gln Thr Val Ala Val Leu Glu
       130                 135                 140
   Gly Asp His Tyr Val Ile Asn Gly Ser Lys Ile Phe Ile Thr Asn Gly
   145                 150                 155                 160
   Gly Val Ala Asp Thr Phe Val Ile Phe Ala Met Thr Asp Arg Thr Lys
                   165                 170                 175
   Gly Thr Lys Gly Ile Ser Ala Phe Ile Ile Glu Lys Gly Phe Lys Gly
               180                 185                 190
   Phe Ser Ile Gly Lys Val Glu Gln Lys Leu Gly Ile Arg Ala Ser Ser
           195                 200                 205
   Thr Thr Glu Leu Val Phe Glu Asp Met Ile Val Pro Val Glu Asn Met
       210                 215                 220
   Ile Gly Lys Glu Gly Lys Gly Phe Pro Ile Ala Met Lys Thr Leu Asp
   225                 230                 235                 240
   Gly Gly Arg Ile Gly Ile Ala Ala Gln Ala Leu Gly Ile Ala Glu Gly
                   245                 250                 255
   Ala Phe Asn Glu Ala Arg Ala Tyr Met Lys Glu Arg Lys Gln Phe Gly
               260                 265                 270
   Arg Ser Leu Asp Lys Phe Gln Gly Leu Ala Trp Met Met Ala Asp Met
           275                 280                 285
   Asp Val Ala Ile Glu Ser Ala Arg Tyr Leu Val Tyr Lys Ala Ala Tyr
       290                 295                 300
   Leu Lys Gln Ala Gly Leu Pro Tyr Thr Val Asp Ala Ala Arg Ala Lys
   305                 310                 315                 320
   Leu His Ala Ala Asn Val Ala Met Asp Val Thr Thr Lys Ala Val Gln
                   325                 330                 335
   Leu Phe Gly Gly Tyr Gly Tyr Thr Lys Asp Tyr Pro Val Glu Arg Met
               340                 345                 350
   Met Arg Asp Ala Lys Ile Thr Glu Ile Tyr Glu Gly Thr Ser Glu Val
           355                 360                 365
   Gln Lys Leu Val Ile Ser Gly Lys Ile Phe Arg
       370                 375
<210> 5
   <211> 858
   <212> PRT
   <213> Clostridium acetobutylicum
<220>
   <221> SOURCE
   <222> 1..858
   <223> /mol_type="protein" /organism="Clostridium acetobutylicum"
<400> 5
   Met Lys Val Thr Asn Gln Lys Glu Leu Lys Gln Lys Leu Asn Glu Leu
   1               5                   10                  15
   Arg Glu Ala Gln Lys Lys Phe Ala Thr Tyr Thr Gln Glu Gln Val Asp
               20                  25                  30
   Lys Ile Phe Lys Gln Cys Ala Ile Ala Ala Ala Lys Glu Arg Ile Asn
           35                  40                  45
   Leu Ala Lys Leu Ala Val Glu Glu Thr Gly Ile Gly Leu Val Glu Asp
       50                  55                  60
   Lys Ile Ile Lys Asn His Phe Ala Ala Glu Tyr Ile Tyr Asn Lys Tyr
   65                  70                  75                  80
   Lys Asn Glu Lys Thr Cys Gly Ile Ile Asp His Asp Asp Ser Leu Gly
                   85                  90                  95
   Ile Thr Lys Val Ala Glu Pro Ile Gly Ile Val Ala Ala Ile Val Pro
               100                 105                 110
   Thr Thr Asn Pro Thr Ser Thr Ala Ile Phe Lys Ser Leu Ile Ser Leu
           115                 120                 125
   Lys Thr Arg Asn Ala Ile Phe Phe Ser Pro His Pro Arg Ala Lys Lys
       130                 135                 140
   Ser Thr Ile Ala Ala Ala Lys Leu Ile Leu Asp Ala Ala Val Lys Ala
   145                 150                 155                 160
   Gly Ala Pro Lys Asn Ile Ile Gly Trp Ile Asp Glu Pro Ser Ile Glu
                   165                 170                 175
   Leu Ser Gln Asp Leu Met Ser Glu Ala Asp Ile Ile Leu Ala Thr Gly
               180                 185                 190
   Gly Pro Ser Met Val Lys Ala Ala Tyr Ser Ser Gly Lys Pro Ala Ile
           195                 200                 205
   Gly Val Gly Ala Gly Asn Thr Pro Ala Ile Ile Asp Glu Ser Ala Asp
       210                 215                 220
   Ile Asp Met Ala Val Ser Ser Ile Ile Leu Ser Lys Thr Tyr Asp Asn
   225                 230                 235                 240
   Gly Val Ile Cys Ala Ser Glu Gln Ser Ile Leu Val Met Asn Ser Ile
                   245                 250                 255
   Tyr Glu Lys Val Lys Glu Glu Phe Val Lys Arg Gly Ser Tyr Ile Leu
               260                 265                 270
   Asn Gln Asn Glu Ile Ala Lys Ile Lys Glu Thr Met Phe Lys Asn Gly
           275                 280                 285
   Ala Ile Asn Ala Asp Ile Val Gly Lys Ser Ala Tyr Ile Ile Ala Lys
       290                 295                 300
   Met Ala Gly Ile Glu Val Pro Gln Thr Thr Lys Ile Leu Ile Gly Glu
   305                 310                 315                 320
   Val Gln Ser Val Glu Lys Ser Glu Leu Phe Ser His Glu Lys Leu Ser
                   325                 330                 335
   Pro Val Leu Ala Met Tyr Lys Val Lys Asp Phe Asp Glu Ala Leu Lys
               340                 345                 350
   Lys Ala Gln Arg Leu Ile Glu Leu Gly Gly Ser Gly His Thr Ser Ser
           355                 360                 365
   Leu Tyr Ile Asp Ser Gln Asn Asn Lys Asp Lys Val Lys Glu Phe Gly
       370                 375                 380
   Leu Ala Met Lys Thr Ser Arg Thr Phe Ile Asn Met Pro Ser Ser Gln
   385                 390                 395                 400
   Gly Ala Ser Gly Asp Leu Tyr Asn Phe Ala Ile Ala Pro Ser Phe Thr
                   405                 410                 415
   Leu Gly Cys Gly Thr Trp Gly Gly Asn Ser Val Ser Gln Asn Val Glu
               420                 425                 430
   Pro Lys His Leu Leu Asn Ile Lys Ser Val Ala Glu Arg Arg Glu Asn
           435                 440                 445
   Met Leu Trp Phe Lys Val Pro Gln Lys Ile Tyr Phe Lys Tyr Gly Cys
       450                 455                 460
   Leu Arg Phe Ala Leu Lys Glu Leu Lys Asp Met Asn Lys Lys Arg Ala
   465                 470                 475                 480
   Phe Ile Val Thr Asp Lys Asp Leu Phe Lys Leu Gly Tyr Val Asn Lys
                   485                 490                 495
   Ile Thr Lys Val Leu Asp Glu Ile Asp Ile Lys Tyr Ser Ile Phe Thr
               500                 505                 510
   Asp Ile Lys Ser Asp Pro Thr Ile Asp Ser Val Lys Lys Gly Ala Lys
           515                 520                 525
   Glu Met Leu Asn Phe Glu Pro Asp Thr Ile Ile Ser Ile Gly Gly Gly
       530                 535                 540
   Ser Pro Met Asp Ala Ala Lys Val Met His Leu Leu Tyr Glu Tyr Pro
   545                 550                 555                 560
   Glu Ala Glu Ile Glu Asn Leu Ala Ile Asn Phe Met Asp Ile Arg Lys
                   565                 570                 575
   Arg Ile Cys Asn Phe Pro Lys Leu Gly Thr Lys Ala Ile Ser Val Ala
               580                 585                 590
   Ile Pro Thr Thr Ala Gly Thr Gly Ser Glu Ala Thr Pro Phe Ala Val
           595                 600                 605
   Ile Thr Asn Asp Glu Thr Gly Met Lys Tyr Pro Leu Thr Ser Tyr Glu
       610                 615                 620
   Leu Thr Pro Asn Met Ala Ile Ile Asp Thr Glu Leu Met Leu Asn Met
   625                 630                 635                 640
   Pro Arg Lys Leu Thr Ala Ala Thr Gly Ile Asp Ala Leu Val His Ala
                   645                 650                 655
   Ile Glu Ala Tyr Val Ser Val Met Ala Thr Asp Tyr Thr Asp Glu Leu
               660                 665                 670
   Ala Leu Arg Ala Ile Lys Met Ile Phe Lys Tyr Leu Pro Arg Ala Tyr
           675                 680                 685
   Lys Asn Gly Thr Asn Asp Ile Glu Ala Arg Glu Lys Met Ala His Ala
       690                 695                 700
   Ser Asn Ile Ala Gly Met Ala Phe Ala Asn Ala Phe Leu Gly Val Cys
   705                 710                 715                 720
   His Ser Met Ala His Lys Leu Gly Ala Met His His Val Pro His Gly
                   725                 730                 735
   Ile Ala Cys Ala Val Leu Ile Glu Glu Val Ile Lys Tyr Asn Ala Thr
               740                 745                 750
   Asp Cys Pro Thr Lys Gln Thr Ala Phe Pro Gln Tyr Lys Ser Pro Asn
           755                 760                 765
   Ala Lys Arg Lys Tyr Ala Glu Ile Ala Glu Tyr Leu Asn Leu Lys Gly
       770                 775                 780
   Thr Ser Asp Thr Glu Lys Val Thr Ala Leu Ile Glu Ala Ile Ser Lys
   785                 790                 795                 800
   Leu Lys Ile Asp Leu Ser Ile Pro Gln Asn Ile Ser Ala Ala Gly Ile
                   805                 810                 815
   Asn Lys Lys Asp Phe Tyr Asn Thr Leu Asp Lys Met Ser Glu Leu Ala
               820                 825                 830
   Phe Asp Asp Gln Cys Thr Thr Ala Asn Pro Arg Tyr Pro Leu Ile Ser
           835                 840                 845
   Glu Leu Lys Asp Ile Tyr Ile Lys Ser Phe
       850                 855
<210> 6
   <211> 1179
   <212> DNA
   <213> Artificial Sequence
<220>
   <221> source
   <222> 1..1179
   <223> /mol_type="DNA" /note="acetyl-CoA-acetyl transferase (THL), optimized codon usage , SEQ ID NO 6" /organism="Artificial Sequence"
<400> 6
   atgaaggagg tggtcattgc ttctgctgtg cgaactgcta ttggatctta cggaaagtct      60
   ctgaaggacg tgcctgctgt ggacctggga gctactgcta ttaaggaggc tgtcaagaag     120
   gctggtatta agcctgagga cgtgaacgag gtgattctgg gaaacgtgct gcaggctgga     180
   ctgggacaga accctgctcg acaggcttct ttcaaggctg gactgcctgt cgagattcct     240
   gctatgacta ttaacaaggt gtgcggatct ggactgcgaa ctgtgtctct ggctgctcag     300
   attattaagg ctggtgacgc tgacgtgatt attgctggtg gaatggagaa catgtctcga     360
   gccccttacc tggctaacaa cgctcgatgg ggataccgaa tgggaaacgc taagttcgtg     420
   gacgagatga ttactgacgg actgtgggac gctttcaacg actaccacat gggaattact     480
   gctgagaaca ttgctgagcg atggaacatt tctcgagagg agcaggacga gttcgctctg     540
   gcttctcaga agaaggctga ggaggctatt aagtctggac agttcaagga cgagattgtg     600
   cctgtggtga ttaagggacg aaagggtgag actgtggtgg acactgacga gcaccctcga     660
   ttcggatcta ctattgaggg actggctaag ctgaagcctg ctttcaagaa ggacggaact     720
   gttactgctg gaaacgcttc tggactgaac gactgcgctg ctgtgctggt gattatgtct     780
   gctgagaagg ctaaggagct gggagtcaag cctctggcta agattgtgtc ttacggatct     840
   gctggtgtgg accctgctat tatgggatac ggacctttct acgctactaa ggctgctatt     900
   gagaaggctg gatggactgt ggacgagctg gacctgattg agtctaacga ggctttcgct     960
   gctcagtctc tggctgtggc taaggacctg aagttcgaca tgaacaaggt gaacgtgaac    1020
   ggtggtgcta ttgctctggg acaccctatt ggagcctctg gtgctcgaat tttggtgact    1080
   ctggtgcacg ctatgcagaa gcgagacgct aagaagggac tggccactct gtgcattgga    1140
   ggtggacagg gaactgctat tcttcttgag aagtgctaa                            1179
<210> 7
   <211> 849
   <212> DNA
   <213> Artificial Sequence
<220>
   <221> source
   <222> 1..849
   <223> /mol_type="DNA" /note="beta-hydroxy butyryl-CoA dehydrogenase (HBD), optimized co don usage, SEQ ID NO 7" /organism="Artificial Sequence"
<400> 7
   atgaagaagg tgtgcgtcat tggagctgga actatgggat ctggaattgc tcaggctttc 60
   gctgctaagg gattcgaggt ggtgctgcga gacattaagg acgagttcgt ggaccgagga 120
   ctggacttca ttaacaagaa cctgtctaag ctggtgaaga agggaaagat tgaggaggct 180
   actaaggtcg agattctgac tcgaatttct ggaactgtgg acctgaacat ggctgctgac 240
   tgcgacctgg tgattgaggc tgctgtcgag cgaatggaca ttaagaagca gattttcgct 300
   gacctggaca acatctgcaa gcctgagact attctggctt ctaacacttc ttctctgtct 360
   attactgagg tggcttctgc tactaagact aacgacaagg tgattggaat gcacttcttc 420
   aaccctgccc ctgtgatgaa gctggtcgag gtgattcgag gaattgctac ttctcaggag 480
   actttcgacg ctgtcaagga gacttctatt gctattggaa aggaccctgt cgaggtggct 540
   gaggctcctg gattcgtggt gaaccgaatt ttgattccta tgattaacga ggctgtggga 600
   attttggctg agggaattgc ttctgtcgag gacattgaca aggctatgaa gctgggagct 660
   aaccacccta tgggacctct tgagctggga gacttcattg gactggacat ttgcctggct 720
   attatggacg tgctgtactc tgagactggt gactctaagt accgacctca cactctgctg 780
   aagaagtacg tccgagctgg atggctggga cgaaagtctg gaaagggatt ctacgactac 840
   tccaagtaa 849
<210> 8
   <211> 786
   <212> DNA
   <213> Artificial Sequence
<220>
   <221> source
   <222> 1..786
   <223> /mol_type="DNA" /note="crotonase (CRT), optimized codon usage, SEQ ID NO 8" /organism="Artificial Sequence"
<400> 8
   atggagctga acaacgtgat tcttgagaag gagggaaagg tcgctgtcgt gactattaac      60
   cgacctaagg ctctgaacgc cctgaactct gacactctga aggagatgga ctacgtcatt     120
   ggagagattg agaacgactc tgaggtgctg gctgtgattc tgactggtgc tggtgagaag     180
   tctttcgtgg ctggtgctga catttctgag atgaaggaga tgaacactat tgagggacga     240
   aagttcggaa ttttgggaaa caaggtgttc cgacgacttg agctgcttga gaagcctgtg     300
   attgctgctg tgaacggatt cgctctggga ggtggatgcg agattgctat gtcttgcgat     360
   attcgaattg cttcttctaa cgctcgattc ggacagcctg aggtgggact gggaattact     420
   cctggattcg gaggaactca gcgactgtct cgactggtgg gaatgggaat ggctaagcag     480
   ctgattttca ctgctcagaa cattaaggct gacgaggctc tgcgaattgg actggtgaac     540
   aaggtggtcg agccttctga gctgatgaac actgctaagg agattgctaa caagattgtg     600
   tctaacgctc ctgtggctgt gaagctgtct aagcaggcta ttaaccgagg aatgcagtgc     660
   gacattgaca ctgctctggc tttcgagtct gaggctttcg gagagtgctt ctctactgag     720
   gaccagaagg acgctatgac tgctttcatt gagaagcgaa agattgaggg attcaagaac     780
   cgataa                                                                786
<210> 9
   <211> 1140
   <212> DNA
   <213> Artificial Sequence
<220>
   <221> source
   <222> 1..1140
   <223> /mol_type="DNA" /note="butyryl-CoA dehydrogenase (BDH), optimized codon usage, SEQ ID NO 9" /organism="Artificial Sequence"
<400> 9
   atggacttca acctgactcg agagcaggag ctggtgcgac agatggtgcg agagttcgct      60
   gagaacgagg tgaagcctat tgctgctgag attgacgaga ctgagcgatt ccctatggag     120
   aacgtgaaga agatgggaca gtacggaatg atgggtattc cattctcgaa ggagtacggt     180
   ggtgctggtg gtgacgtgct gtcttacatt attgctgtcg aggagctgtc taaggtgtgc     240
   ggaactactg gtgtgattct gtctgctcac acttctctgt gcgcttctct gattaacgag     300
   cacggaactg aggagcagaa gcagaagtac ctggtccctc tggctaaggg tgagaagatt     360
   ggagcttacg gactgactga gcctaacgct ggaactgact ctggtgctca gcagactgtg     420
   gctgtgcttg agggtgacca ctacgtgatt aacggatcta agattttcat cactaacggt     480
   ggtgtggctg acactttcgt gattttcgct atgactgacc gaactaaggg tactaaggga     540
   atttctgcct tcattattga gaagggattc aagggatttt ccattggaaa ggtcgagcag     600
   aagctgggta ttcgagcttc ttctactact gagctggtgt tcgaggacat gattgtgcct     660
   gtcgagaaca tgattggaaa ggagggaaag ggattcccta ttgctatgaa gactctggac     720
   ggtggacgaa ttggaattgc tgctcaggct ctgggaattg ctgagggtgc tttcaacgag     780
   gctcgagctt acatgaagga gcgaaagcag ttcggacgat ctctggacaa gttccaggga     840
   ctggcttgga tgatggctga catggacgtg gctattgagt ctgctcgata cctggtgtac     900
   aaggctgctt acctgaagca ggctggactg ccttacactg tggacgctgc tcgagctaag     960
   ctgcacgctg ctaacgtggc tatggacgtg actactaagg ctgtgcagct gttcggagga    1020
   tacggataca ctaaggacta ccctgtcgag cgaatgatgc gagatgctaa gattactgag    1080
   atctacgagg gaacttctga ggtgcagaag ctggtcattt ctggaaagat tttccgataa    1140
<210> 10
   <211> 2577
   <212> DNA
   <213> Artificial Sequence
<220>
   <221> source
   <222> 1..2577
   <223> /mol_type="DNA" /note="butyraldehyde dehydrogenase (ADHE2), optimized codon usage , SEQ ID NO 10" /organism="Artificial Sequence"
<400> 10
   atgaaggtca ctaaccagaa ggagctgaag cagaagctga acgagctgcg agaggctcag 60
   aagaagttcg ctacttacac tcaggagcag gtcgataaga ttttcaagca gtgcgctatt 120
   gctgctgcta aggagcgaat caacctggct aagctggctg tcgaggagac tggaattgga 180
   ctggtcgagg acaagattat taagaaccac ttcgctgctg agtacatcta caacaagtac 240
   aagaacgaga agacctgcgg aattattgac cacgacgact ccctgggaat tactaaggtc 300
   gctgagccta ttggaattgt cgctgctatt gtccctacta ccaaccctac ttctactgct 360
   attttcaagt ctctgatttc ccttaagacc cgaaacgcta ttttcttctc gcctcaccct 420
   cgagctaaga agtctactat tgctgccgcc aagctgattc tggacgctgc tgtcaaggct 480
   ggtgctccta agaacattat tggatggatt gacgagcctt ctattgagct gtctcaggac 540
   ctgatgtctg aggctgacat tattctggct actggtggac cttctatggt caaggctgct 600
   tactcttctg gaaagcctgc tattggagtc ggagctggaa acactcctgc tattattgac 660
   gagtctgctg acattgacat ggctgtctct tctattattc tgtccaagac ttacgacaac 720
   ggtgtcattt gcgcttccga gcagtctatt ctggtcatga actctatcta cgagaaggtc 780
   aaggaggagt tcgtcaagcg aggatcttac attctgaacc agaacgagat tgctaagatc 840
   aaggagacta tgttcaagaa cggtgctatt aacgctgaca ttgtcggaaa gtctgcttac 900
   attattgcta agatggctgg aattgaggtc cctcagacta ctaagattct gattggagag 960
   gtccagtctg tcgagaagtc tgagctgttc tctcacgaga agctgtctcc cgtcctggct 1020
   atgtacaagg tcaaggactt cgacgaggct ctgaagaagg ctcagcgatt gattgagctg 1080
   ggaggatctg gacacacttc ttctctgtac attgactctc agaacaacaa ggacaaggtg 1140
   aaggagttcg gactggctat gaagacttct cgaaccttca ttaacatgcc ttcgtcccag 1200
   ggtgcttctg gtgacctgta caacttcgct attgctcctt ctttcactct gggatgcgga 1260
   acttggggag gaaactctgt ctctcagaac gtcgagccta agcaccttct gaacattaag 1320
   tctgtcgctg agcgacgaga gaacatgctg tggttcaagg tgcctcagaa gatctacttc 1380
   aagtacggat gcctgcgatt cgctctgaag gagcttaagg acatgaacaa gaagcgagct 1440
   ttcattgtca ctgacaagga cctgttcaag ctgggatacg tcaacaagat tactaaggtt 1500
   ctggacgaga ttgacatcaa gtactctatt ttcaccgaca ttaagtccga ccctactatt 1560
   gactctgtca agaagggtgc taaggagatg ctgaacttcg agcctgacac tattatttct 1620
   attggaggtg gatctcctat ggacgctgct aaggtcatgc acctgttgta cgagtaccct 1680
   gaggctgaga ttgagaacct ggctattaac ttcatggaca ttcgaaagcg aatctgcaac 1740
   ttccctaagc tgggaactaa ggctatttct gtcgctattc ctactactgc tggaactgga 1800
   tctgaggcta ctcctttcgc tgtcattacc aacgacgaga ctggaatgaa gtaccctctg 1860
   acttcttacg agctgactcc taacatggct attattgata ctgagctgat gctgaacatg 1920
   cctcgaaagc tgactgctgc tactggaatt gacgctctgg tccacgctat tgaggcttac 1980
   gtgtctgtca tggctactga ctacactgac gagctggctc tgcgagctat taagatgatt 2040
   ttcaagtacc tgcctcgagc ttacaagaac ggaactaacg acattgaggc tcgagagaag 2100
   atggctcacg cttctaacat tgctggaatg gctttcgcta acgctttcct gggagtctgc 2160
   cactctatgg ctcacaagct gggagctatg caccacgtcc ctcacggaat tgcttgcgct 2220
   gtcctgattg aggaggtcat taagtacaac gctactgact gccctactaa gcagactgca 2280
   ttccctcagt acaagtctcc taacgctaag cgaaagtacg ctgagattgc tgagtacctg 2340
   aacctgaagg gaacttctga cactgagaag gtcactgctc tgattgaggc tatttctaag 2400
   ctgaagattg acctgtctat tccccagaac atttctgctg ctggtattaa caagaaggac 2460
   ttctacaaca ctctggacaa gatgtctgag ctggctttcg acgaccagtg cactactgct 2520
   aaccctcgat accctctgat ttctgagctg aaggacatct acattaagtc cttctag 2577
<210> 11
   <211> 885
   <212> DNA
   <213> Artificial Sequence
<220>
   <221> source
   <222> 1..885
   <223> /mol_type="DNA" /note="promoter element AYNR1, SEQ ID NO 11" /organism="Artificial Sequence"
<400> 11
   agcagtgatt tagcagcttc tagtcccagc agaccaccgc cgacgacaat ggcgcgcttt 60
   cctttagtct caaacttgtc ggtagaaaag cgaatcattt cctgaagatc atccactgtg 120
   cgatagacaa aaattccatg cgaacgagat tcctccacgg atggctcgta tccaagggac 180
   cgagagatga tgtccgatgg tagaactgcc cggcttcctg tagccatgat taggagatca 240
   tagctatatt cgtgccccaa gttagtcaaa accttcttgg tcttagtatc aatgctagtg 300
   acgggctcgt taactcggca caccatatgg tgtggatcta tgctttcata ccaggcagtg 360
   tcattgagat agaggtcatt gactgactgg tgcgaaaagt agtcggccaa tcccactcgg 420
   ttgtaggcca aatacttctc ttctcctaga atggtaacgg aatattctct ggttaataga 480
   tccctttcca gaagctgttc cacaaacgac atgccaacca ttccaaagcc caccaggaca 540
   atttgctttg gcggagtggc caatccgttg tgtgttattg tcatggatcc tgtaagtgtg 600
   tagtcaagca attgcaaaac gttgaccagg atgatccaag taataaatta ctttaccctt 660
   atcatgctta tcgtccactc gggtggcctc ggggtttttc gaaccgaact gataagatag 720
   tgccccattt tcgggacgaa agacgccgat ccgagataag acccctcgga acatggtgat 780
   aagtaatcct cgcaaataga ttggacagtt tgacaatgat tagagttttg gagaatatat 840
   agacgttatc agtacatggg gggggatagt tggcattgac tattg 885
<210> 12
   <211> 465
   <212> DNA
   <213> Artificial Sequence
<220>
   <221> source
   <222> 1..465
   <223> /mol_type="DNA" /note="promoter element AYNR1Ã⁻Â¿Â½, SEQ ID NO 12" /organism="Artificial Sequence"
<400> 12
   ttgtaggcca aatacttctc ttctcctaga atggtaacgg aatattctct ggttaataga 60
   tccctttcca gaagctgttc cacaaacgac atgccaacca ttccaaagcc caccaggaca 120
   atttgctttg gcggagtggc caatccgttg tgtgttattg tcatggatcc tgtaagtgtg 180
   tagtcaagca attgcaaaac gttgaccagg atgatccaag taataaatta ctttaccctt 240
   atcatgctta tcgtccactc gggtggcctc ggggtttttc gaaccgaact gataagatag 300
   tgccccattt tcgggacgaa agacgccgat ccgagataag acccctcgga acatggtgat 360
   aagtaatcct cgcaaataga ttggacagtt tgacaatgat tagagttttg gagaatatat 420
   agacgttatc agtacatggg gggggatagt tggcattgac tattg 465
<210> 13
   <211> 599
   <212> DNA
   <213> Artificial Sequence
<220>
   <221> source
   <222> 1..599
   <223> /mol_type="DNA" /note="promoter element AYNI1, SEQ ID NO 13" /organism="Artificial Sequence"
<400> 13
   tctcaaccat gccatcaatc tcaatctccc agtccagcac gtcgttcgta gattcaggta      60
   ccggtccatg gttacggaca aagtggagcg acgagggtgt aatgaatcca gcgttgtaca     120
   acaagtccaa aggtgcctca gaattaaacg gatgagctcc agtcaacctc acgagccgtt     180
   cgtcccgagg aacatgcgag tctggtgtct tcttgtcgag ttccagcaca tgttcagctt     240
   tggacggtcc ttgaaacgct ggcccggtca gaattttttc aactgtttgg tattctggtg      300
   ccatcaatag tcaatgccaa ctatcccccc ccatgtactg ataacgtcta tatattctcc     360
   aaaactctaa tcattgtcaa actgtccaat ctatttgcga ggattactta tcaccatgtt     420
   ccgaggggtc ttatctcgga tcggcgtctt tcgtcccgaa aatggggcac tatcttatca     480
   gttcggttcg aaaaaccccg aggccacccg agtggacgat aagcatgata agggtaaagt     540
   aatttattac ttggatcatc ctggtcaacg ttttgcaatt gcttgactac acacttaca      599
<210> 14
   <211> 299
   <212> DNA
   <213> Artificial Sequence
<220>
   <221> source
   <222> 1..299
   <223> /mol_type="DNA" /note="promoter element AYNI1Ã⁻Â¿Â½, SEQ ID NO 14" /organism="Artificial Sequence"
<400> 14
   ccatcaatag tcaatgccaa ctatcccccc ccatgtactg ataacgtcta tatattctcc      60
   aaaactctaa tcattgtcaa actgtccaat ctatttgcga ggattactta tcaccatgtt     120
   ccgaggggtc ttatctcgga tcggcgtctt tcgtcccgaa aatggggcac tatcttatca     180
   gttcggttcg aaaaaccccg aggccacccg agtggacgat aagcatgata agggtaaagt     240
   aatttattac ttggatcatc ctggtcaacg ttttgcaatt gcttgactac acacttaca      299
<210> 15
   <211> 663
   <212> DNA
   <213> Artificial Sequence
<220>
   <221> source
   <222> 1..663
   <223> /mol_type="DNA" /note="promoter element AYNT1, SEQ ID NO 15" /organism="Artificial Sequence"
<400> 15
   cttcggaaac agatatgagg atgatatttt atgcaaaagc gaattgtgca gatttgaaca      60
   aatgacgcag gggcggatcc ggataaagct ctggctatct ggcaaaatgg accaatgcaa     120
   tactcagcat actcccggaa ggatcgatct gcaaataatg gatcgtgagc tgtcgcacat     180
   gaaggagggt cacgattcaa tgctgctagt atgtggacca gaaggaatgg tggagactgc     240
   taaaacatgg caaaagtcca ggaacattga agattttagg gtagtctatt tttaagcata     300
   taacagatat tttaagaaaa ttggcatagc tctacttcac ttttgtaacg acagctcgga     360
   tcctggtaat accgagtttt aaagaaaaat gaattgttca catggaccat gcagtatctg     420
   ataaagcata gataacgtta agtgagtgcg caccgtcctt aacctcggtt cgggtcaatg     480
   aaaaccgagc gaaagcggaa ttttcagacc gagacaccct tatcaggtcg ttatcagaac     540
   cttatctcga tcttatcagc tataacgctc agcggggtcg tatgcaaggg acaggtttaa     600
   ataatgtctc ggaattggca tataataaat accgttgaaa ctttcataac tacagcatag     660
   aca                                                                   663
<210> 16
   <211> 331
   <212> DNA
   <213> Artificial Sequence
<220>
   <221> source
   <222> 1..331
   <223> /mol_type="DNA" /note="promoter element AYNT1Ã⁻Â¿Â½, SEQ ID NO 16" /organism="Artificial Sequence"
<400> 16
   tacttcactt ttgtaacgac agctcggatc ctaccgagtt ttaaagaaaa atgaattgtt      60
   cacatggacc atgcagtatc tgataaagca ttggtaaaga taacgttaag tgagtgcgca     120
   ccgtccttaa cctcggttcg ggtcaatgaa aaccgagcga aagcggaatt ttcagaccga     180
   gacaccctta tcaggtcgtt atcagaacct tatctcgatc ttatcagcta taacgctcag     240
   cggggtcgta tgcaagggac aggtttaaat aatgtctcgg aattggcata taataaatac     300
   cgttgaaact ttcataacta cagcatagac a                                    331
<210> 17
   <211> 839
   <212> DNA
   <213> Artificial Sequence
<220>
   <221> source
   <222> 1..839
   <223> /mol_type="DNA" /note="promoter element AYNR1-2, SEQ ID NO 17" /organism="Artificial Sequence"
<400> 17
   agcagtgatt tagcagcttc tagtcccagc agaccaccgc cgacgacaat ggcgcgcttt      60
   cctttagtct caaacttgtc ggtagaaaag cgaatcattt cctgaagatc atccactgtg     120
   cgatagacaa aaattccatg cgaacgagat tcctccacgg atggctcgta tccaagggac     180
   cgagagatga tgtccgatgg tagaactgcc cggcttcctg tagccatgat taggagatca     240
   tagctatatt cgtgccccaa gttagtcaaa accttcttgg tcttagtatc aatgctagtg     300
   acgggctcgt taactcggca caccatatgg tgtggatcta tgctttcata ccaggcagtg     360
   tcattgagat agaggtcatt gactgactgg tgcgaaaagt agtcggccaa tcccactcgg     420
   ttgtaggcca aatacttctc ttctcctaga atggtaacgg aatattctct ggttaataga     480
   tccctttcca gaagctgttc cacaaacgac atgccaacca ttccaaagcc caccaggaca     540
   atttgctttg gcggagtggc caatccgttg tctgttattg tcattgtaag tgtgtagtca     600
   agcaattgca aaacgttgac caggatgtcc actcgggtgg cctcggggtt tttcgaaccg     660
   aactgataag atagtgcccc attttcggga cgaaagacgc cgatccgaga taagacccct     720
   cggaacatgg tgataagtaa tcctcgcaaa tagattggac agtttgacaa tgattagagt     780
   tttggagaat atatagacgt tatcagtaca tgggggggga tagttggcat tgactattg      839
<210> 18
   <211> 420
   <212> DNA
   <213> Artificial Sequence
<220>
   <221> source
   <222> 1..420
   <223> /mol_type="DNA" /note="promoter element AYNR1Ã⁻Â¿Â½-2, SEQ ID NO 18" /organism="Artificial Sequence"
<400> 18
   gttgtaggcc aaatacttct cttctcctag aatggtaacg gaatattctc tggttaatag 60
   atccctttcc agaagctgtt ccacaaacga catgccaacc attccaaagc ccaccaggac 120
   aatttgcttt ggcggagtgg ccaatccgtt gtctgttatt gtcattgtaa gtgtgtagtc 180
   aagcaattgc aaaacgttga ccaggatgtc cactcgggtg gcctcggggt ttttcgaacc 240
   gaactgataa gatagtgccc cattttcggg acgaaagacg ccgatccgag ataagacccc 300
   tcggaacatg gtgataagta atcctcgcaa atagattgga cagtttgaca atgattagag 360
   ttttggagaa tatatagacg ttatcagtac atgggggggg atagttggca ttgactattg 420
<210> 19
   <211> 1194
   <212> DNA
   <213> Treponema denticola
<220>
   <221> source
   <222> 1..1194
   <223> /mol_type="DNA" /note="codon-optimized acetyl-CoA reductase" /organism="Treponema denticola"
<220>
   <221> CDS
   <222> 1..1194
   <223> /gene="trans-enoyl-CoA-reductase" /transl_table=11
<400> 19
   atgattgtga agcctatggt gcgaaacaac atttgcctga acgctcaccc tcagggatgc      60
   aagaagggtg tcgaggacca gattgagtac actaagaagc gaattactgc tgaggtgaag     120
   gctggtgcta aggctcctaa gaacgtgctg gtgctgggat gctctaacgg atacggactg     180
   gcttctcgaa ttactgccgc tttcggatac ggtgctgcta ctattggagt gtctttcgag     240
   aaggctggat ctgagactaa gtacggaact cctggatggt acaacaacct ggctttcgac     300
   gaggctgcta agcgagaggg actgtactct gtgactattg acggtgacgc tttctctgac     360
   gagattaagg ctcaggtgat tgaggaggct aagaagaagg gtattaagtt cgacctgatt     420
   gtgtactctc tggcttctcc tgtgcgaact gaccctgaca ctggaattat gcacaagtct     480
   gtgctgaagc ctttcggaaa gactttcact ggaaagactg tggacccttt cactggtgag     540
   ctgaaggaga tttctgctga gcctgctaac gacgaggagg ctgctgctac tgtcaaggtg     600
   atgggaggtg aggactggga gcgatggatt aagcagctgt ctaaggaggg actgcttgag     660
   gagggatgca ttactctggc ttactcttac attggacctg aggctactca ggctctgtac     720
   cgaaagggaa ctattggaaa ggctaaggag caccttgagg ctactgctca ccgactgaac     780
   aaggagaacc cttctattcg agctttcgtg tctgtgaaca agggactggt gactcgagct     840
   tctgctgtga ttcctgtcat tcctctgtac ctggcttctc tgttcaaggt gatgaaggag     900
   aagggaaacc acgagggatg tattgagcag attactcgac tgtacgctga gcgactttac     960
   cgaaaggacg gaactattcc tgtggacgag gagaaccgaa tccgaattga cgactgggag    1020
   cttgaggagg acgtgcagaa ggctgtctct gctctgatgg agaaggtgac tggtgagaac    1080
   gctgagtctc tgactgacct ggctggatac cgacacgact ttctggcttc taacggattc    1140
   gacgtcgagg gtattaacta cgaggctgag gtcgagcgat tcgaccgaat ctaa          1194
<210> 20
   <211> 397
   <212> PRT
   <213> Treponema denticola
<220>
   <221> SOURCE
   <222> 1..397
   <223> /mol_type="protein" /note="This sequence is generated from feature [CDS]:1..1194 plac ed under sequence number 19" /note="[CDS]:1..1194 from SEQ ID NO 19" /organism="Treponema denticola"
<400> 20
   Met Ile Val Lys Pro Met Val Arg Asn Asn Ile Cys Leu Asn Ala His
   1               5                   10                  15
   Pro Gln Gly Cys Lys Lys Gly Val Glu Asp Gln Ile Glu Tyr Thr Lys
               20                  25                  30
   Lys Arg Ile Thr Ala Glu Val Lys Ala Gly Ala Lys Ala Pro Lys Asn
           35                  40                  45
   Val Leu Val Leu Gly Cys Ser Asn Gly Tyr Gly Leu Ala Ser Arg Ile
       50                  55                  60
   Thr Ala Ala Phe Gly Tyr Gly Ala Ala Thr Ile Gly Val Ser Phe Glu
   65                  70                  75                  80
   Lys Ala Gly Ser Glu Thr Lys Tyr Gly Thr Pro Gly Trp Tyr Asn Asn
                   85                  90                  95
   Leu Ala Phe Asp Glu Ala Ala Lys Arg Glu Gly Leu Tyr Ser Val Thr
               100                 105                 110
   Ile Asp Gly Asp Ala Phe Ser Asp Glu Ile Lys Ala Gln Val Ile Glu
           115                 120                 125
   Glu Ala Lys Lys Lys Gly Ile Lys Phe Asp Leu Ile Val Tyr Ser Leu
       130                 135                 140
   Ala Ser Pro Val Arg Thr Asp Pro Asp Thr Gly Ile Met His Lys Ser
   145                 150                 155                 160
   Val Leu Lys Pro Phe Gly Lys Thr Phe Thr Gly Lys Thr Val Asp Pro
                   165                 170                 175
   Phe Thr Gly Glu Leu Lys Glu Ile Ser Ala Glu Pro Ala Asn Asp Glu
               180                 185                 190
   Glu Ala Ala Ala Thr Val Lys Val Met Gly Gly Glu Asp Trp Glu Arg
           195                 200                 205
   Trp Ile Lys Gln Leu Ser Lys Glu Gly Leu Leu Glu Glu Gly Cys Ile
       210                 215                 220
   Thr Leu Ala Tyr Ser Tyr Ile Gly Pro Glu Ala Thr Gln Ala Leu Tyr
   225                 230                 235                 240
   Arg Lys Gly Thr Ile Gly Lys Ala Lys Glu His Leu Glu Ala Thr Ala
                   245                 250                 255
   His Arg Leu Asn Lys Glu Asn Pro Ser Ile Arg Ala Phe Val Ser Val
               260                 265                 270
   Asn Lys Gly Leu Val Thr Arg Ala Ser Ala Val Ile Pro Val Ile Pro
           275                 280                 285
   Leu Tyr Leu Ala Ser Leu Phe Lys Val Met Lys Glu Lys Gly Asn His
       290                 295                 300
   Glu Gly Cys Ile Glu Gln Ile Thr Arg Leu Tyr Ala Glu Arg Leu Tyr
   305                 310                 315                 320
   Arg Lys Asp Gly Thr Ile Pro Val Asp Glu Glu Asn Arg Ile Arg Ile
                   325                 330                 335
   Asp Asp Trp Glu Leu Glu Glu Asp Val Gln Lys Ala Val Ser Ala Leu
               340                 345                 350
   Met Glu Lys Val Thr Gly Glu Asn Ala Glu Ser Leu Thr Asp Leu Ala
           355                 360                 365
   Gly Tyr Arg His Asp Phe Leu Ala Ser Asn Gly Phe Asp Val Glu Gly
       370                 375                 380
   Ile Asn Tyr Glu Ala Glu Val Glu Arg Phe Asp Arg Ile
   385                 390                 395
<210> 21
   <211> 1245
   <212> DNA
   <213> Blastobotrys adeninivorans
<220>
   <221> source
   <222> 1..1245
   <223> /mol_type="DNA" /note="acetyl-CoA-acetyltransferase" /organism="Blastobotrys adeninivorans"
<220>
   <221> CDS
   <222> 1..1245
   <223> /gene="acetyl-CoA-acetyl transferase" /transl_table=11
<400> 21
   atgagactaa gcgctgctgc cactgtggcg aaacgatctt tccacacttc aagaatggtc      60
   caaaatgtat acatcctttc tggcgctcga actgccattg gtaactttgg cggttccctc     120
   aagagcgttt cggccacaga cctcggtgcc gtcgcagcaa aggctgccat tgagcgagct     180
   ggaatcaagc ctgagaacgt agaggactgt tactttggaa atgtactatc ggctaacctt     240
   ggacaggccc ccgcccgtca ggtggtcatc aaggctggac tgcccgaaac taccgaggca     300
   actaccatca acaaggtgtg cgcatctggt ctcaagaccg tgtccctggc agctcagtcc     360
   attgctctgg gtgatcgtga tgttgtgctt actggaggaa tggagtccat gtctaacact     420
   ccattctact tccctcgaaa tgccgctttt ggccaccagc aggctaagga tgccattgta     480
   ggcgatggat tgtgggatgt gtacaaccag atccacatgg gttcgtgctg cgagaacact     540
   aaccgccgag agggaattac tcgacaggat caggatgact ttgctcttga gtcctaccga     600
   cgagctaatg ccgccattga cgccggtgag tttgatgccg agattgcccc tgtgactatc     660
   aagtccaaga agggcgatgt agtggtcaag actgacgaat ctccaaagtc gctcaagcct     720
   gaagctgttc ccaagctccg acccgcattt gagaaggacg gaaccgtcac tgctgctaat     780
   gcctcttctc tgaacgacgg tgctgccgct ctggtgttgg cttctggcac caaggctaag     840
   gagctcggag ctaacgttct tggacgagtt gtcggctacg ctgatgctgc tctatcccca     900
   attgacttta ctgttgctcc ttctaaggcc attcctttgg ctcttaagaa ggcgggacta     960
   accaaggacg acattgcaaa gtgggagatc aatgaggcct tctctggagt atccattgcc    1020
   aataacaagc tgctgggtct tgaccccagc aaagtcaacg tcaagggtgg agctgtggct    1080
   ctgggacacc ccattggtgc ttcaggagct cgtattcttg tcactctttt gcaccttctc    1140
   aaggatggtg agtatggagt cgccggtatc tgcaatggag gtggagctgc cactgccgtc    1200
   gtagttcaga aggtctctgc tgttggaggc gattccaagt tgtaa                    1245
<210> 22
   <211> 414
   <212> PRT
   <213> Blastobotrys adeninivorans
<220>
   <221> SOURCE
   <222> 1..414
   <223> /mol_type="protein" /note="[CDS]:1..1245 from SEQ ID NO 21" /note="This sequence is generated from feature [CDS]:1..1245 plac ed under sequence number 21" /organism="Blastobotrys adeninivorans"
<400> 22
   Met Arg Leu Ser Ala Ala Ala Thr Val Ala Lys Arg Ser Phe His Thr
   1               5                   10                  15
   Ser Arg Met Val Gln Asn Val Tyr Ile Leu Ser Gly Ala Arg Thr Ala
               20                  25                  30
   Ile Gly Asn Phe Gly Gly Ser Leu Lys Ser Val Ser Ala Thr Asp Leu
           35                  40                  45
   Gly Ala Val Ala Ala Lys Ala Ala Ile Glu Arg Ala Gly Ile Lys Pro
       50                  55                  60
   Glu Asn Val Glu Asp Cys Tyr Phe Gly Asn Val Leu Ser Ala Asn Leu
   65                  70                  75                  80
   Gly Gln Ala Pro Ala Arg Gln Val Val Ile Lys Ala Gly Leu Pro Glu
                   85                  90                  95
   Thr Thr Glu Ala Thr Thr Ile Asn Lys Val Cys Ala Ser Gly Leu Lys
               100                 105                 110
   Thr Val Ser Leu Ala Ala Gln Ser Ile Ala Leu Gly Asp Arg Asp Val
           115                 120                 125
   Val Leu Thr Gly Gly Met Glu Ser Met Ser Asn Thr Pro Phe Tyr Phe
       130                 135                 140
   Pro Arg Asn Ala Ala Phe Gly His Gln Gln Ala Lys Asp Ala Ile Val
   145                 150                 155                 160
   Gly Asp Gly Leu Trp Asp Val Tyr Asn Gln Ile His Met Gly Ser Cys
                   165                 170                 175
   Cys Glu Asn Thr Asn Arg Arg Glu Gly Ile Thr Arg Gln Asp Gln Asp
               180                 185                 190
   Asp Phe Ala Leu Glu Ser Tyr Arg Arg Ala Asn Ala Ala Ile Asp Ala
           195                 200                 205
   Gly Glu Phe Asp Ala Glu Ile Ala Pro Val Thr Ile Lys Ser Lys Lys
       210                 215                 220
   Gly Asp Val Val Val Lys Thr Asp Glu Ser Pro Lys Ser Leu Lys Pro
   225                 230                 235                 240
   Glu Ala Val Pro Lys Leu Arg Pro Ala Phe Glu Lys Asp Gly Thr Val
                   245                 250                 255
   Thr Ala Ala Asn Ala Ser Ser Leu Asn Asp Gly Ala Ala Ala Leu Val
               260                 265                 270
   Leu Ala Ser Gly Thr Lys Ala Lys Glu Leu Gly Ala Asn Val Leu Gly
           275                 280                 285
   Arg Val Val Gly Tyr Ala Asp Ala Ala Leu Ser Pro Ile Asp Phe Thr
       290                 295                 300
   Val Ala Pro Ser Lys Ala Ile Pro Leu Ala Leu Lys Lys Ala Gly Leu
   305                 310                 315                 320
   Thr Lys Asp Asp Ile Ala Lys Trp Glu Ile Asn Glu Ala Phe Ser Gly
                   325                 330                 335
   Val Ser Ile Ala Asn Asn Lys Leu Leu Gly Leu Asp Pro Ser Lys Val
               340                 345                 350
   Asn Val Lys Gly Gly Ala Val Ala Leu Gly His Pro Ile Gly Ala Ser
           355                 360                 365
   Gly Ala Arg Ile Leu Val Thr Leu Leu His Leu Leu Lys Asp Gly Glu
       370                 375                 380
   Tyr Gly Val Ala Gly Ile Cys Asn Gly Gly Gly Ala Ala Thr Ala Val
   385                 390                 395                 400
   Val Val Gln Lys Val Ser Ala Val Gly Gly Asp Ser Lys Leu
                   405                 410
<210> 23
   <211> 1758
   <212> DNA
   <213> Blastobotrys adeninivorans
<220>
   <221> source
   <222> 1..1758
   <223> /mol_type="DNA" /organism="Blastobotrys adeninivorans"
<220>
   <221> CDS
   <222> 1..1758
   <223> /gene="pyruvate decarboxylase" /transl_table=1
<400> 23
   gtgtggggta ccttattccg cggcggggta tatatacatg acgtgagcac tggagcggta      60
   gaatcggtca tgtcagctat tccactcggt cagtatctat tcacgcgcct caagcaacta     120
   gatgttggca ctgtgtttgg tgcccctgga gactttaacc tttcgctatt agaccatctt     180
   tataatgttc cagatatgtg ctgggcaggt agtacaaatg agctcaacgg cggatacgcg     240
   gccgatgggt atgctcgtat tagaagacta ggagcaattg tgacgacatt tggagtaggg     300
   gagctgtcag ctataaatgc tctagcgggg gcctattctg agcatgttgg agtcgtccac     360
   attgtggggt acccttcgac gtctgcgcag gacagcaaac gcatgatcca tcacaccttg     420
   ggaaatggtg actttcgggc ctttcatcgc atgagctccg aagtgtcaat tgtttcccat     480
   gttctggatg atattgagaa agcaccgggg cagattgacg atgcgttgcg ggcggcatat     540
   gtgcaccagc gcccagtgta ccttggcatt ccgtccaatt ttgctgacat ggacgtgccc     600
   tccgacctcc taagtgcccc aattgatctg tcattagaat caaatccgac ccatgcggaa     660
   aaggaggtac tgagtcgcgt agtagagctt atcaacaaag cacaaaggcc tgtaattctc     720
   gtggatgcgt tcgcagtacg tcatggcgtt ggtaacgaaa caaggcagtt tgcagacaag     780
   accaattttc cagtgtttgc tactcctact ggtcgttcaa tagtcgatga aactaaccca     840
   aggtttggtg gaatttacat tggtttcctt tcgaatcctg atgtcaaggc agtcgtagag     900
   acggccgatc tggtactttc aataggacca atgccctccg atttcaatac tgcctccttt     960
   tcatacgagt ttagcactga caatgtggtc gagtttcatt ttgatcatac aactgtatgc    1020
   tccatggact atggcggagt gaaaatggcc catctgctcc caaggctctc tgaaaagctc    1080
   acatccaagg aactggccaa gccgttggtg ccagtaccat caattcccac agatgttccc    1140
   tctgtcaaga gcacagaccc cataaatcat gaatggctgt ggcctagact aagtggttgg    1200
   cttcgtcctg gagatattct cctgtcagag tcaggaacct ctcattttgg tacactgaga    1260
   accaagcttc caagtggagc tgctgcaatt acttccttca tgtgggctgc aattggttat    1320
   actgttgggg ctgcttttgg agctgccatg gcagcaaggg atgtgggaga cgatagaagg    1380
   gtcgttcttt ttgtaggcga tggatcactg caaatgactg ctactgaagt gtcctcaatg    1440
   gtacgctggg gtttaaaacc gtacatcttt gttatcaaca atgatggata tacagtggaa    1500
   agactgattc atggtgaacg ccaaacctac aatgaccttc acatgtggca gtatacaaag    1560
   atggtcgacc tttacaacgc taagcagggc gaagctcacc tggtggagag cactgcggat    1620
   ttggaatctt tatttagttc caatgacttc agtgaggcgt ctaaatttcg agttatcgag    1680
   gtcaaaatgc cattgatgga tgcgccttcg aatataaaga tgcaaactgc aaaaatggcg    1740
   gcagccaatg caaagaaa                                                  1758
<210> 24
   <211> 586
   <212> PRT
   <213> Blastobotrys adeninivorans
<220>
   <221> SOURCE
   <222> 1..586
   <223> /mol_type="protein" /note="[CDS]:1..1758 from SEQ ID NO 23" /note="This sequence is generated from feature [CDS]:1..1758 plac ed under sequence number 23" /organism="Blastobotrys adeninivorans"
<400> 24
   Val Trp Gly Thr Leu Phe Arg Gly Gly Val Tyr Ile His Asp Val Ser
   1               5                   10                  15
   Thr Gly Ala Val Glu Ser Val Met Ser Ala Ile Pro Leu Gly Gln Tyr
               20                  25                  30
   Leu Phe Thr Arg Leu Lys Gln Leu Asp Val Gly Thr Val Phe Gly Ala
           35                  40                  45
   Pro Gly Asp Phe Asn Leu Ser Leu Leu Asp His Leu Tyr Asn Val Pro
       50                  55                  60
   Asp Met Cys Trp Ala Gly Ser Thr Asn Glu Leu Asn Gly Gly Tyr Ala
   65                  70                  75                  80
   Ala Asp Gly Tyr Ala Arg Ile Arg Arg Leu Gly Ala Ile Val Thr Thr
                   85                  90                  95
   Phe Gly Val Gly Glu Leu Ser Ala Ile Asn Ala Leu Ala Gly Ala Tyr
               100                 105                 110
   Ser Glu His Val Gly Val Val His Ile Val Gly Tyr Pro Ser Thr Ser
           115                 120                 125
   Ala Gln Asp Ser Lys Arg Met Ile His His Thr Leu Gly Asn Gly Asp
       130                 135                 140
   Phe Arg Ala Phe His Arg Met Ser Ser Glu Val Ser Ile Val Ser His
   145                 150                 155                 160
   Val Leu Asp Asp Ile Glu Lys Ala Pro Gly Gln Ile Asp Asp Ala Leu
                   165                 170                 175
   Arg Ala Ala Tyr Val His Gln Arg Pro Val Tyr Leu Gly Ile Pro Ser
               180                 185                 190
   Asn Phe Ala Asp Met Asp Val Pro Ser Asp Leu Leu Ser Ala Pro Ile
           195                 200                 205
   Asp Leu Ser Leu Glu Ser Asn Pro Thr His Ala Glu Lys Glu Val Leu
       210                 215                 220
   Ser Arg Val Val Glu Leu Ile Asn Lys Ala Gln Arg Pro Val Ile Leu
   225                 230                 235                 240
   Val Asp Ala Phe Ala Val Arg His Gly Val Gly Asn Glu Thr Arg Gln
                   245                 250                 255
   Phe Ala Asp Lys Thr Asn Phe Pro Val Phe Ala Thr Pro Thr Gly Arg
               260                 265                 270
   Ser Ile Val Asp Glu Thr Asn Pro Arg Phe Gly Gly Ile Tyr Ile Gly
           275                 280                 285
   Phe Leu Ser Asn Pro Asp Val Lys Ala Val Val Glu Thr Ala Asp Leu
       290                 295                 300
   Val Leu Ser Ile Gly Pro Met Pro Ser Asp Phe Asn Thr Ala Ser Phe
   305                 310                 315                 320
   Ser Tyr Glu Phe Ser Thr Asp Asn Val Val Glu Phe His Phe Asp His
                   325                 330                 335
   Thr Thr Val Cys Ser Met Asp Tyr Gly Gly Val Lys Met Ala His Leu
               340                 345                 350
   Leu Pro Arg Leu Ser Glu Lys Leu Thr Ser Lys Glu Leu Ala Lys Pro
           355                 360                 365
   Leu Val Pro Val Pro Ser Ile Pro Thr Asp Val Pro Ser Val Lys Ser
       370                 375                 380
   Thr Asp Pro Ile Asn His Glu Trp Leu Trp Pro Arg Leu Ser Gly Trp
   385                 390                 395                 400
   Leu Arg Pro Gly Asp Ile Leu Leu Ser Glu Ser Gly Thr Ser His Phe
                   405                 410                 415
   Gly Thr Leu Arg Thr Lys Leu Pro Ser Gly Ala Ala Ala Ile Thr Ser
               420                 425                 430
   Phe Met Trp Ala Ala Ile Gly Tyr Thr Val Gly Ala Ala Phe Gly Ala
           435                 440                 445
   Ala Met Ala Ala Arg Asp Val Gly Asp Asp Arg Arg Val Val Leu Phe
       450                 455                 460
   Val Gly Asp Gly Ser Leu Gln Met Thr Ala Thr Glu Val Ser Ser Met
   465                 470                 475                 480
   Val Arg Trp Gly Leu Lys Pro Tyr Ile Phe Val Ile Asn Asn Asp Gly
                   485                 490                 495
   Tyr Thr Val Glu Arg Leu Ile His Gly Glu Arg Gln Thr Tyr Asn Asp
               500                 505                 510
   Leu His Met Trp Gln Tyr Thr Lys Met Val Asp Leu Tyr Asn Ala Lys
           515                 520                 525
   Gln Gly Glu Ala His Leu Val Glu Ser Thr Ala Asp Leu Glu Ser Leu
       530                 535                 540
   Phe Ser Ser Asn Asp Phe Ser Glu Ala Ser Lys Phe Arg Val Ile Glu
   545                 550                 555                 560
   Val Lys Met Pro Leu Met Asp Ala Pro Ser Asn Ile Lys Met Gln Thr
                   565                 570                 575
   Ala Lys Met Ala Ala Ala Asn Ala Lys Lys
               580                 585
<210> 25
   <211> 4006
   <212> DNA
   <213> Blastobotrys adeninivorans
<220>
   <221> source
   <222> 1..4006
   <223> /mol_type="DNA" /note="amplified genomic section" /organism="Blastobotrys adeninivorans"
<220>
   <221> gene
   <222> 1262..3019
   <223> /gene="pyruvate decarboxylase"
<400> 25
   agtcatttta tggacccggt ggtccttatg ccaactttgc tggacacgac gcttcgcgag 60
   ggttggcaaa gaactcgttt gatcccagca tgatcacccc tgtgaacgag cctatcgaca 120
   acttgagtga tctggatgat gaggagcgca aggctcttga cgagtggttt aaccatttcg 180
   aggcaaagta cccaatttgc ggtgagcttg tcaacgagtc tgccaagtca aagtagagct 240
   atttaaatta ttcaatccat ttggccaatg ctcggtaata gtaacccaat attgaaatta 300
   aagaagcgta gtgacactag aaattcacgc atgtaatcac gatttcatag gttatagact 360
   aaggatagga caacacgcca taggcatatc cattcctacg agtctctgag tgtcaatcca 420
   aacggtgctt gaagtgaagg gagaattagc cagtggatac tgacatcaac tccatcgaaa 480
   ggattcaatt ctgttcaatt aatggcaggt ctgccagaga agggccgtac tgtcgctgag 540
   ctttccgggt tcgtgccccc cccccccccc cccccccccc ccagctgcca gtaaagtcta 600
   taaaaacttt aatgggtcaa ataccatcaa ctatccactc tgctagagtg caaatctttt 660
   aaggtgatgg tctcttgaga gttttcaagt gttttcacta ctggccacat cttaacaccg 720
   cccccacaac cattcacaac agctacggcc atatctaggt gaaaacacga cttcccgtcc 780
   gatcagtcat agttaagcac ctaagagcca tacccagtaa tgcggtcgga gacgacgcgt 840
   gaacagatgg tgctgtagtt gaactttttt tgttgtcttc atcctggaca tgtatctcct 900
   agacgatggg ccgttacacg gctgagagta caatattgta tgacgcggag atactagtca 960
   gttgcaacca gaacaaccaa tttgggggcc cgacattgtc tttacatttt acagcccgta 1020
   tgctttattt cattgaaaat taatctgatt gttgtgcgtg tccgtaacgg aacgataccg 1080
   ggaacgttgt tgcgccgcac ctttatctta tcagtcaata gccaaatggg caaccgaggg 1140
   ccaaagggat ataccaaatc tcaccctggc gatcaaatca ccatatactg tgctgaagca 1200
   gacagggtat tgcgagaact gctacagagt gcctgaacat attcatcgga actgttatcc 1260
   gacgtgtggg gtaccttatt ccgcggcggg gtatatatac atgacgtgag cactggagcg 1320
   gtagaatcgg tcatgtcagc tattccactc ggtcagtatc tattcacgcg cctcaagcaa 1380
   ctagatgttg gcactgtgtt tggtgcccct ggagacttta acctttcgct attagaccat 1440
   ctttataatg ttccagatat gtgctgggca ggtagtacaa atgagctcaa cggcggatac 1500
   gcggccgatg ggtatgctcg tattagaaga ctaggagcaa ttgtgacgac atttggagta 1560
   ggggagctgt cagctataaa tgctctagcg ggggcctatt ctgagcatgt tggagtcgtc 1620
   cacattgtgg ggtacccttc gacgtctgcg caggacagca aacgcatgat ccatcacacc 1680
   ttgggaaatg gtgactttcg ggcctttcat cgcatgagct ccgaagtgtc aattgtttcc 1740
   catgttctgg atgatattga gaaagcaccg gggcagattg acgatgcgtt gcgggcggca 1800
   tatgtgcacc agcgcccagt gtaccttggc attccgtcca attttgctga catggacgtg 1860
   ccctccgacc tcctaagtgc cccaattgat ctgtcattag aatcaaatcc gacccatgcg 1920
   gaaaaggagg tactgagtcg cgtagtagag cttatcaaca aagcacaaag gcctgtaatt 1980
   ctcgtggatg cgttcgcagt acgtcatggc gttggtaacg aaacaaggca gtttgcagac 2040
   aagaccaatt ttccagtgtt tgctactcct actggtcgtt caatagtcga tgaaactaac 2100
   ccaaggtttg gtggaattta cattggtttc ctttcgaatc ctgatgtcaa ggcagtcgta 2160
   gagacggccg atctggtact ttcaatagga ccaatgccct ccgatttcaa tactgcctcc 2220
   ttttcatacg agtttagcac tgacaatgtg gtcgagtttc attttgatca tacaactgta 2280
   tgctccatgg actatggcgg agtgaaaatg gcccatctgc tcccaaggct ctctgaaaag 2340
   ctcacatcca aggaactggc caagccgttg gtgccagtac catcaattcc cacagatgtt 2400
   ccctctgtca agagcacaga ccccataaat catgaatggc tgtggcctag actaagtggt 2460
   tggcttcgtc ctggagatat tctcctgtca gagtcaggaa cctctcattt tggtacactg 2520
   agaaccaagc ttccaagtgg agctgctgca attacttcct tcatgtgggc tgcaattggt 2580
   tatactgttg gggctgcttt tggagctgcc atggcagcaa gggatgtggg agacgataga 2640
   agggtcgttc tttttgtagg cgatggatca ctgcaaatga ctgctactga agtgtcctca 2700
   atggtacgct ggggtttaaa accgtacatc tttgttatca acaatgatgg atatacagtg 2760
   gaaagactga ttcatggtga acgccaaacc tacaatgacc ttcacatgtg gcagtataca 2820
   aagatggtcg acctttacaa cgctaagcag ggcgaagctc acctggtgga gagcactgcg 2880
   gatttggaat ctttatttag ttccaatgac ttcagtgagg cgtctaaatt tcgagttatc 2940
   gaggtcaaaa tgccattgat ggatgcgcct tcgaatataa agatgcaaac tgcaaaaatg 3000
   gcggcagcca tgcaaagaaa tgatgtacac tttgataagt tcaattaagc ctacatggga 3060
   gactataggt aacaattata ctacagtttg taatacaata agaaacagaa gaaaggatat 3120
   taagcaatgc tcacttcagt cagatgtcct ccaacgccca tgccctcatg ggatccggaa 3180
   attgcaattt ccacaaatcg agcattgaag gtcgaattaa ggcggagaga agtgtcattt 3240
   cctgggcgca tcacgatttc actcatgtta gtgccgttct tccagggatt ggaaagagaa 3300
   atgctgtggt tcgagacaat ccaatggacc ttcgacacat tctctgcctg agagacatta 3360
   gatccgtgga atccctgggg aataccaacc gacagagatt ttggaggatt ggcaccccag 3420
   aggaaataga gggaagagaa agattgagat gaacccagat cgactgtaat actcgactca 3480
   gacccattga atggttgcca gaaggtagag ttgtcaccat cgttggcaga gaatggaaag 3540
   tgccccgaag tccatggctc cttgctaccc acaggtacac actgggcgac atttccttcg 3600
   aatagagtgg agtttttgtc agatcgataa gtgggaatgg tcagagtatc gttcacccta 3660
   agactaaagt tgcctgcctt ttcattgcga gaaccaattt ggacggtaat gggaccagac 3720
   gtgacattat ccttcgacat tgacaatgac tcaatcttat ttggccgtct tgtaatagtc     3780
   gtgtttgtaa gtgtcacatt aacgtcaaat acgcctccct ggaatttgag tcccttgagc     3840
   atatagccat ccttgagctg agggggaagt gaaggatcta gataaagcga atcttcgcgc     3900
   gggcggaatc cagtgaaccc gtgagtgaaa gtttgcaggt aaccaccatg tccggtcaag     3960
   aatgggaatg ctgggttggt acctccatta atagtagcat tatcca                    4006
<210> 26
   <211> 3441
   <212> DNA
   <213> Artificial Sequence
<220>
   <221> source
   <222> 1..3441
   <223> /mol_type="DNA" /note="synthetic construct" /organism="Artificial Sequence"
<220>
   <221> CDS
   <222> 1607..2323
   <223> /gene="TRP" /transl_table=11
<400> 26
   ggagtcattt tatggacccg gtggtcctta tgccaacttt gctggacacg acgcttcgcg        60
   agggttggca aagaactcgt ttgatcccag catgatcacc cctgtgaacg agcctatcga       120
   caacttgagt gatctggatg atgaggagcg caaggctctt gacgagtggt ttaaccattt       180
   cgaggcaaag tacccaattt gcggtgagct tgtcaacgag tctgccaagt caaagtagag       240
   ctatttaaat tattcaatcc atttggccaa tgctcggtaa tagtaaccca atattgaaat       300
   taaagaagcg tagtgacact agaaattcac gcatgtaatc acgatttcat aggttataga       360
   ctaaggatag gacaacacgc cataggcata tccattccta cgagtctctg agtgtcaatc       420
   caaacggtgc ttgaagtgaa gggagaatta gccagtggat actgacatca actccatcga       480
   aaggattcaa ttctgttcaa ttaatggcag gtctgccaga gaagggccgt actgtcgctg       540
   agctttccgg gttcgtgccc cccccccccc cccccccccc ccccagctgc cagtaaagtc       600
   tataaaaact ttaatgggtc aaataccatc aactatccac tctgctagag tgcaaatctt       660
   ttaaggtgat ggtctcttga gagttttcaa gtgttttcac tactggccac atcttaacac       720
   cgcccccaca accattcaca acagctacgg ccatatctag gtgaaaacac gacttcccgt       780
   ccgatcagtc atagttaagc acctaagagc catacccagt aatgcggtcg gagacgacgc       840
   gtgaacagat ggtgctgtag ttgaactttt tttgttgtct tcatcctgga catgtatctc       900
   ctagacgatg ggccgttaca cggctgagag tacaatattg tatgacgcgg agatactagt 960
   ggatcccccg ggctgcagga attcgatatc gtgtctagat acagtcccta ttaattgtga 1020
   ggaaacccct tagcgctttc aatcgacgaa ttgcaattga caaagagaga acatgctctt 1080
   aagactgaac atcttatcag acaagggccg catcgagcgg ctacctcgta attgtggtca 1140
   gtcattgaca accattagca agccattggc aacgggacaa cgcaggcgaa tctcactcgc 1200
   agttactgcc tgcaatgaca attgctcctc ttcgcgcaaa cataatagac tttgataagc 1260
   accacaatcg cttcttccct cggtcgtact ggcaaatttc atgcatgaat gcggctgcag 1320
   cctcctgtcg atatgggacg gagctctgtt gctttcggcg ccctttccaa gcttcaatcc 1380
   actaatcgga gaatgttcat caactggctc agctaatcag ataggtcaac ttgttcccca 1440
   gcggtttcac cacatgctgt cctttcctcc ccccgctcgg aaggatacgc agaatgcttt 1500
   atcgtggggc tgccccgggc cgaaaccggc attttgggtt gactctttta aggaacgaca 1560
   tgcccagggg actctccact cacaaaccct ttcaaaaacc tgtacaatga caacgtcgcc 1620
   aatcgtcaag gtgtgcggaa tcaggaccgt ggaggcggcc caggtcgctt tggatgctgg 1680
   agccaacatg attggaatga ttatggtccc taatagagcg cgcactgttg atatggagac 1740
   agcaggcaag atatcacgac tggtacgatc atatagacgt caaggatcct ttgagctgga 1800
   cactaatgag ctgggcggat cgactcactc tgcactcgcc cacatcattg aagccaagac 1860
   aaggacccgg ccagcaatcg taggcgtgtt ccgcaaccag ccgctgtcag aagtcctgcg 1920
   gttgcaacag gaattgtcgc tggatttcgt tcagcttcac ggatcagagc ccctagaatg 1980
   gtgtagactg attccatgtc ctgtgatccg tcggttcacc cccgatactc cagatttcta 2040
   tgcctcgccg gttactggat accacagcct atccctcata gacggtgagg ttggcggaga 2100
   gggcaagatg gtggactgga gcggagtcca gtctcgggcg cgaatgggag cgaggttcct 2160
   tatggctgga ggattgacag ccgacaatgt tgcccaagct ctagcggtcg agggtgcctg 2220
   tggtgttgat gtcagcggag gagtcgaatc aagcccgggg gtcaaagatc tcaacgagat 2280
   tgagcggttt gtcaaagctg ccaagggggt tactgtgaag tattaacact ccagcagcag 2340
   catcaatagg tgatactgac gacaggtaat gtgtactaat aggtctgaca gcagaataca 2400
   gctatattac ttctcatttg aaacttgagg tgattgacaa tttactattt atacaagcta 2460
   agcttcaata cagactcgat cgaattcagg tcgacaacta taggtaacaa ttatactaca 2520
   gtttgtaata caataagaaa cagaagaaag gatattaagc aatgctcact tcagtcagat 2580
   gtcctccaac gcccatgccc tcatgggatc cggaaattgc aatttccaca aatcgagcat 2640
   tgaaggtcga attaaggcgg agagaagtgt catttcctgg gcgcatcacg atttcactca 2700
   tgttagtgcc gttcttccag ggattggaaa gagaaatgct gtggttcgag acaatccaat 2760
   ggaccttcga cacattctct gcctgagaga cattagatcc gtggaatccc tggggaatac     2820
   caaccgacag agattttgga ggattggcac cccagaggaa atagagggaa gagaaagatt     2880
   gagatgaacc cagatcgact gtaatactcg actcagaccc attgaatggt tgccagaagg     2940
   tagagttgtc accatcgttg gcagagaatg gaaagtgccc cgaagtccat ggctccttgc     3000
   tacccacagg tacacactgg gcgacatttc cttcgaatag agtggagttt ttgtcagatc     3060
   gataagtggg aatggtcaga gtatcgttca ccctaagact aaagttgcct gccttttcat     3120
   tgcgagaacc aatttggacg gtaatgggac cagacgtgac attatccttc gacattgaca     3180
   atgactcaat cttatttggc cgtcttgtaa tagtcgtgtt tgtaagtgtc acattaacgt     3240
   caaatacgcc tccctggaat ttgagtccct tgagcatata gccatccttg agctgagggg     3300
   gaagtgaagg atctagataa agcgaatctt cgcgcgggcg gaatccagtg aacccgtgag     3360
   tgaaagtttg caggtaacca ccatgtccgg tcaagaatgg gaatgctggg ttggtacctc     3420
   cattaatagt agcattatcc a                                               3441
<210> 27
   <211> 239
   <212> PRT
   <213> Artificial Sequence
<220>
   <221> SOURCE
   <222> 1..239
   <223> /mol_type="protein" /note="This sequence is generated from feature [CDS]:1607..2323 p laced under sequence number 26" /note="[CDS]:1607..2323 from SEQ ID NO 26" /organism="Artificial Sequence"
<400> 27
   Met Thr Thr Ser Pro Ile Val Lys Val Cys Gly Ile Arg Thr Val Glu
   1               5                   10                  15
   Ala Ala Gln Val Ala Leu Asp Ala Gly Ala Asn Met Ile Gly Met Ile
               20                  25                  30
   Met Val Pro Asn Arg Ala Arg Thr Val Asp Met Glu Thr Ala Gly Lys
           35                  40                  45
   Ile Ser Arg Leu Val Arg Ser Tyr Arg Arg Gln Gly Ser Phe Glu Leu
       50                  55                  60
   Asp Thr Asn Glu Leu Gly Gly Ser Thr His Ser Ala Leu Ala His Ile
   65                  70                  75                  80
   Ile Glu Ala Lys Thr Arg Thr Arg Pro Ala Ile Val Gly Val Phe Arg
                   85                  90                  95
   Asn Gln Pro Leu Ser Glu Val Leu Arg Leu Gln Gln Glu Leu Ser Leu
               100                 105                 110
   Asp Phe Val Gln Leu His Gly Ser Glu Pro Leu Glu Trp Cys Arg Leu
           115                 120                 125
   Ile Pro Cys Pro Val Ile Arg Arg Phe Thr Pro Asp Thr Pro Asp Phe
       130                 135                 140
   Tyr Ala Ser Pro Val Thr Gly Tyr His Ser Leu Ser Leu Ile Asp Gly
   145                 150                 155                 160
   Glu Val Gly Gly Glu Gly Lys Met Val Asp Trp Ser Gly Val Gln Ser
                   165                 170                 175
   Arg Ala Arg Met Gly Ala Arg Phe Leu Met Ala Gly Gly Leu Thr Ala
                   180                 185                 190
   Asp Asn Val Ala Gln Ala Leu Ala Val Glu Gly Ala Cys Gly Val Asp
               195                 200                 205
   Val Ser Gly Gly Val Glu Ser Ser Pro Gly Val Lys Asp Leu Asn Glu
       210                 215                 220
   Ile Glu Arg Phe Val Lys Ala Ala Lys Gly Val Thr Val Lys Tyr
   225                 230                 235
<210> 28
   <211> 1641
   <212> DNA
   <213> Blastobotrys adeninivorans
<220>
   <221> source
   <222> 1..1641
   <223> /mol_type="DNA" /organism="Blastobotrys adeninivorans"
<220>
   <221> CDS
   <222> 1..1641
   <223> /gene="isocitrate lyase" /transl_table=1
<400> 28
   atgtcttcta aacaagagat tcttaaacaa gaagaagcgc aattccaaaa gcaggtcaag      60
   gacctcaagc agtggtggtc cgagcctcga tggagacaca ccaagcgtat ctaccctgct     120
   gaggatattg ctcgaaagcg aggaaacctc gaggttcgac acccttcctc tacccaggct     180
   gacaagctgt tcaagctcct ggagcagcac gacaagaaca agactgcatc catcacctac     240
   ggatgtctgg accccgttca gctgacccag atggcaaagt acttggacac tgtgtacgtt     300
   tctggatggc aatcatcttc tactgcctct acttccaacg agccttctcc cgatcttgct     360
   gattacccca tggacactgt gccaaacaag gtgcagcagc tgtggtttgc ccagttgttc     420
   cacgatcgca agcagcgaga ggatcgtctg acccagccca tcgaaaagcg cggagacctg     480
   cccaacattg actatctgag acctattatt gctgatgccg atactggtca cggaggtctg     540
   tctgctatcg tcaagctgac caagatgttc attgagcgcg gtgctgctgg aatccacatt     600
   gaggaccaga tgcccggtac caagaagtgt ggacacatgg ccggaaaggt gctggtgccc     660
   gtccaggaac acattaaccg tctggtggcc attcgtctcg ccgccgacct gtttggctct     720
   aaccttcttg ccattgcccg aactgactct gaggccgcta ctctcatcac ctccactgtc     780
   gactaccggg atcattactt tattgttgga tccaccaacc ctgatgtgcc tcctttggta     840
   gacctgctgg tagaggccga gcagaaggga ctccagggtg ctgctctgga gcgaatcgag     900
   gccgagtggg tcaagaaggc ccagcctaag ctgttcgccg atgctgtcat tgacgagatc     960
   aaggctggaa actactccaa caaggacgct cttatcaagg agtttacctc taaggtcaac    1020
   cctctttcca acactcacat ccgacaggct cgtgccattg cccgaaagct gcttggaaag    1080
   gacgtcttct ttgactggga cgctgcccga gtccgtgagg gatactaccg ataccacgga    1140
   ggtgtcgagt gcgccatcaa ccgtgctcgt gcctacgctc cttttgccga cctcatctgg    1200
   atggagtcta agctccccga ttacgagcag gccaagcgat ttgctgatgg tgtccacgag    1260
   gccgtccctg gcaagtggct tgcttacaac cagtctcctt ccttcaactg gaccactgct    1320
   atgagcagcg ctgagcaaaa gacctacgtt gaccgactgg ccaagcttgg ttacgtgtgg    1380
   cagttcatca ccctggccgg tctgcatacc aatgctctcg tttccgacaa ctttgcccga    1440
   gactactcgc agcatggtat gttcgcctac gctaacaatg tccaggagcc tgagatcaag    1500
   tctggaattg acattgtcaa gcaccagaag tggtctggag cagaatacat tgataacatt    1560
   ctcagaaccg ctaccggagg aatctcatct acttcggcca tgggagaggg tgtcaccgaa    1620
   gaccagttca agtctaaaat c                                              1641
<210> 29
   <211> 547
   <212> PRT
   <213> Blastobotrys adeninivorans
<220>
   <221> SOURCE
   <222> 1..547
   <223> /mol_type="protein" /note="[CDS]:1..1641 from SEQ ID NO 28" /note="This sequence is generated from feature [CDS]:1..1641 plac ed under sequence number 28" /organism="Blastobotrys adeninivorans"
<400> 29
   Met Ser Ser Lys Gln Glu Ile Leu Lys Gln Glu Glu Ala Gln Phe Gln
   1               5                   10                  15
   Lys Gln Val Lys Asp Leu Lys Gln Trp Trp Ser Glu Pro Arg Trp Arg
               20                  25                  30
   His Thr Lys Arg Ile Tyr Pro Ala Glu Asp Ile Ala Arg Lys Arg Gly
           35                  40                  45
   Asn Leu Glu Val Arg His Pro Ser Ser Thr Gln Ala Asp Lys Leu Phe
       50                  55                  60
   Lys Leu Leu Glu Gln His Asp Lys Asn Lys Thr Ala Ser Ile Thr Tyr
   65                  70                  75                  80
   Gly Cys Leu Asp Pro Val Gln Leu Thr Gln Met Ala Lys Tyr Leu Asp
                   85                  90                  95
   Thr Val Tyr Val Ser Gly Trp Gln Ser Ser Ser Thr Ala Ser Thr Ser
               100                 105                 110
   Asn Glu Pro Ser Pro Asp Leu Ala Asp Tyr Pro Met Asp Thr Val Pro
           115                 120                 125
   Asn Lys Val Gln Gln Leu Trp Phe Ala Gln Leu Phe His Asp Arg Lys
       130                 135                 140
   Gln Arg Glu Asp Arg Leu Thr Gln Pro Ile Glu Lys Arg Gly Asp Leu
   145                 150                 155                 160
   Pro Asn Ile Asp Tyr Leu Arg Pro Ile Ile Ala Asp Ala Asp Thr Gly
                   165                 170                 175
   His Gly Gly Leu Ser Ala Ile Val Lys Leu Thr Lys Met Phe Ile Glu
               180                 185                 190
   Arg Gly Ala Ala Gly Ile His Ile Glu Asp Gln Met Pro Gly Thr Lys
           195                 200                 205
   Lys Cys Gly His Met Ala Gly Lys Val Leu Val Pro Val Gln Glu His
       210                 215                 220
   Ile Asn Arg Leu Val Ala Ile Arg Leu Ala Ala Asp Leu Phe Gly Ser
   225                 230                 235                 240
   Asn Leu Leu Ala Ile Ala Arg Thr Asp Ser Glu Ala Ala Thr Leu Ile
                   245                 250                 255
   Thr Ser Thr Val Asp Tyr Arg Asp His Tyr Phe Ile Val Gly Ser Thr
               260                 265                 270
   Asn Pro Asp Val Pro Pro Leu Val Asp Leu Leu Val Glu Ala Glu Gln
           275                 280                 285
   Lys Gly Leu Gln Gly Ala Ala Leu Glu Arg Ile Glu Ala Glu Trp Val
       290                 295                 300
   Lys Lys Ala Gln Pro Lys Leu Phe Ala Asp Ala Val Ile Asp Glu Ile
   305                 310                 315                 320
   Lys Ala Gly Asn Tyr Ser Asn Lys Asp Ala Leu Ile Lys Glu Phe Thr
                   325                 330                 335
   Ser Lys Val Asn Pro Leu Ser Asn Thr His Ile Arg Gln Ala Arg Ala
               340                 345                 350
   Ile Ala Arg Lys Leu Leu Gly Lys Asp Val Phe Phe Asp Trp Asp Ala
           355                 360                 365
   Ala Arg Val Arg Glu Gly Tyr Tyr Arg Tyr His Gly Gly Val Glu Cys
       370                 375                 380
   Ala Ile Asn Arg Ala Arg Ala Tyr Ala Pro Phe Ala Asp Leu Ile Trp
   385                 390                 395                 400
   Met Glu Ser Lys Leu Pro Asp Tyr Glu Gln Ala Lys Arg Phe Ala Asp
                   405                 410                 415
   Gly Val His Glu Ala Val Pro Gly Lys Trp Leu Ala Tyr Asn Gln Ser
               420                 425                 430
   Pro Ser Phe Asn Trp Thr Thr Ala Met Ser Ser Ala Glu Gln Lys Thr
           435                 440                 445
   Tyr Val Asp Arg Leu Ala Lys Leu Gly Tyr Val Trp Gln Phe Ile Thr
       450                 455                 460
   Leu Ala Gly Leu His Thr Asn Ala Leu Val Ser Asp Asn Phe Ala Arg
   465                 470                 475                 480
   Asp Tyr Ser Gln His Gly Met Phe Ala Tyr Ala Asn Asn Val Gln Glu
                   485                 490                 495
   Pro Glu Ile Lys Ser Gly Ile Asp Ile Val Lys His Gln Lys Trp Ser
               500                 505                 510
   Gly Ala Glu Tyr Ile Asp Asn Ile Leu Arg Thr Ala Thr Gly Gly Ile
           515                 520                 525
   Ser Ser Thr Ser Ala Met Gly Glu Gly Val Thr Glu Asp Gln Phe Lys
       530                 535                 540
   Ser Lys Ile
   545
<210> 30
   <211> 3945
   <212> DNA
   <213> Blastobotrys adeninivorans
<220>
   <221> source
   <222> 1..3945
   <223> /mol_type="DNA" /note="synthetic construct" /organism="Blastobotrys adeninivorans"
<220>
   <221> gene
   <222> 989..2629
   <223> /gene="isocitrate lyase"
<400> 30
   actggcaacc ttactacggg caaccaaaag gatctcaaat ggccaggtag cccagaaagg      60
   caccagagct acaaaggagt tgttgatatc cacaattcgg ttgttctttt cgtcatcacc     120
   aaactgaatc tccttggcaa cgtagtcctg aagcatgcct cggccgttgt gcttgagctc     180
   gtactcggtc aaagcgtcgt gctcaatagc aggctcctca ggaataatgt tggtcatcca     240
   tgcctgaccg tggggatggg ggttggagca acccatggcg gcgcccttgt tctcgaaaat     300
   ctggcagtag ttgatttcag catcttcctg ggctcccttg tacacgtcct tccaagcgtt     360
   gatgacggaa gtgatctgct caggggtcat ctgggccaga gtcagatcgt gacgagggtt     420
   gaagcaaatg actgcgcact ttccagtggc ctgttgggcc tggaacagct catcattgta     480
   caagggaccg gattcagccg actcaatagc cttttcaggc ttgacagcgg catagtcgtt     540
   gacaaacaca aaagtctcat cgtactttgg gtttgcatct ccctgagcac gagtgtttcc     600
   cgggcacagg tagcattttt catcgtactg gggtcgcttc tcgacctggg ccgattcctg     660
   ctggccctgc caggggcgct gagttcggtg aggagagcac aacacccagc tctttcggag     720
   agggttgtaa cgcttgtggg cttcgttcca gtgcagcata atgtacaaaa gtgcaagttg     780
   aagagtgggg tgagaaaaat gctatttata ccggaaaaat cgcttcggcc ggggaaaccg     840
   gggaaaaccc cggaaaaata tgcacaacgg tttggagcca cgtttctcca tgactagtga     900
   ttatgaaatg gatattggaa tgaagagagc gagaaacaaa agattaaatg atgatattcc     960
   tgatgtcaat tagttaaata tacaactaga ttttagactt gaactggtct tcggtgacac    1020
   cctctcccat ggccgaagta gatgagattc ctccggtagc ggttctgaga atgttatcaa    1080
   tgtattctgc tccagaccac ttctggtgct tgacaatgtc aattccagac ttgatctcag    1140
   gctcctggac attgttagcg taggcgaaca taccatgctg cgagtagtct cgggcaaagt    1200
   tgtcggaaac gagagcattg gtatgcagac cggccagggt gatgaactgc cacacgtaac    1260
   caagcttggc cagtcggtca acgtaggtct tttgctcagc gctgctcata gcagtggtcc    1320
   agttgaagga aggagactgg ttgtaagcaa gccacttgcc agggacggcc tcgtggacac    1380
   catcagcaaa tcgcttggcc tgctcgtaat cggggagctt agactccatc cagatgaggt    1440
   cggcaaaagg agcgtaggca cgagcacggt tgatggcgca ctcgacacct ccgtggtatc    1500
   ggtagtatcc ctcacggact cgggcagcgt cccagtcaaa gaagacgtcc tttccaagca    1560
   gctttcgggc aatggcacga gcctgtcgga tgtgagtgtt ggaaagaggg ttgaccttag    1620
   aggtaaactc cttgataaga gcgtccttgt tggagtagtt tccagccttg atctcgtcaa    1680
   tgacagcatc ggcgaacagc ttaggctggg ccttcttgac ccactcggcc tcgattcgct 1740
   ccagagcagc accctggagt cccttctgct cggcctctac cagcaggtct accaaaggag 1800
   gcacatcagg gttggtggat ccaacaataa agtaatgatc ccggtagtcg acagtggagg 1860
   tgatgagagt agcggcctca gagtcagttc gggcaatggc aagaaggtta gagccaaaca 1920
   ggtcggcggc gagacgaatg gccaccagac ggttaatgtg ttcctggacg ggcaccagca 1980
   cctttccggc catgtgtcca cacttcttgg taccgggcat ctggtcctca atgtggattc 2040
   cagcagcacc gcgctcaatg aacatcttgg tcagcttgac gatagcagac agacctccgt 2100
   gaccagtatc ggcatcagca ataataggtc tcagatagtc aatgttgggc aggtctccgc 2160
   gcttttcgat gggctgggtc agacgatcct ctcgctgctt gcgatcgtgg aacaactggg 2220
   caaaccacag ctgctgcacc ttgtttggca cagtgtccat ggggtaatca gcaagatcgg 2280
   gagaaggctc gttggaagta gaggcagtag aagatgattg ccatccagaa acgtacacag 2340
   tgtccaagta ctttgccatc tgggtcagct gaacggggtc cagacatccg taggtgatgg 2400
   atgcagtctt gttcttgtcg tgctgctcca ggagcttgaa cagcttgtca gcctgggtag 2460
   aggaagggtg tcgaacctcg aggtttcctc gctttcgagc aatatcctca gcagggtaga 2520
   tacgcttggt gtgtctccat cgaggctcgg accaccactg cttgaggtcc ttgacctgct 2580
   tttggaattg cgcttcttct tgtttaagaa tctcttgttt agaagacatt tttatggaat 2640
   ttaccaaatg tcaataactt aaaaccaatt ggttgattaa agatcggaga ggggatgtgg 2700
   ccctttttat agaccaaatt ttcaggtgat tcattcttgt tctgtcaatg atccgagggc 2760
   cgaggcagcc ttatggtctg gggttatcgt ctgcacccca gattcgggct tagggaggaa 2820
   ggagaaacag tttggccaga caaagacccg gaaatgtacc gatctggata gcaaaataca 2880
   tggttctact cggaaaaatt caggcaatct cgcaatcact tttatattct atccatagtc 2940
   tacaagctct caaaatactg cctggcttgt cctccctttc tcctcgggat tgactaatta 3000
   ctgggccctg cagggtaggt aacaaccaaa aaatggcccg aacaattacc gcgccgtgca 3060
   gtccgatggt cggccgagcc tgaccgagcg aaaacctttc ggggaaatca cgtgccacca 3120
   tcacgttgga gccgatgagg gaacaatgac tcagttttag cttatcgttc tttacaattc 3180
   tatcccgaat catgtccgaa tgtcggccac aaaggggggg gcgatggcaa ttgtggccgc 3240
   ctgtcatcgc cattaacaac tttgccacct gtccactttt agctctgact gttcttgttt 3300
   ttgttctcct ttagatgcaa gcctagtgcc tgccagggct cacgccattt tgcgtgtgtc 3360
   tagacgattc caccggggag ggggggcact gccgagcccc acccgccgca gcctgtaggg 3420
   cgcacttttt cccgtctccg cagtaacaaa actttgaccg cctttttttc ggtgatgtca 3480
   tttttctctc gctcaagctc cattcccact ggctacccct ccaccactat tgtctggtcg 3540
   taacgtcact gtacagttac gacacacggc ccacgcgcac gcgtagatat acagagtcgt 3600
   tatcgctaca ggcgttgctt gaacctagct ggccgctacc agcgctggta cgttttgaag 3660
   gcgcttcgtg aatttgtggg ttttctgtcg cctccttatc gcccttctgg tgtgccttag 3720
   taattaccag gggtctcagg tttcaccatc tcaaacccac agagtattca agatctgaca 3780
   gttccttttt catctattct attctgtttg agcataagct tggcctcttg ctacgtggtc 3840
   tggtagacga tgccggagat tatcaatcaa aagtcaaacg agccttcccc tggccctggg 3900
   gctaataata atggtaataa tactaggcct ccgggcccaa accat 3945
<210> 31
   <211> 3329
   <212> DNA
   <213> Artificial Sequence
<220>
   <221> source
   <222> 1..3329
   <223> /mol_type="DNA" /note="synthetic construct" /organism="Artificial Sequence"
<220>
   <221> CDS
   <222> 1547..2263
   <223> /gene="TRP" /transl_table=11
<400> 31
   ggactggcaa ccttactacg ggcaaccaaa aggatctcaa atggccaggt agcccagaaa 60
   ggcaccagag ctacaaagga gttgttgata tccacaattc ggttgttctt ttcgtcatca 120
   ccaaactgaa tctccttggc aacgtagtcc tgaagcatgc ctcggccgtt gtgcttgagc 180
   tcgtactcgg tcaaagcgtc gtgctcaata gcaggctcct caggaataat gttggtcatc 240
   catgcctgac cgtggggatg ggggttggag caacccatgg cggcgccctt gttctcgaaa 300
   atctggcagt agttgatttc agcatcttcc tgggctccct tgtacacgtc cttccaagcg 360
   ttgatgacgg aagtgatctg ctcaggggtc atctgggcca gagtcagatc gtgacgaggg 420
   ttgaagcaaa tgactgcgca ctttccagtg gcctgttggg cctggaacag ctcatcattg 480
   tacaagggac cggattcagc cgactcaata gccttttcag gcttgacagc ggcatagtcg 540
   ttgacaaaca caaaagtctc atcgtacttt gggtttgcat ctccctgagc acgagtgttt 600
   cccgggcaca ggtagcattt ttcatcgtac tggggtcgct tctcgacctg ggccgattcc 660
   tgctggccct gccaggggcg ctgagttcgg tgaggagagc acaacaccca gctctttcgg 720
   agagggttgt aacgcttgtg ggcttcgttc cagtgcagca taatgtacaa aagtgcaagt 780
   tgaagagtgg ggtgagaaaa atgctattta taccggaaaa atcgcttcgg ccggggaaac 840
   cggggaaaac cccggaaaaa tatgcacaac ggtttggagc cacgtttctc catgactagt 900
   ggatcccccg ggctgcagga attcgatatc gtgtctagat acagtcccta ttaattgtga 960
   ggaaacccct tagcgctttc aatcgacgaa ttgcaattga caaagagaga acatgctctt 1020
   aagactgaac atcttatcag acaagggccg catcgagcgg ctacctcgta attgtggtca 1080
   gtcattgaca accattagca agccattggc aacgggacaa cgcaggcgaa tctcactcgc 1140
   agttactgcc tgcaatgaca attgctcctc ttcgcgcaaa cataatagac tttgataagc 1200
   accacaatcg cttcttccct cggtcgtact ggcaaatttc atgcatgaat gcggctgcag 1260
   cctcctgtcg atatgggacg gagctctgtt gctttcggcg ccctttccaa gcttcaatcc 1320
   actaatcgga gaatgttcat caactggctc agctaatcag ataggtcaac ttgttcccca 1380
   gcggtttcac cacatgctgt cctttcctcc ccccgctcgg aaggatacgc agaatgcttt 1440
   atcgtggggc tgccccgggc cgaaaccggc attttgggtt gactctttta aggaacgaca 1500
   tgcccagggg actctccact cacaaaccct ttcaaaaacc tgtacaatga caacgtcgcc 1560
   aatcgtcaag gtgtgcggaa tcaggaccgt ggaggcggcc caggtcgctt tggatgctgg 1620
   agccaacatg attggaatga ttatggtccc taatagagcg cgcactgttg atatggagac 1680
   agcaggcaag atatcacgac tggtacgatc atatagacgt caaggatcct ttgagctgga 1740
   cactaatgag ctgggcggat cgactcactc tgcactcgcc cacatcattg aagccaagac 1800
   aaggacccgg ccagcaatcg taggcgtgtt ccgcaaccag ccgctgtcag aagtcctgcg 1860
   gttgcaacag gaattgtcgc tggatttcgt tcagcttcac ggatcagagc ccctagaatg 1920
   gtgtagactg attccatgtc ctgtgatccg tcggttcacc cccgatactc cagatttcta 1980
   tgcctcgccg gttactggat accacagcct atccctcata gacggtgagg ttggcggaga 2040
   gggcaagatg gtggactgga gcggagtcca gtctcgggcg cgaatgggag cgaggttcct 2100
   tatggctgga ggattgacag ccgacaatgt tgcccaagct ctagcggtcg agggtgcctg 2160
   tggtgttgat gtcagcggag gagtcgaatc aagcccgggg gtcaaagatc tcaacgagat 2220
   tgagcggttt gtcaaagctg ccaagggggt tactgtgaag tattaacact ccagcagcag 2280
   catcaatagg tgatactgac gacaggtaat gtgtactaat aggtctgaca gcagaataca 2340
   gctatattac ttctcatttg aaacttgagg tgattgacaa tttactattt atacaagcta 2400
   agcttcaata cagactcgat cgaattcagg tcgacaatta ccgcgccgtg cagtccgatg 2460
   gtcggccgag cctgaccgag cgaaaacctt tcggggaaat cacgtgccac catcacgttg 2520
   gagccgatga gggaacaatg actcagtttt agcttatcgt tctttacaat tctatcccga 2580
   atcatgtccg aatgtcggcc acaaaggggg gggcgatggc aattgtggcc gcctgtcatc 2640
   gccattaaca actttgccac ctgtccactt ttagctctga ctgttcttgt ttttgttctc 2700
   ctttagatgc aagcctagtg cctgccaggg ctcacgccat tttgcgtgtg tctagacgat      2760
   tccaccgggg agggggggca ctgccgagcc ccacccgccg cagcctgtag ggcgcacttt      2820
   ttcccgtctc cgcagtaaca aaactttgac cgcctttttt tcggtgatgt catttttctc      2880
   tcgctcaagc tccattccca ctggctaccc ctccaccact attgtctggt cgtaacgtca      2940
   ctgtacagtt acgacacacg gcccacgcgc acgcgtagat atacagagtc gttatcgcta      3000
   caggcgttgc ttgaacctag ctggccgcta ccagcgctgg tacgttttga aggcgcttcg      3060
   tgaatttgtg ggttttctgt cgcctcctta tcgcccttct ggtgtgcctt agtaattacc      3120
   aggggtctca ggtttcacca tctcaaaccc acagagtatt caagatctga cagttccttt      3180
   ttcatctatt ctattctgtt tgagcataag cttggcctct tgctacgtgg tctggtagac      3240
   gatgccggag attatcaatc aaaagtcaaa cgagccttcc cctggccctg gggctaataa      3300
   taatggtaat aatactaggc ctccgggcc                                        3329
<210> 32
   <211> 239
   <212> PRT
   <213> Artificial Sequence
<220>
   <221> SOURCE
   <222> 1..239
   <223> /mol_type="protein" /note="[CDS]:1547..2263 from SEQ ID NO 31" /note="This sequence is generated from feature [CDS]:1547..2263 p laced under sequence number 31" /organism="Artificial Sequence"
<400> 32
   Met Thr Thr Ser Pro Ile Val Lys Val Cys Gly Ile Arg Thr Val Glu
   1               5                   10                  15
   Ala Ala Gln Val Ala Leu Asp Ala Gly Ala Asn Met Ile Gly Met Ile
               20                  25                  30
   Met Val Pro Asn Arg Ala Arg Thr Val Asp Met Glu Thr Ala Gly Lys
           35                  40                  45
   Ile Ser Arg Leu Val Arg Ser Tyr Arg Arg Gln Gly Ser Phe Glu Leu
       50                  55                  60
   Asp Thr Asn Glu Leu Gly Gly Ser Thr His Ser Ala Leu Ala His Ile
   65                  70                  75                  80
   Ile Glu Ala Lys Thr Arg Thr Arg Pro Ala Ile Val Gly Val Phe Arg
                   85                  90                  95
   Asn Gln Pro Leu Ser Glu Val Leu Arg Leu Gln Gln Glu Leu Ser Leu
               100                 105                 110
   Asp Phe Val Gln Leu His Gly Ser Glu Pro Leu Glu Trp Cys Arg Leu
           115                 120                 125
   Ile Pro Cys Pro Val Ile Arg Arg Phe Thr Pro Asp Thr Pro Asp Phe
       130                 135                 140
   Tyr Ala Ser Pro Val Thr Gly Tyr His Ser Leu Ser Leu Ile Asp Gly
   145                 150                 155                 160
   Glu Val Gly Gly Glu Gly Lys Met Val Asp Trp Ser Gly Val Gln Ser
                   165                 170                 175
   Arg Ala Arg Met Gly Ala Arg Phe Leu Met Ala Gly Gly Leu Thr Ala
               180                 185                 190
   Asp Asn Val Ala Gln Ala Leu Ala Val Glu Gly Ala Cys Gly Val Asp
           195                 200                 205
   Val Ser Gly Gly Val Glu Ser Ser Pro Gly Val Lys Asp Leu Asn Glu
       210                 215                 220
   Ile Glu Arg Phe Val Lys Ala Ala Lys Gly Val Thr Val Lys Tyr
   225                 230                 235
<210> 33
   <211> 1560
   <212> DNA
   <213> Blastobotrys adeninivorans
<220>
   <221> source
   <222> 1..1560
   <223> /mol_type="DNA" /note="acetyl-CoA hydrolase" /organism="Blastobotrys adeninivorans"
<220>
   <221> CDS
   <222> 1..1560
   <223> /gene="acetyl-CoA hydrolase" /transl_table=1
<400> 33
   atgacaatct ctgctgccct caagtctaga atccgggatc ctcgcttgtt gaacaaggtt      60
   gctacacctg ctcaactgct tcctttattc caagatggcc agtacctggg ctggtctgga     120
   ttcactggag taggaggtcc taaggttcta cctagtgcta ttgctgacca agtggaacag     180
   tctggcaaca agatgggttt caatttgttt gtaggagcct cctcagatat cgacatcgag     240
   tctcgatggg cacagttggg aatgatcaac cgtcgctcgc ctcatcaggt cggaaagcca     300
   attgccaatg gcatcaacga gggtcgcatc cacttttttg acaagcactt gtcaatgtgg     360
   gctcaggact tgacctacgg ttactacact atggatcgat ccaagaacag cattgatgtg     420
   gccattattg aggctactgc catcactgag gacggtggtg tcattcttgg accttcggtt     480
   ggaggtactc cagagctggt gtggatggct gacaaggtta tcattgagct taacacccga     540
   ttgccctcgt ttgagggtct gcacgacatt aacatgcccg tgctgcctcc taaccgacag     600
   cccctgccca tcaccactgt atctgagcga tctggaacct ctgctctgta cattcctccc     660
   gagaagattg ctggaatcat tgaatctcag gctcaggaca ctgtgcctga taacgccccc     720
   gctgacgcta cttctcaggc aattgctggt caccttgtcg agtttttgga gcatgaggtc     780
   aagatgggcc gtcagcctgc taaccttaac cccatccagt ccggaattgg taacattgcc     840
   aacgctgtag tggagggtct ggcttctgcc tctttcgaaa acattaacgt atggactgaa     900
   gtgttccagg actcattcct tgatttcctc gagtcgggca agctggagtt tgctaccgcc     960
   acttccatcc gtctcaccaa caagggattt gagcgattct ttggaaactg ggacaccttc    1020
   tccaagaaga ttttgctgcg atctcagcag acttctaacc accctgagat cattcgtcgt    1080
   ctcggtgtca ttgccatgaa cactcccgtg gagattgata tctatggaca cgcaaactcc    1140
   accaacgtgg caggatcgcg aatgctccac ggaattggag gctccggtga tttccttcga    1200
   aatgccaagc tctccgttat gcatactcct tcgttccgtc ctaccaagac tgatccttac    1260
   ggtctgtcct gcgtggtgcc atttgccact cacgtcgacc acactgagca tgacctagat    1320
   gtggttgtta ctgagcaggg actggctgat ctgcgaggca agtctcctcg tgagcgatcc    1380
   cgagagatca tcaagaagtg tgctcaccct gtttaccagc caattctttc tgagtacgtt    1440
   gagcgtgctg agcagtatgc taccaagcgc aagtcgcttc acgaacccca cattcttcgc    1500
   gatgtcttcc gccttcacac caatctagag gagaagggta ccatgcgcat tgactcgtgg    1560
<210> 34
   <211> 520
   <212> PRT
   <213> Blastobotrys adeninivorans
<220>
   <221> SOURCE
   <222> 1..520
   <223> /mol_type="protein" /note="[CDS]:1..1560 from SEQ ID NO 33" /note="This sequence is generated from feature [CDS]:1..1560 plac ed under sequence number 33" /organism="Blastobotrys adeninivorans"
<400> 34
   Met Thr Ile Ser Ala Ala Leu Lys Ser Arg Ile Arg Asp Pro Arg Leu
   1               5                   10                  15
   Leu Asn Lys Val Ala Thr Pro Ala Gln Leu Leu Pro Leu Phe Gln Asp
               20                  25                  30
   Gly Gln Tyr Leu Gly Trp Ser Gly Phe Thr Gly Val Gly Gly Pro Lys
           35                  40                  45
   Val Leu Pro Ser Ala Ile Ala Asp Gln Val Glu Gln Ser Gly Asn Lys
       50                  55                  60
   Met Gly Phe Asn Leu Phe Val Gly Ala Ser Ser Asp Ile Asp Ile Glu
   65                  70                  75                  80
   Ser Arg Trp Ala Gln Leu Gly Met Ile Asn Arg Arg Ser Pro His Gln
                   85                  90                  95
   Val Gly Lys Pro Ile Ala Asn Gly Ile Asn Glu Gly Arg Ile His Phe
               100                 105                 110
   Phe Asp Lys His Leu Ser Met Trp Ala Gln Asp Leu Thr Tyr Gly Tyr
           115                 120                 125
   Tyr Thr Met Asp Arg Ser Lys Asn Ser Ile Asp Val Ala Ile Ile Glu
       130                 135                 140
   Ala Thr Ala Ile Thr Glu Asp Gly Gly Val Ile Leu Gly Pro Ser Val
   145                 150                 155                 160
   Gly Gly Thr Pro Glu Leu Val Trp Met Ala Asp Lys Val Ile Ile Glu
                   165                 170                 175
   Leu Asn Thr Arg Leu Pro Ser Phe Glu Gly Leu His Asp Ile Asn Met
                   180                 185                 190
   Pro Val Leu Pro Pro Asn Arg Gln Pro Leu Pro Ile Thr Thr Val Ser
           195                 200                 205
   Glu Arg Ser Gly Thr Ser Ala Leu Tyr Ile Pro Pro Glu Lys Ile Ala
       210                 215                 220
   Gly Ile Ile Glu Ser Gln Ala Gln Asp Thr Val Pro Asp Asn Ala Pro
   225                 230                 235                 240
   Ala Asp Ala Thr Ser Gln Ala Ile Ala Gly His Leu Val Glu Phe Leu
                   245                 250                 255
   Glu His Glu Val Lys Met Gly Arg Gln Pro Ala Asn Leu Asn Pro Ile
               260                 265                 270
   Gln Ser Gly Ile Gly Asn Ile Ala Asn Ala Val Val Glu Gly Leu Ala
           275                 280                 285
   Ser Ala Ser Phe Glu Asn Ile Asn Val Trp Thr Glu Val Phe Gln Asp
       290                 295                 300
   Ser Phe Leu Asp Phe Leu Glu Ser Gly Lys Leu Glu Phe Ala Thr Ala
   305                 310                 315                 320
   Thr Ser Ile Arg Leu Thr Asn Lys Gly Phe Glu Arg Phe Phe Gly Asn
                   325                 330                 335
   Trp Asp Thr Phe Ser Lys Lys Ile Leu Leu Arg Ser Gln Gln Thr Ser
               340                 345                 350
   Asn His Pro Glu Ile Ile Arg Arg Leu Gly Val Ile Ala Met Asn Thr
           355                 360                 365
   Pro Val Glu Ile Asp Ile Tyr Gly His Ala Asn Ser Thr Asn Val Ala
       370                 375                 380
   Gly Ser Arg Met Leu His Gly Ile Gly Gly Ser Gly Asp Phe Leu Arg
   385                 390                 395                 400
   Asn Ala Lys Leu Ser Val Met His Thr Pro Ser Phe Arg Pro Thr Lys
                   405                 410                 415
   Thr Asp Pro Tyr Gly Leu Ser Cys Val Val Pro Phe Ala Thr His Val
               420                 425                 430
   Asp His Thr Glu His Asp Leu Asp Val Val Val Thr Glu Gln Gly Leu
           435                 440                 445
   Ala Asp Leu Arg Gly Lys Ser Pro Arg Glu Arg Ser Arg Glu Ile Ile
       450                 455                 460
   Lys Lys Cys Ala His Pro Val Tyr Gln Pro Ile Leu Ser Glu Tyr Val
   465                 470                 475                 480
   Glu Arg Ala Glu Gln Tyr Ala Thr Lys Arg Lys Ser Leu His Glu Pro
                   485                 490                 495
   His Ile Leu Arg Asp Val Phe Arg Leu His Thr Asn Leu Glu Glu Lys
               500                 505                 510
   Gly Thr Met Arg Ile Asp Ser Trp
           515                 520
<210> 35
   <211> 4148
   <212> DNA
   <213> Blastobotrys adeninivorans
<220>
   <221> source
   <222> 1..4148
   <223> /mol_type="DNA" /note="synthetic construct" /organism="Blastobotrys adeninivorans"
<220>
   <221> gene
   <222> 1332..2891
   <223> /gene="isocitrate lyase"
<400> 35
<210> 36
   <211> 3530
   <212> DNA
   <213> Artificial Sequence
<220>
   <221> source
   <222> 1..3530
   <223> /mol_type="DNA" /note="synthetic construct" /organism="Artificial Sequence"
<220>
   <221> CDS
   <222> 1630..2346
   <223> /gene="TRP" /transl_table=11
<400> 36
   ggtctttcct gatccactgc atcccgcaat tccaatcaaa taaggctctc tccagggggg 60
   catgtaccgc tggtttttaa aagaggggtt ctcattgttc tctggagcca ttgttatcta 120
   aactggttag agtagaggag gttgaacaaa aaaatattaa gatgccctgt gagcacaaaa 180
   gaaattgagc cgcacggtgc gatataggtt atcttgacga tgggcctcgt gcatcattat 240
   ctgcatcagt acaacgatta ggtgaaaacg actggaggat ttggtttttg gcctagaaat 300
   ttcttggtga ggatggtgga agcgaagtta ctatgtgatg cgcattgtca tccaactgat 360
   catcctgagc aactggatga gattgcaaag atgaacgctt gctctctggc cattatggca 420
   actcggatgg acgatttgga tctggtatat gagacgtccc agaaataccc cgacaagact 480
   attgctgcgt ttgggtatca tccctggttt tcacatctgg tttataccac tcctgatccc 540
   cctgaaagtg cttgggatca ctacaaagag gtattggtgc ctactcccac gccagagttt 600
   gttgagctat tgccatttcc caaaagtttt gatgagcact tgaagcggat tgaaagttac 660
   ttgaaaaatg acccaaattc aatagtgggc gaatgtggcc tggacaaggc atttacactt 720
   ccgatccctg ggtccaaccc tcgaaacttg agccagtatc atgtcagcat ggaccaccaa 780
   aaggaagttt ttatgaaaca gctcgaatta gcgactgcgt acaaccggcc tgtgtctatc 840
   catggagtac actgtccaca aacggtgtac gattgcatta taaatatgga gaaccaacca 900
   cccagcatct gtctgcattc attcactggg tccccagctt ttctccagtc ttggtttaca 960
   ccaggccgca agcagaaact agtggatccc ccgggctgca ggaattcgat atcgtgtcta 1020
   gatacagtcc ctattaattg tgaggaaacc ccttagcgct ttcaatcgac gaattgcaat 1080
   tgacaaagag agaacatgct cttaagactg aacatcttat cagacaaggg ccgcatcgag 1140
   cggctacctc gtaattgtgg tcagtcattg acaaccatta gcaagccatt ggcaacggga 1200
   caacgcaggc gaatctcact cgcagttact gcctgcaatg acaattgctc ctcttcgcgc 1260
   aaacataata gactttgata agcaccacaa tcgcttcttc cctcggtcgt actggcaaat 1320
   ttcatgcatg aatgcggctg cagcctcctg tcgatatggg acggagctct gttgctttcg 1380
   gcgccctttc caagcttcaa tccactaatc ggagaatgtt catcaactgg ctcagctaat 1440
   cagataggtc aacttgttcc ccagcggttt caccacatgc tgtcctttcc tccccccgct 1500
   cggaaggata cgcagaatgc tttatcgtgg ggctgccccg ggccgaaacc ggcattttgg 1560
   gttgactctt ttaaggaacg acatgcccag gggactctcc actcacaaac cctttcaaaa 1620
   acctgtacaa tgacaacgtc gccaatcgtc aaggtgtgcg gaatcaggac cgtggaggcg 1680
   gcccaggtcg ctttggatgc tggagccaac atgattggaa tgattatggt ccctaataga 1740
   gcgcgcactg ttgatatgga gacagcaggc aagatatcac gactggtacg atcatataga 1800
   cgtcaaggat cctttgagct ggacactaat gagctgggcg gatcgactca ctctgcactc 1860
   gcccacatca ttgaagccaa gacaaggacc cggccagcaa tcgtaggcgt gttccgcaac 1920
   cagccgctgt cagaagtcct gcggttgcaa caggaattgt cgctggattt cgttcagctt 1980
   cacggatcag agcccctaga atggtgtaga ctgattccat gtcctgtgat ccgtcggttc 2040
   acccccgata ctccagattt ctatgcctcg ccggttactg gataccacag cctatccctc 2100
   atagacggtg aggttggcgg agagggcaag atggtggact ggagcggagt ccagtctcgg 2160
   gcgcgaatgg gagcgaggtt ccttatggct ggaggattga cagccgacaa tgttgcccaa 2220
   gctctagcgg tcgagggtgc ctgtggtgtt gatgtcagcg gaggagtcga atcaagcccg 2280
   ggggtcaaag atctcaacga gattgagcgg tttgtcaaag ctgccaaggg ggttactgtg 2340
   aagtattaac actccagcag cagcatcaat aggtgatact gacgacaggt aatgtgtact 2400
   aataggtctg acagcagaat acagctatat tacttctcat ttgaaacttg aggtgattga 2460
   caatttacta tttatacaag ctaagcttca atacagactc gatcgaattc aggtcgacaa 2520
   aaggtgcgtc cggagcgcag ttggtctcac tgacactgta agacaaaagt caaagtatac 2580
   tcagcaagct ccgtctctcc ccagtaactc tctacctacc attctcccat ccctctacgc 2640
   ttttgtccgt tgagtttttg actcaacaat ccgggttggg ttgccgcacg gaaaattagt 2700
   cttctcgcgg ctgtgcatga cgtggggcag atcatgaggc gacttgctaa ggttttagtt     2760
   gccaagaaga gacaatttgc aataattccg gcattgagtt tgccggagca gttgttgaac     2820
   cccatgggga cagagaggac gaccgattga ggtacaataa ctttgaattg gtcatctttt     2880
   gtcgctgtta acagtgacta ttgtgtgcct cactgccgtg gtctctttac gtcggtttaa     2940
   tcaccagttg ttgttttttt ttgtgttttc ttttctcatt cctttcccac gctggcaaat     3000
   gcattggcga tttacgattg gtttacaatg gcccaggttc caaattcccc ccggggaaat     3060
   ggtgaccgaa taggacaaca gggggtctcg ggaaaccgga gaatccgcag tctggagaca     3120
   aacggaaata ctagcggttg tgtgttagag cttgggttcc aacgccgcct gaataatccg     3180
   gaactttgga gccgttgacc cacccacagt tttacaaaca agctaccagt tggtctaatg     3240
   attagtgtac agggtcaatt ccactgctct tttggcacca aatcgtcatg tcttcgtagg     3300
   ccatcgcaag acgccgcctt aaccggacca gtgcggcggg cttgtatgct ccgacgatgg     3360
   cccgagtttg tctggggcca attagaaagc gccgattacg gccctaatac ggtcccacgg     3420
   ctgcgcccgt gtaaggtaga tttagttaat aactctgcag gaaacattca ttagctgctg     3480
   aggctagacc ccggggaccg tgcggccctt acgtcagccc cgtttgggcc                3530
<210> 37
   <211> 239
   <212> PRT
   <213> Artificial Sequence
<220>
   <221> SOURCE
   <222> 1..239
   <223> /mol_type="protein" /note="This sequence is generated from feature [CDS]:1630..2346 p laced under sequence number 36" /note="[CDS]:1630..2346 from SEQ ID NO 36" /organism="Artificial Sequence"
<400> 37
   Met Thr Thr Ser Pro Ile Val Lys Val Cys Gly Ile Arg Thr Val Glu
   1               5                   10                  15
   Ala Ala Gln Val Ala Leu Asp Ala Gly Ala Asn Met Ile Gly Met Ile
               20                  25                  30
   Met Val Pro Asn Arg Ala Arg Thr Val Asp Met Glu Thr Ala Gly Lys
           35                  40                  45
   Ile Ser Arg Leu Val Arg Ser Tyr Arg Arg Gln Gly Ser Phe Glu Leu
       50                  55                  60
   Asp Thr Asn Glu Leu Gly Gly Ser Thr His Ser Ala Leu Ala His Ile
   65                  70                  75                  80
   Ile Glu Ala Lys Thr Arg Thr Arg Pro Ala Ile Val Gly Val Phe Arg
                   85                  90                  95
   Asn Gln Pro Leu Ser Glu Val Leu Arg Leu Gln Gln Glu Leu Ser Leu
               100                 105                 110
   Asp Phe Val Gln Leu His Gly Ser Glu Pro Leu Glu Trp Cys Arg Leu
           115                 120                 125
   Ile Pro Cys Pro Val Ile Arg Arg Phe Thr Pro Asp Thr Pro Asp Phe
       130                 135                 140
   Tyr Ala Ser Pro Val Thr Gly Tyr His Ser Leu Ser Leu Ile Asp Gly
   145                 150                 155                 160
   Glu Val Gly Gly Glu Gly Lys Met Val Asp Trp Ser Gly Val Gln Ser
                   165                 170                 175
   Arg Ala Arg Met Gly Ala Arg Phe Leu Met Ala Gly Gly Leu Thr Ala
               180                 185                 190
   Asp Asn Val Ala Gln Ala Leu Ala Val Glu Gly Ala Cys Gly Val Asp
           195                 200                 205
   Val Ser Gly Gly Val Glu Ser Ser Pro Gly Val Lys Asp Leu Asn Glu
       210                 215                 220
   Ile Glu Arg Phe Val Lys Ala Ala Lys Gly Val Thr Val Lys Tyr
   225                 230                 235
<210> 38
   <211> 1632
   <212> DNA
   <213> Blastobotrys adeninivorans
<220>
   <221> source
   <222> 1..1632
   <223> /mol_type="DNA" /organism="Blastobotrys adeninivorans"
<220>
   <221> CDS
   <222> 1..1632
   <223> /gene="acetyl-CoA hydrolase" /transl_table=1
<400> 38
   gtgcgggtag agaacggaag tgatccgggg acctgtgggg acggatattt caatgtccgg      60
   aaatttcaat catccggacc ccgggtgtta actgctcggg tgctgagttt ccgatacatt     120
   ttcgctgaac atatatatcg ttgcaaactg cttttggagt gcaggctggc gaatatgttc     180
   tcattaaagc attcaattag gcttgctagt gcccaagctg gcgttagatt cttttccaac     240
   aaggtcgttg gagtggaagc tgctctcagt gcagtcaaat ccgggaacac tgtgctgtgc     300
   ggaggatttg gactcagtgg tgttcctgat actctgatca atggactaga gaaacgcccg     360
   gagattggaa acctcactgc agtatcgaac aatgctggta ttgatgggtc aggcttgtca     420
   aagctactgg tatccggcca ggtcactaaa atgattgcat cttacattgg tggaaacaag     480
   acctttgaaa gactatatct tactggaaag ctagatgtag aactctgtcc tcagggaaca     540
   attgctgaaa aatgccgagc tggtggtgct ggtgtgcctg ctttttacac tcccgctggc     600
   gtgggtacct ggctgcagga gggaaaaatc cccatgagat atgatgataa gggccaggtg     660
   atcaagacta acgagcctcg agaggttcgt gtgtttgatg gccgcgagta cgtcttggaa     720
   aagtcaatct tcgctgatgt ggccttcatc aaggcttaca aggtggacaa ggccggtaac     780
   tgttacttcc gaggatcatc cagaaacttc aaccaggcaa tgggtaaggc cgctaaacac     840
   actgttgtcg aagccgagaa cattgttgaa attggagaga ttgatccagc tgatatccat     900
   ttgtcgtctg tctatgtcaa gcagattgtt caatccgaaa ctcccaagaa tattgaaatc     960
   tacaagttca ccggtgatga gagtgctcct gttaactccc gccgagagaa gattattcga    1020
   agagctgcta aggagcttga gaataacgat cacattaact tgggaattgg catgcctact    1080
   ttgattccaa actacctgcc cgagggtgtg cacgtcactc ttcagtctga gaacggtatt    1140
   ctgggtcttg gtccttaccc tactaagggt caggaggacc ctgatttgat taatgctgga    1200
   aaggagactg tgactctggc tcctggtgcc tcgttgtttg gttctgagga gtcctttgga    1260
   atgatccgat cgggacgaat tgacaaggca attctcggtg ccatgcaggt atctgccaag    1320
   ggagaccttg caaactgggg tcttccagga atggtcaagg gaatgggtgg tgccatggat    1380
   cttgtcgcca acccttatgc tacacgagtg attgtgctta ctgaccatac tgataagcat    1440
   ggaaacccca aggttgtcga cacttgctct ttccctctga caggaaagaa ctgtgtgtcc    1500
   cgcatcatca ctgacctgtg tgtgtttgat gtcaatccca ctgagggact tactttgatt    1560
   gagctccagg acggtgttac tgtagatgag gtcaaggaaa aaactggagc tcccttcaag    1620
   gtcgtgctta ac                                                        1632
<210> 39
   <211> 544
   <212> PRT
   <213> Blastobotrys adeninivorans
<220>
   <221> SOURCE
   <222> 1..544
   <223> /mol_type="protein" /note="[CDS]:1..1632 from SEQ ID NO 38" /note="This sequence is generated from feature [CDS]:1..1632 plac ed under sequence number 38" /organism="Blastobotrys adeninivorans"
<400> 39
   Val Arg Val Glu Asn Gly Ser Asp Pro Gly Thr Cys Gly Asp Gly Tyr
   1               5                   10                  15
   Phe Asn Val Arg Lys Phe Gln Ser Ser Gly Pro Arg Val Leu Thr Ala
               20                  25                  30
   Arg Val Leu Ser Phe Arg Tyr Ile Phe Ala Glu His Ile Tyr Arg Cys
           35                  40                  45
   Lys Leu Leu Leu Glu Cys Arg Leu Ala Asn Met Phe Ser Leu Lys His
       50                  55                  60
   Ser Ile Arg Leu Ala Ser Ala Gln Ala Gly Val Arg Phe Phe Ser Asn
   65                  70                  75                  80
   Lys Val Val Gly Val Glu Ala Ala Leu Ser Ala Val Lys Ser Gly Asn
                   85                  90                  95
   Thr Val Leu Cys Gly Gly Phe Gly Leu Ser Gly Val Pro Asp Thr Leu
               100                 105                 110
   Ile Asn Gly Leu Glu Lys Arg Pro Glu Ile Gly Asn Leu Thr Ala Val
           115                 120                 125
   Ser Asn Asn Ala Gly Ile Asp Gly Ser Gly Leu Ser Lys Leu Leu Val
       130                 135                 140
   Ser Gly Gln Val Thr Lys Met Ile Ala Ser Tyr Ile Gly Gly Asn Lys
   145                 150                 155                 160
   Thr Phe Glu Arg Leu Tyr Leu Thr Gly Lys Leu Asp Val Glu Leu Cys
                   165                 170                 175
   Pro Gln Gly Thr Ile Ala Glu Lys Cys Arg Ala Gly Gly Ala Gly Val
               180                 185                 190
   Pro Ala Phe Tyr Thr Pro Ala Gly Val Gly Thr Trp Leu Gln Glu Gly
           195                 200                 205
   Lys Ile Pro Met Arg Tyr Asp Asp Lys Gly Gln Val Ile Lys Thr Asn
       210                 215                 220
   Glu Pro Arg Glu Val Arg Val Phe Asp Gly Arg Glu Tyr Val Leu Glu
   225                 230                 235                 240
   Lys Ser Ile Phe Ala Asp Val Ala Phe Ile Lys Ala Tyr Lys Val Asp
                   245                 250                 255
   Lys Ala Gly Asn Cys Tyr Phe Arg Gly Ser Ser Arg Asn Phe Asn Gln
               260                 265                 270
   Ala Met Gly Lys Ala Ala Lys His Thr Val Val Glu Ala Glu Asn Ile
           275                 280                 285
   Val Glu Ile Gly Glu Ile Asp Pro Ala Asp Ile His Leu Ser Ser Val
       290                 295                 300
   Tyr Val Lys Gln Ile Val Gln Ser Glu Thr Pro Lys Asn Ile Glu Ile
   305                 310                 315                 320
   Tyr Lys Phe Thr Gly Asp Glu Ser Ala Pro Val Asn Ser Arg Arg Glu
                   325                 330                 335
   Lys Ile Ile Arg Arg Ala Ala Lys Glu Leu Glu Asn Asn Asp His Ile
               340                 345                 350
   Asn Leu Gly Ile Gly Met Pro Thr Leu Ile Pro Asn Tyr Leu Pro Glu
           355                 360                 365
   Gly Val His Val Thr Leu Gln Ser Glu Asn Gly Ile Leu Gly Leu Gly
       370                 375                 380
   Pro Tyr Pro Thr Lys Gly Gln Glu Asp Pro Asp Leu Ile Asn Ala Gly
   385                 390                 395                 400
   Lys Glu Thr Val Thr Leu Ala Pro Gly Ala Ser Leu Phe Gly Ser Glu
                   405                 410                 415
   Glu Ser Phe Gly Met Ile Arg Ser Gly Arg Ile Asp Lys Ala Ile Leu
               420                 425                 430
   Gly Ala Met Gln Val Ser Ala Lys Gly Asp Leu Ala Asn Trp Gly Leu
           435                 440                 445
   Pro Gly Met Val Lys Gly Met Gly Gly Ala Met Asp Leu Val Ala Asn
       450                 455                 460
   Pro Tyr Ala Thr Arg Val Ile Val Leu Thr Asp His Thr Asp Lys His
   465                 470                 475                 480
   Gly Asn Pro Lys Val Val Asp Thr Cys Ser Phe Pro Leu Thr Gly Lys
                   485                 490                 495
   Asn Cys Val Ser Arg Ile Ile Thr Asp Leu Cys Val Phe Asp Val Asn
               500                 505                 510
   Pro Thr Glu Gly Leu Thr Leu Ile Glu Leu Gln Asp Gly Val Thr Val
           515                 520                 525
   Asp Glu Val Lys Glu Lys Thr Gly Ala Pro Phe Lys Val Val Leu Asn
       530                 535                 540
<210> 40
   <211> 3583
   <212> DNA
   <213> Blastobotrys adeninivorans
<220>
   <221> source
   <222> 1..3583
   <223> /mol_type="DNA" /note="synthetic construct" /organism="Blastobotrys adeninivorans"
<220>
   <221> gene
   <222> 1029..2660
   <223> /gene="acetyl-CoA hydrolase"
<400> 40
   aggtggcgat tctgctggtg caaaccttac cgagactgta acacagaggc tggtagctca 60
   taatgagcag tcctcttcgc cctatccaca attgctacac caggtactta tagttcctca 120
   agcagacgct acagatcgaa ccaagtacca gtcgttcagg aaatatgcaa acgctgcggg 180
   cctgggggaa aaagaagtag actattttta caaacactat tttggagacc gaccggtaca 240
   ccatgatatt cgagcatcgc cattgctaaa tgatgacaag gtctttgcca aactcccacc 300
   cgcgacagta ttaattgcag gaatggatcc cttgagagat gagggtatag cttatgtgca 360
   aaagatggaa aagaacggag tcaaagtgga cactactgta tttgaagggc ttcctcatgg 420
   atttatgaca tttccaatcg caaagacaga tgagcttgtg cagctctacg taaaagccgt 480
   ccagagtgca atccacgacg gccagtctta gtctgtgcgg acacgaatag gcgaattgga 540
   ccttgattcc gtggcagcgg aacttgaact tacaaaagta aagatttctg agataaggct 600
   caatacagta gacaattggt tagacattga taagacggca atcgttgtcc atgaccttcc 660
   tcattggtgg agccatcgta tttgatagtt ctacatcaat ggatacgatt atcaacgacc 720
   gttctcgaat agtgaatctc tagatactaa tatgctctct gtataagtga cccaacggcc 780
   tcgacaactg tttcggcccc accttctacc gacttctgat tttaactaac attgaattgc 840
   cggaatatac caacttaaaa actgtcacta cacaataaat atggtgacaa gctgcaaatt 900
   atttacaaac actaactcac ccatccaaga tagttcacgg tctgcccaag ggctcctccg 960
   ccatggtctc agaccgttaa agtggggtaa tatacggctt taaccccctg gcgtaactgc 1020
   ggtagtgagt gcgggtagag aacggaagtg atccggggac ctgtggggac ggatatttca 1080
   atgtccggaa atttcaatca tccggacccc gggtgttaac tgctcgggtg ctgagtttcc 1140
   gatacatttt cgctgaacat atatatcgtt gcaaactgct tttggagtgc aggctggcga 1200
   atatgttctc attaaagcat tcaattaggc ttgctagtgc ccaagctggc gttagattct 1260
   tttccaacaa ggtcgttgga gtggaagctg ctctcagtgc agtcaaatcc gggaacactg 1320
   tgctgtgcgg aggatttgga ctcagtggtg ttcctgatac tctgatcaat ggactagaga 1380
   aacgcccgga gattggaaac ctcactgcag tatcgaacaa tgctggtatt gatgggtcag 1440
   gcttgtcaaa gctactggta tccggccagg tcactaaaat gattgcatct tacattggtg 1500
   gaaacaagac ctttgaaaga ctatatctta ctggaaagct agatgtagaa ctctgtcctc 1560
   agggaacaat tgctgaaaaa tgccgagctg gtggtgctgg tgtgcctgct ttttacactc 1620
   ccgctggcgt gggtacctgg ctgcaggagg gaaaaatccc catgagatat gatgataagg 1680
   gccaggtgat caagactaac gagcctcgag aggttcgtgt gtttgatggc cgcgagtacg 1740
   tcttggaaaa gtcaatcttc gctgatgtgg ccttcatcaa ggcttacaag gtggacaagg 1800
   ccggtaactg ttacttccga ggatcatcca gaaacttcaa ccaggcaatg ggtaaggccg 1860
   ctaaacacac tgttgtcgaa gccgagaaca ttgttgaaat tggagagatt gatccagctg 1920
   atatccattt gtcgtctgtc tatgtcaagc agattgttca atccgaaact cccaagaata 1980
   ttgaaatcta caagttcacc ggtgatgaga gtgctcctgt taactcccgc cgagagaaga 2040
   ttattcgaag agctgctaag gagcttgaga ataacgatca cattaacttg ggaattggca 2100
   tgcctacttt gattccaaac tacctgcccg agggtgtgca cgtcactctt cagtctgaga 2160
   acggtattct gggtcttggt ccttacccta ctaagggtca ggaggaccct gatttgatta 2220
   atgctggaaa ggagactgtg actctggctc ctggtgcctc gttgtttggt tctgaggagt 2280
   cctttggaat gatccgatcg ggacgaattg acaaggcaat tctcggtgcc atgcaggtat 2340
   ctgccaaggg agaccttgca aactggggtc ttccaggaat ggtcaaggga atgggtggtg 2400
   ccatggatct tgtcgccaac ccttatgcta cacgagtgat tgtgcttact gaccatactg 2460
   ataagcatgg aaaccccaag gttgtcgaca cttgctcttt ccctctgaca ggaaagaact 2520
   gtgtgtcccg catcatcact gacctgtgtg tgtttgatgt caatcccact gagggactta 2580
   ctttgattga gctccaggac ggtgttactg tagatgaggt caaggaaaaa actggagctc 2640
   ccttcaaggt cgtgcttaac taagctgtat acaattcaat ggcatttcac tctaactctt 2700
   ccaaaactga tgatattgct tagatttaat atgttgaact aatttcggtg aatagtgttt 2760
   gatagataga tctgcatgta tgtgaatgga tctattcttc gaggacatgg cgaatggcgc 2820
   ctacgaaatc atcaaaaaac tctagggtct tcgcgagggg tgggaatgtg aaccacacat 2880
   gcgggtagcc aacatactcc ttggtcttga catctacacc gttttcagca agcatttgag 2940
   cgtaattggc gccgtcgtca tggagacagt cttcagaatt gaagaaaacg aatgcttttg 3000
   gaagatcctt gtgaccatct gggtgtagat aagggcaaat gtatttgcct aacgctccat 3060
   caggccgaga tgcattccac atggtagtta gcgtttcgct attgacgcaa gcaccatcct 3120
   ttcctactct gtcaaatgag gtaaacaagt gcttcttatc gtcgggaaga aggtcctgct 3180
   gaactgtatt aggctgaatc agtacctgac ccttgattgc tcgggtgcca tcatcgcgat 3240
   ctttcagagc gacagaggca gccaaacctc ctccagccga atcaccagca acaaacaatc 3300
   gctttttatc cccgcccagc ttctcggcat tctctagagt ccattggaat cctgcgtaca 3360
   catcctcgaa aatgccatgg aatttcgtct ctggagctaa gcgataatcc acctgtacaa 3420
   ttacggcggg aatagcctgg ctggcacatc ttagcaagta gtcgtcattc tcaaggtcgc 3480
   ccaatatgag gcctcctcca tgggcataga atccaagcgg cagcggcccc tcaacacctt 3540
   ttggcttata cactctaagg gcaattttcg gatttgtggg gga 3583
<210> 41
   <211> 3382
   <212> DNA
   <213> Artificial Sequence
<220>
   <221> source
   <222> 1..3382
   <223> /mol_type="DNA" /note="synthetic construct" /organism="Artificial Sequence"
<220>
   <221> gene
   <222> 1543..2259
   <223> /gene="reverse complementary sequence for TRP"
<400> 41
   aggtggcgat tctgctggtg caaaccttac cgagactgta acacagaggc tggtagctca 60
   taatgagcag tcctcttcgc cctatccaca attgctacac caggtactta tagttcctca 120
   agcagacgct acagatcgaa ccaagtacca gtcgttcagg aaatatgcaa acgctgcggg 180
   cctgggggaa aaagaagtag actattttta caaacactat tttggagacc gaccggtaca 240
   ccatgatatt cgagcatcgc cattgctaaa tgatgacaag gtctttgcca aactcccacc 300
   cgcgacagta ttaattgcag gaatggatcc cttgagagat gagggtatag cttatgtgca 360
   aaagatggaa aagaacggag tcaaagtgga cactactgta tttgaagggc ttcctcatgg 420
   atttatgaca tttccaatcg caaagacaga tgagcttgtg cagctctacg taaaagccgt 480
   ccagagtgca atccacgacg gccagtctta gtctgtgcgg acacgaatag gcgaattgga 540
   ccttgattcc gtggcagcgg aacttgaact tacaaaagta aagatttctg agataaggct 600
   caatacagta gacaattggt tagacattga taagacggca atcgttgtcc atgaccttcc 660
   tcattggtgg agccatcgta tttgatagtt ctacatcaat ggatacgatt atcaacgacc 720
   gttctcgaat agtgaatctc tagatactaa tatgctctct gtataagtga cccaacggcc 780
   tcgacaactg tttcggcccc accttctacc gacttctgat tttaactaac attgaattgc 840
   cggaatatac caacttaaaa actgtcacta cacaataaat atggtgacaa gctgcaaatt 900
   atttacaaac actaactcac ccatccaaga tagttcacgg tctgcccaag ggtcgacctg 960
   aattcgatcg agtctgtatt gaagcttagc ttgtataaat agtaaattgt caatcacctc 1020
   aagtttcaaa tgagaagtaa tatagctgta ttctgctgtc agacctatta gtacacatta 1080
   cctgtcgtca gtatcaccta ttgatgctgc tgctggagtg ttaatacttc acagtaaccc 1140
   ccttggcagc tttgacaaac cgctcaatct cgttgagatc tttgaccccc gggcttgatt 1200
   cgactcctcc gctgacatca acaccacagg caccctcgac cgctagagct tgggcaacat 1260
   tgtcggctgt caatcctcca gccataagga acctcgctcc cattcgcgcc cgagactgga 1320
   ctccgctcca gtccaccatc ttgccctctc cgccaacctc accgtctatg agggataggc 1380
   tgtggtatcc agtaaccggc gaggcataga aatctggagt atcgggggtg aaccgacgga 1440
   tcacaggaca tggaatcagt ctacaccatt ctaggggctc tgatccgtga agctgaacga 1500
   aatccagcga caattcctgt tgcaaccgca ggacttctga cagcggctgg ttgcggaaca 1560
   cgcctacgat tgctggccgg gtccttgtct tggcttcaat gatgtgggcg agtgcagagt 1620
   gagtcgatcc gcccagctca ttagtgtcca gctcaaagga tccttgacgt ctatatgatc 1680
   gtaccagtcg tgatatcttg cctgctgtct ccatatcaac agtgcgcgct ctattaggga 1740
   ccataatcat tccaatcatg ttggctccag catccaaagc gacctgggcc gcctccacgg 1800
   tcctgattcc gcacaccttg acgattggcg acgttgtcat tgtacaggtt tttgaaaggg 1860
   tttgtgagtg gagagtcccc tgggcatgtc gttccttaaa agagtcaacc caaaatgccg 1920
   gtttcggccc ggggcagccc cacgataaag cattctgcgt atccttccga gcggggggag 1980
   gaaaggacag catgtggtga aaccgctggg gaacaagttg acctatctga ttagctgagc 2040
   cagttgatga acattctccg attagtggat tgaagcttgg aaagggcgcc gaaagcaaca 2100
   gagctccgtc ccatatcgac aggaggctgc agccgcattc atgcatgaaa tttgccagta 2160
   cgaccgaggg aagaagcgat tgtggtgctt atcaaagtct attatgtttg cgcgaagagg 2220
   agcaattgtc attgcaggca gtaactgcga gtgagattcg cctgcgttgt cccgttgcca 2280
   atggcttgct aatggttgtc aatgactgac cacaattacg aggtagccgc tcgatgcggc 2340
   ccttgtctga taagatgttc agtcttaaga gcatgttctc tctttgtcaa ttgcaattcg 2400
   tcgattgaaa gcgctaaggg gtttcctcac aattaatagg gactgtatct agacacgata 2460
   tcgaattcct gcagcccggg ggatccacta gtaatggcat ttcactctaa ctcttccaaa 2520
   actgatgata ttgcttagat ttaatatgtt gaactaattt cggtgaatag tgtttgatag 2580
   atagatctgc atgtatgtga atggatctat tcttcgagga catggcgaat ggcgcctacg 2640
   aaatcatcaa aaaactctag ggtcttcgcg aggggtggga atgtgaacca cacatgcggg 2700
   tagccaacat actccttggt cttgacatct acaccgtttt cagcaagcat ttgagcgtaa 2760
   ttggcgccgt cgtcatggag acagtcttca gaattgaaga aaacgaatgc ttttggaaga 2820
   tccttgtgac catctgggtg tagataaggg caaatgtatt tgcctaacgc tccatcaggc 2880
   cgagatgcat tccacatggt agttagcgtt tcgctattga cgcaagcacc atcctttcct 2940
   actctgtcaa atgaggtaaa caagtgcttc ttatcgtcgg gaagaaggtc ctgctgaact 3000
   gtattaggct gaatcagtac ctgacccttg attgctcggg tgccatcatc gcgatctttc 3060
   agagcgacag aggcagccaa acctcctcca gccgaatcac cagcaacaaa caatcgcttt 3120
   ttatccccgc ccagcttctc ggcattctct agagtccatt ggaatcctgc gtacacatcc     3180
   tcgaaaatgc catggaattt cgtctctgga gctaagcgat aatccacctg tacaattacg     3240
   gcgggaatag cctggctggc acatcttagc aagtagtcgt cattctcaag gtcgcccaat     3300
   atgaggcctc ctccatgggc atagaatcca agcggcagcg gcccctcaac accttttggc      3360
   ttatacactc taagggcaat tt                                              3382
<210> 42
   <211> 239
   <212> PRT
   <213> Blastobotrys adeninivorans
<220>
   <221> SOURCE
   <222> 1..239
   <223> /mol_type="protein" /note="TRP" /organism="Blastobotrys adeninivorans"
<400> 42
   Met Thr Thr Ser Pro Ile Val Lys Val Cys Gly Ile Arg Thr Val Glu
   1               5                   10                  15
   Ala Ala Gln Val Ala Leu Asp Ala Gly Ala Asn Met Ile Gly Met Ile
               20                  25                  30
   Met Val Pro Asn Arg Ala Arg Thr Val Asp Met Glu Thr Ala Gly Lys
           35                  40                  45
   Ile Ser Arg Leu Val Arg Ser Tyr Arg Arg Gln Gly Ser Phe Glu Leu
       50                  55                  60
   Asp Thr Asn Glu Leu Gly Gly Ser Thr His Ser Ala Leu Ala His Ile
   65                  70                  75                  80
   Ile Glu Ala Lys Thr Arg Thr Arg Pro Ala Ile Val Gly Val Phe Arg
                   85                  90                  95
   Asn Gln Pro Leu Ser Glu Val Leu Arg Leu Gln Gln Glu Leu Ser Leu
               100                 105                 110
   Asp Phe Val Gln Leu His Gly Ser Glu Pro Leu Glu Trp Cys Arg Leu
           115                 120                 125
   Ile Pro Cys Pro Val Ile Arg Arg Phe Thr Pro Asp Thr Pro Asp Phe
       130                 135                 140
   Tyr Ala Ser Pro Val Thr Gly Tyr His Ser Leu Ser Leu Ile Asp Gly
   145                 150                 155                 160
   Glu Val Gly Gly Glu Gly Lys Met Val Asp Trp Ser Gly Val Gln Ser
                   165                 170                 175
   Arg Ala Arg Met Gly Ala Arg Phe Leu Met Ala Gly Gly Leu Thr Ala
               180                 185                 190
   Asp Asn Val Ala Gln Ala Leu Ala Val Glu Gly Ala Cys Gly Val Asp
           195                 200                 205
   Val Ser Gly Gly Val Glu Ser Ser Pro Gly Val Lys Asp Leu Asn Glu
       210                 215                 220
   Ile Glu Arg Phe Val Lys Ala Ala Lys Gly Val Thr Val Lys Tyr
   225                 230                 235
<210> 43
   <211> 2319
   <212> DNA
   <213> Blastobotrys adeninivorans
<220>
   <221> source
   <222> 1..2319
   <223> /mol_type="DNA" /note="acyl-carnitine transferase 1" /organism="Blastobotrys adeninivorans"
<220>
   <221> CDS
   <222> 1..2319
   <223> /gene="acyl-carnitine transferase 1" /transl_table=1
<400> 43
   atgtcaaaag gaattacgtt tgcccaccag gacaagctgg aaaagctccc gatcccagag      60
   ctagaggata catgccgcca gtatttggag gtgctgaaac cgctgcagac ccctagggag     120
   catgctcata ccgagcaggc ggtgaagcgg ttcttggacg ggccaggacc catgctccag     180
   aagaagctcc aggagtatgc cgactctaga gcatcatata ttgagcagtt ttggtacgat     240
   tcgtatttga attacgacaa cccggtagtg ttaaatctca accctttctt tatgctcgag     300
   gatgactcca cccctcaccc tactggcatc tctcagattg ctcgcgccgc caccttgacc     360
   atgagttcgc tgcggttcat tcgagcattg cgacgagagg agctgcctcc tgaccagttc     420
   cggggtcaga aactttgcat gtaccagtac tctcgtctgt ttgggtcggc tagaattccc     480
   acaaacactg gctgtgtcat gcagtcgtac aatgcttctc gtcacattgt agtagtgtgc     540
   cgatctcagt actactggtt tgacgtgctg gacgagaaca gcgatctcat catgtccgag     600
   caggatatta tgctcaactt ccagactatc atggaggacg caaagaacac ccccatcacc     660
   gaggcttgca agggtgccgt aggagtcttg accactgaga accgacgggt gtgggccaac     720
   attcgtgatt ccatgatgaa cgacgccgac gtgcctggaa ccgccgaatc aaacaacttt     780
   gagtgcctgc gaaagattga tctggccctg tttgtggtat gcttggacca cgcctctccc     840
   gagtcactgt ctgatctgtc ccgcaacatg ctgtgtggtc tgtccgagct tgaaaagggc     900
   attcaggttg gtacctgtac caaccgatgg tacgacaagc tgcagattat cgttactgcc     960
   aacgcaaagg ccggaatcaa ctttgagcac actggtgtag acggtcacac ggtgctgcga    1020
   ttcgcttctg atatttacac tgacaccatt ctgcggtttg caaagacaat taacggccag    1080
   tcccctagtc tgtgggccac tcaatctcct gaccctgctt ctcgcgaccc ggcttcgttt    1140
   ggcgaggtgt cgaccactcc tcgaaagctg gaatgggccc ctactccaga gctcactttg    1200
   gccctgcgat tcgcagagac tcgtctggcc gatctgattg agcagaatga ctttcaggtc    1260
   ctggagtttg attcgtacgg tatgaactac attaagcgca tgggcttcag tcccgacgcc    1320
   tttgtgcaga tggcattcca ggcctcctac tactcgctat atggccgagt ggagtgtacc    1380
   tatgagcccg ccatgaccaa gaagttcctg cacggtagaa ccgagagtat ccgagtagtg    1440
   tcacaagagt cagcagcgtt tgtacgcaag ttctgcgaag acgctccccc tgagcagaag    1500
   attgaagctc tgcgacgggc ctgccagcgg cactcaaaga ccaccgcccg atgctctcag    1560
   ggtctgggcc aggacagaca cttgtacgcg ctcttctgcg tgtggaagcg actgctggag    1620
   tcggaggatc tgtctactgt agagaacgga acccaagaca atgagctcta ttctgcggca    1680
   gagtctgtgg ttcgtaacga aagcgtttct gatggcatgt ctgacgtttc tggttcctcc    1740
   gacacttctg gcagcgactc gtctcttgac ctgccatcca tctttgctga tgggggctgg    1800
   gacacgctgt ctaacgtgat tctgtccacc tcaaactgcg gaaaccctgc cctcaagttg    1860
   tttggtttcg gtcctacttc ggctgatggt tttggaattg gttacattat caaggaggac    1920
   agcatcacaa tctgcgcatc gtccaagcac cgtcagacca aacgattcct agacactctg    1980
   cgatcttact ttgtcgaggt ccgtaatcag taccgcacca cccagaagaa gaaggagcct    2040
   gcgcttgccg actctggggc ctccactggc accgctactc cagtctcttc tcagcgtcac    2100
   tttggtcgtt cactgggcgc tccaatgaca aaggcccgcc aatccgagga ggaatcgtcg    2160
   taccttcttg gaggttacgg ttactttgat gtgggcgatt ttgagtaccg atctcggcct    2220
   ccttctcctg ctcctggagt tcatactcat cacaatcgcc atcaccacca tcaccatcgc    2280
   aatcgagaga ttggtcgcaa gctccgcttg gcagagtac                           2319
<210> 44
   <211> 773
   <212> PRT
   <213> Blastobotrys adeninivorans
<220>
   <221> SOURCE
   <222> 1..773
   <223> /mol_type="protein" /note="This sequence is generated from feature [CDS]:1..2319 plac ed under sequence number 43" /note="[CDS]:1..2319 from SEQ ID NO 43" /organism="Blastobotrys adeninivorans"
<400> 44
   Met Ser Lys Gly Ile Thr Phe Ala His Gln Asp Lys Leu Glu Lys Leu
   1               5                   10                  15
   Pro Ile Pro Glu Leu Glu Asp Thr Cys Arg Gln Tyr Leu Glu Val Leu
               20                  25                  30
   Lys Pro Leu Gln Thr Pro Arg Glu His Ala His Thr Glu Gln Ala Val
           35                  40                  45
   Lys Arg Phe Leu Asp Gly Pro Gly Pro Met Leu Gln Lys Lys Leu Gln
       50                  55                  60
   Glu Tyr Ala Asp Ser Arg Ala Ser Tyr Ile Glu Gln Phe Trp Tyr Asp
   65                  70                  75                  80
   Ser Tyr Leu Asn Tyr Asp Asn Pro Val Val Leu Asn Leu Asn Pro Phe
                   85                  90                  95
   Phe Met Leu Glu Asp Asp Ser Thr Pro His Pro Thr Gly Ile Ser Gln
               100                 105                 110
   Ile Ala Arg Ala Ala Thr Leu Thr Met Ser Ser Leu Arg Phe Ile Arg
           115                 120                 125
   Ala Leu Arg Arg Glu Glu Leu Pro Pro Asp Gln Phe Arg Gly Gln Lys
       130                 135                 140
   Leu Cys Met Tyr Gln Tyr Ser Arg Leu Phe Gly Ser Ala Arg Ile Pro
   145                 150                 155                 160
   Thr Asn Thr Gly Cys Val Met Gln Ser Tyr Asn Ala Ser Arg His Ile
                   165                 170                 175
   Val Val Val Cys Arg Ser Gln Tyr Tyr Trp Phe Asp Val Leu Asp Glu
               180                 185                 190
   Asn Ser Asp Leu Ile Met Ser Glu Gln Asp Ile Met Leu Asn Phe Gln
           195                 200                 205
   Thr Ile Met Glu Asp Ala Lys Asn Thr Pro Ile Thr Glu Ala Cys Lys
       210                 215                 220
   Gly Ala Val Gly Val Leu Thr Thr Glu Asn Arg Arg Val Trp Ala Asn
   225                 230                 235                 240
   Ile Arg Asp Ser Met Met Asn Asp Ala Asp Val Pro Gly Thr Ala Glu
                   245                 250                 255
   Ser Asn Asn Phe Glu Cys Leu Arg Lys Ile Asp Leu Ala Leu Phe Val
               260                 265                 270
   Val Cys Leu Asp His Ala Ser Pro Glu Ser Leu Ser Asp Leu Ser Arg
           275                 280                 285
   Asn Met Leu Cys Gly Leu Ser Glu Leu Glu Lys Gly Ile Gln Val Gly
       290                 295                 300
   Thr Cys Thr Asn Arg Trp Tyr Asp Lys Leu Gln Ile Ile Val Thr Ala
   305                 310                 315                 320
   Asn Ala Lys Ala Gly Ile Asn Phe Glu His Thr Gly Val Asp Gly His
                   325                 330                 335
   Thr Val Leu Arg Phe Ala Ser Asp Ile Tyr Thr Asp Thr Ile Leu Arg
               340                 345                 350
   Phe Ala Lys Thr Ile Asn Gly Gln Ser Pro Ser Leu Trp Ala Thr Gln
           355                 360                 365
   Ser Pro Asp Pro Ala Ser Arg Asp Pro Ala Ser Phe Gly Glu Val Ser
       370                 375                 380
   Thr Thr Pro Arg Lys Leu Glu Trp Ala Pro Thr Pro Glu Leu Thr Leu
   385                 390                 395                 400
   Ala Leu Arg Phe Ala Glu Thr Arg Leu Ala Asp Leu Ile Glu Gln Asn
                   405                 410                 415
   Asp Phe Gln Val Leu Glu Phe Asp Ser Tyr Gly Met Asn Tyr Ile Lys
               420                 425                 430
   Arg Met Gly Phe Ser Pro Asp Ala Phe Val Gln Met Ala Phe Gln Ala
           435                 440                 445
   Ser Tyr Tyr Ser Leu Tyr Gly Arg Val Glu Cys Thr Tyr Glu Pro Ala
       450                 455                 460
   Met Thr Lys Lys Phe Leu His Gly Arg Thr Glu Ser Ile Arg Val Val
   465                 470                 475                 480
   Ser Gln Glu Ser Ala Ala Phe Val Arg Lys Phe Cys Glu Asp Ala Pro
                   485                 490                 495
   Pro Glu Gln Lys Ile Glu Ala Leu Arg Arg Ala Cys Gln Arg His Ser
               500                 505                 510
   Lys Thr Thr Ala Arg Cys Ser Gln Gly Leu Gly Gln Asp Arg His Leu
           515                 520                 525
   Tyr Ala Leu Phe Cys Val Trp Lys Arg Leu Leu Glu Ser Glu Asp Leu
       530                 535                 540
   Ser Thr Val Glu Asn Gly Thr Gln Asp Asn Glu Leu Tyr Ser Ala Ala
   545                 550                 555                 560
   Glu Ser Val Val Arg Asn Glu Ser Val Ser Asp Gly Met Ser Asp Val
                   565                 570                 575
   Ser Gly Ser Ser Asp Thr Ser Gly Ser Asp Ser Ser Leu Asp Leu Pro
               580                 585                 590
   Ser Ile Phe Ala Asp Gly Gly Trp Asp Thr Leu Ser Asn Val Ile Leu
           595                 600                 605
   Ser Thr Ser Asn Cys Gly Asn Pro Ala Leu Lys Leu Phe Gly Phe Gly
       610                 615                 620
   Pro Thr Ser Ala Asp Gly Phe Gly Ile Gly Tyr Ile Ile Lys Glu Asp
   625                 630                 635                 640
   Ser Ile Thr Ile Cys Ala Ser Ser Lys His Arg Gln Thr Lys Arg Phe
                   645                 650                 655
   Leu Asp Thr Leu Arg Ser Tyr Phe Val Glu Val Arg Asn Gln Tyr Arg
               660                 665                 670
   Thr Thr Gln Lys Lys Lys Glu Pro Ala Leu Ala Asp Ser Gly Ala Ser
           675                 680                 685
   Thr Gly Thr Ala Thr Pro Val Ser Ser Gln Arg His Phe Gly Arg Ser
       690                 695                 700
   Leu Gly Ala Pro Met Thr Lys Ala Arg Gln Ser Glu Glu Glu Ser Ser
   705                 710                 715                 720
   Tyr Leu Leu Gly Gly Tyr Gly Tyr Phe Asp Val Gly Asp Phe Glu Tyr
                   725                 730                 735
   Arg Ser Arg Pro Pro Ser Pro Ala Pro Gly Val His Thr His His Asn
               740                 745                 750
   Arg His His His His His His Arg Asn Arg Glu Ile Gly Arg Lys Leu
           755                 760                 765
   Arg Leu Ala Glu Tyr
       770
<210> 45
   <211> 4969
   <212> DNA
   <213> Blastobotrys adeninivorans
<220>
   <221> source
   <222> 1..4969
   <223> /mol_type="DNA" /note="synthetic construct" /organism="Blastobotrys adeninivorans"
<220>
   <221> gene
   <222> 1541..3859
   <223> /gene="acyl-carnitine transferase"
<400> 45
   aacaagaccc tgccaacgct cctccagcgg cgatagctcc tgggcccagc acgtccccaa      60
   atgcagactt tatagtttca taaacggtaa agtaaattcc tgatcccagg gcgtctcgca     120
   gcatgtggta tttccaccct gagtacagaa ctttgactcc ttggagcttg accagcttct     180
   tggctacctg gaatgttgac tgaggaggct gctgacgaac cagcccgtca atctgggacg     240
   ctagctttgt aaattcaaac ggacatccca acattgtgca cgaggtaccg gcgcacaccc     300
   ctgccaggaa agccacgggc attcttcgca caagagcatc aggggaatgg ccgtgatcct     360
   ttggcggtgc gtacaccgag aaggctagtt tggaagtata gggcagcgcg tagttgtaca     420
   ccgacactga cagagctctc accaccgacg acgacaccag tggagcgaca atgcctcggt     480
   agaacccccg aatgccctcg gtacggtgag tttcgaccac acagttccac accgagcggt 540
   acttgtgagt ttgttggcgg gtctttatgg aatcaagagg gaatcctact ccaatagccc 600
   acattgacgc tgcgtatgcg cacaacggtt gacgatatgg cagcagccac tgagggatcc 660
   gatcatttct ggactgagga gccagggaca gagtcctcag cgcatccctg gcgtcttctg 720
   acatgtcacg acgaagctac tcacagtgaa caaggttggc tcacggggat gacgcagatc 780
   aaggtaaatc agcaggttga acctcgggca cacggacaca cggacacacg gaccgagtct 840
   tcgcggcgct ccgagtatgc gtaggtagag aggctgttgt gactcaaccg taacttctcg 900
   aatctccaat ccatactaca tgatatacag atattaatgg cgtaggacta gaccagtact 960
   gagatgtaac tgaaatacag cttaaactga aatgaatgat ggaatggtat gaaatggtat 1020
   gaaatggtat gaaatagtat gggctaggga gtacccctga gaacatggtg cttaagcata 1080
   ttcgaactat ttccaaatgc tcacgacccc catagggtta caggaaagcc agattctttt 1140
   atttgagatg aacaatactt gacctaactg aagcaattga caaggaattc acatcttggg 1200
   atgaacttct aaactccaag cgaaactggg gtttgtatga ttgttaccaa ttgatcccac 1260
   agatgatcta gtttctatag actcaggatt tgttatattc caaactacag atccaaaata 1320
   tgtgcgcatt catcggtttg gcggctaggt ctggacgttc ccagagcctc ttcatggatg 1380
   cctgtaccaa cgttcccaga gcctcttgat ggatgcctgt accaggagtg aattccccta 1440
   tgtttcgagt tagagttcta tcatggcccg gagcaatccc agtgatacta gtaatagtgc 1500
   atagatgtaa tttataacct ttatatccgt tagtatgtta gtactctgcc aagcggagct 1560
   tgcgaccaat ctctcgattg cgatggtgat ggtggtgatg gcgattgtga tgagtatgaa 1620
   ctccaggagc aggagaagga ggccgagatc ggtactcaaa atcgcccaca tcaaagtaac 1680
   cgtaacctcc aagaaggtac gacgattcct cctcggattg gcgggccttt gtcattggag 1740
   cgcccagtga acgaccaaag tgacgctgag aagagactgg agtagcggtg ccagtggagg 1800
   ccccagagtc ggcaagcgca ggctccttct tcttctgggt ggtgcggtac tgattacgga 1860
   cctcgacaaa gtaagatcgc agagtgtcta ggaatcgttt ggtctgacgg tgcttggacg 1920
   atgcgcagat tgtgatgctg tcctccttga taatgtaacc aattccaaaa ccatcagccg 1980
   aagtaggacc gaaaccaaac aacttgaggg cagggtttcc gcagtttgag gtggacagaa 2040
   tcacgttaga cagcgtgtcc cagcccccat cagcaaagat ggatggcagg tcaagagacg 2100
   agtcgctgcc agaagtgtcg gaggaaccag aaacgtcaga catgccatca gaaacgcttt 2160
   cgttacgaac cacagactct gccgcagaat agagctcatt gtcttgggtt ccgttctcta 2220
   cagtagacag atcctccgac tccagcagtc gcttccacac gcagaagagc gcgtacaagt 2280
   gtctgtcctg gcccagaccc tgagagcatc gggcggtggt ctttgagtgc cgctggcagg 2340
   cccgtcgcag agcttcaatc ttctgctcag ggggagcgtc ttcgcagaac ttgcgtacaa 2400
   acgctgctga ctcttgtgac actactcgga tactctcggt tctaccgtgc aggaacttct 2460
   tggtcatggc gggctcatag gtacactcca ctcggccata tagcgagtag taggaggcct 2520
   ggaatgccat ctgcacaaag gcgtcgggac tgaagcccat gcgcttaatg tagttcatac 2580
   cgtacgaatc aaactccagg acctgaaagt cattctgctc aatcagatcg gccagacgag 2640
   tctctgcgaa tcgcagggcc aaagtgagct ctggagtagg ggcccattcc agctttcgag 2700
   gagtggtcga cacctcgcca aacgaagccg ggtcgcgaga agcagggtca ggagattgag 2760
   tggcccacag actaggggac tggccgttaa ttgtctttgc aaaccgcaga atggtgtcag 2820
   tgtaaatatc agaagcgaat cgcagcaccg tgtgaccgtc tacaccagtg tgctcaaagt 2880
   tgattccggc ctttgcgttg gcagtaacga taatctgcag cttgtcgtac catcggttgg 2940
   tacaggtacc aacctgaatg cccttttcaa gctcggacag accacacagc atgttgcggg 3000
   acagatcaga cagtgactcg ggagaggcgt ggtccaagca taccacaaac agggccagat 3060
   caatctttcg caggcactca aagttgtttg attcggcggt tccaggcacg tcggcgtcgt 3120
   tcatcatgga atcacgaatg ttggcccaca cccgtcggtt ctcagtggtc aagactccta 3180
   cggcaccctt gcaagcctcg gtgatggggg tgttctttgc gtcctccatg atagtctgga 3240
   agttgagcat aatatcctgc tcggacatga tgagatcgct gttctcgtcc agcacgtcaa 3300
   accagtagta ctgagatcgg cacactacta caatgtgacg agaagcattg tacgactgca 3360
   tgacacagcc agtgtttgtg ggaattctag ccgacccaaa cagacgagag tactggtaca 3420
   tgcaaagttt ctgaccccgg aactggtcag gaggcagctc ctctcgtcgc aatgctcgaa 3480
   tgaaccgcag cgaactcatg gtcaaggtgg cggcgcgagc aatctgagag atgccagtag 3540
   ggtgaggggt ggagtcatcc tcgagcataa agaaagggtt gagatttaac actaccgggt 3600
   tgtcgtaatt caaatacgaa tcgtaccaaa actgctcaat atatgatgct ctagagtcgg 3660
   catactcctg gagcttcttc tggagcatgg gtcctggccc gtccaagaac cgcttcaccg 3720
   cctgctcggt atgagcatgc tccctagggg tctgcagcgg tttcagcacc tccaaatact 3780
   ggcggcatgt atcctctagc tctgggatcg ggagcttttc cagcttgtcc tggtgggcaa 3840
   acgtaattcc ttttgacatg accgctgtct acaatgcaac caatcaaacg aataggacta 3900
   tggaaccaat caactaatgg aaccagtcaa cgaaatagtt ggcgttcgta ctgcaaatca 3960
   atggcaaccc ttcccaaccg ctctttatat actgactccc gtacgtccgg gttagcggaa 4020
   tgtctttgca tcttggagcg gctcaagctc cgtccacccg atgaccccac ggggaaacgg 4080
   actgagatgg caaacaagca atagcttatc ttagactagg attggtttgg cgacgacgct 4140
   gggcgggtgg caggtgattg aggttgttgg aggctttgtt gaaagtaatt gaaccggttg 4200
   aggatggggg ttgggggatg ggggttgggg ttggatagtt ggacgagttg aggccaggaa 4260
   ctgcagcatg aaactcgggt ccgcaatgta tcatgtacga cagctatgat gttatgggcg 4320
   ggtgctattg gtggatacat tgtgggcatt agtgatctat ggagaatatg ttgattatgc 4380
   atgaggtgaa gtgacaaagt ggtgaatatg attggtagat gggtgtagtt aagcggtgaa 4440
   gctgttggta gaactaggtg gctgtgaagg ggacagtgat gattggcgag atgtagatac 4500
   ggtgtagtta aaccagtgca ttttgctaca ctgccataga tgctgagcag cttcactttg 4560
   ctgccctgat agtaggctag tattggtcat tgatctatgg tcgttatcat tactgctatt 4620
   ggggagctgt aggcagcttc gttactgtgc tagagacgct gagcagcttc actttgttac 4680
   tgctgctggc tacgctgctg gttacgctgc tggttacgct gctggttacg ctgcttggtt 4740
   acgctcagct gctggtttgc gtcccggcct gcctcggggc ctcttgacaa ccccatctcc 4800
   cgtagatgac ttgcgcttct tgggaacatc ctgcaacagc tcctcgactt catcatctgc 4860
   ctctggcgac ccgggagtag tagtgggagt accggccatg gaccccgtag cactgtcctg 4920
   attggcagca gacttccgtg gcccactctt cctcttcatt gcatccgga 4969
<210> 46
   <211> 3515
   <212> DNA
   <213> Artificial Sequence
<220>
   <221> source
   <222> 1..3515
   <223> /mol_type="DNA" /note="synthetic construct" /organism="Artificial Sequence"
<220>
   <221> gene
   <222> 1116-1832
   <223> /gene="reverse complementary TRP"
<400> 46
<210> 47
   <211> 239
   <212> PRT
   <213> Blastobotrys adeninivorans
<220>
   <221> SOURCE
   <222> 1..239
   <223> /mol_type="protein" /note="TRP" /organism="Blastobotrys adeninivorans"
<400> 47
   Met Thr Thr Ser Pro Ile Val Lys Val Cys Gly Ile Arg Thr Val Glu
   1 5 10 15
   Ala Ala Gln Val Ala Leu Asp Ala Gly Ala Asn Met Ile Gly Met Ile
   20 25 30
   Met Val Pro Asn Arg Ala Arg Thr Val Asp Met Glu Thr Ala Gly Lys
   35 40 45
   Ile Ser Arg Leu Val Arg Ser Tyr Arg Arg Gln Gly Ser Phe Glu Leu
   50 55 60
   Asp Thr Asn Glu Leu Gly Gly Ser Thr His Ser Ala Leu Ala His Ile
   65 70 75 80
   Ile Glu Ala Lys Thr Arg Thr Arg Pro Ala Ile Val Gly Val Phe Arg
   85 90 95
   Asn Gln Pro Leu Ser Glu Val Leu Arg Leu Gln Gln Glu Leu Ser Leu
   100 105 110
   Asp Phe Val Gln Leu His Gly Ser Glu Pro Leu Glu Trp Cys Arg Leu
   115 120 125
   Ile Pro Cys Pro Val Ile Arg Arg Phe Thr Pro Asp Thr Pro Asp Phe
   130 135 140
   Tyr Ala Ser Pro Val Thr Gly Tyr His Ser Leu Ser Leu Ile Asp Gly
   145 150 155 160
   Glu Val Gly Gly Glu Gly Lys Met Val Asp Trp Ser Gly Val Gln Ser
   165 170 175
   Arg Ala Arg Met Gly Ala Arg Phe Leu Met Ala Gly Gly Leu Thr Ala
   180 185 190
   Asp Asn Val Ala Gln Ala Leu Ala Val Glu Gly Ala Cys Gly Val Asp
   195 200 205
   Val Ser Gly Gly Val Glu Ser Ser Pro Gly Val Lys Asp Leu Asn Glu
   210 215 220
   Ile Glu Arg Phe Val Lys Ala Ala Lys Gly Val Thr Val Lys Tyr
   225 230 235
<210> 48
   <211> 1854
   <212> DNA
   <213> Blastobotrys adeninivorans
<220>
   <221> source
   <222> 1..1854
   <223> /mol_type="DNA" /note="acyl-carnitine transferase 2" /organism="Blastobotrys adeninivorans"
<220>
   <221> CDS
   <222> 1..1854
   <223> /gene="acyl-carnitine transferase 2" /transl_table=1
<400> 48
   atgttatccc gaatgtccag aactgcagta aagacacccg tttcacgtat ggggatgatt      60
   cgtctcaact ccactcacgc ttacgtggag gacaagtcgc agggaccaat gcttcgatac     120
   cagaactcgc tccctcgact gcccgtgcct agtctggagg agaccgctgc gcgatacctc     180
   cgatctgtca aggccgtggc tacttcggac cagtacgctc gaaccgagca ggcggtcaag     240
   aagtttcttc agtctgaggg ccctgctctc cagaagcagc tcgaggcccg tgccgccgac     300
   cctaacatga agaactggct ggctgagtgg tggaacaacg gagcctacct tgatgtgcgc     360
   gaccctgtgg tgccctacgt gtcgtacttt tactcccaca aggacgacaa gctccgaaag     420
   aagcctgctg cccgagctgc ttccctgacc tcggtggccc tgaccttcaa ggaccaggtt     480
   gacaacaaga ctctggagcc tgagtacatg aagaagaacc ccatctgcat ggacgcctac     540
   aagtacatgt tcaacacttg ccgtatcccc gtccagggcg gtaccgacta ccctgaaact 600
   tactcggcca aggacaacca gtacattgtg gccatccgca agaaccgatt cttcaagatt 660
   taccacgatg tcaatggcca gcgattgtct gctggtgacc tggagaagca gtttgagcag 720
   gtttaccagc tcgctgacac ccgtggtcct gccattggtg ccctgacctc ctggaaccgt 780
   gacaagtggg gtgctgcccg caccgagctg ctgtccgccg gcaacaagga ggctcttaag 840
   gccattgagg ccgcctcttt cgtggtgtgc ctggacgacg ctgctcctgt cactcgtgag 900
   gagcgcgctc accagtactg gcacggagac ggaaagaacc gattctacga caagccagtt 960
   cagtttattg tcaacaacaa cggtacctct ggtttcatgg gagagcactc catgatggac 1020
   ggaacccaga ctcaccgtct caacgactac actcttgatg ccatcttcaa caagaaggtt 1080
   gagatttcta acaaggagac tggctcgtac ggtgctcccg aggagatcaa gttcaacgtc 1140
   aacggccgaa ttgaggctct cattgacgag gctcttgccg actttgatgc cgagattggc 1200
   gcccacgagc tggcagtgtt caactaccag ggatacggaa agggactcat gaagaagttc 1260
   aagtgctctc ctgatgccta cctgcagatg atcattcagc tcgcttacta caagatgttc 1320
   ggcgtgtctc gacctaccta tgagtctgct gccacccgac gatttgctct gggacgaact 1380
   gagacctgcc gatcggtgtc tctcgagtcg attgactttg tcaagactat ggaggaccct 1440
   actgcttccg ccaaggagaa ggttgaggct gcccgaaagg ccattgccgc tcacggaaag 1500
   actatcaatg atgcctctgc cggactggga gtcgaccgtc acttgtttgg tctcaagaag 1560
   tttgtcaagg ctcaggatgc tcttcccgag ctgtaccagg accccatgta cagctactct 1620
   tgctcgtggt acctgtctac ttctcagctc tcttctgagt actacaacgg atacggatgg 1680
   tctcaggtga ttgaccccgg atttggatgc gcttacatga ttaacgagaa caacattaac 1740
   attaacattg tttctaagaa gcagggatct gagaagctgc gattctacct caacgaggcc 1800
   gctgatgaca tggctgcagt cttttcttcc gagctttcgg aggctcccaa gctg 1854
<210> 49
   <211> 618
   <212> PRT
   <213> Blastobotrys adeninivorans
<220>
   <221> SOURCE
   <222> 1..618
   <223> /mol_type="protein" /note="[CDS]:1..1854 from SEQ ID NO 48" /note="This sequence is generated from feature [CDS]:1..1854 plac ed under sequence number 48" /organism="Blastobotrys adeninivorans"
<400> 49
   Met Leu Ser Arg Met Ser Arg Thr Ala Val Lys Thr Pro Val Ser Arg
   1 5 10 15
   Met Gly Met Ile Arg Leu Asn Ser Thr His Ala Tyr Val Glu Asp Lys
   20 25 30
   Ser Gln Gly Pro Met Leu Arg Tyr Gln Asn Ser Leu Pro Arg Leu Pro
   35 40 45
   Val Pro Ser Leu Glu Glu Thr Ala Ala Arg Tyr Leu Arg Ser Val Lys
   50 55 60
   Ala Val Ala Thr Ser Asp Gln Tyr Ala Arg Thr Glu Gln Ala Val Lys
   65 70 75 80
   Lys Phe Leu Gln Ser Glu Gly Pro Ala Leu Gln Lys Gln Leu Glu Ala
   85 90 95
   Arg Ala Ala Asp Pro Asn Met Lys Asn Trp Leu Ala Glu Trp Trp Asn
   100 105 110
   Asn Gly Ala Tyr Leu Asp Val Arg Asp Pro Val Val Pro Tyr Val Ser
   115 120 125
   Tyr Phe Tyr Ser His Lys Asp Asp Lys Leu Arg Lys Lys Pro Ala Ala
   130 135 140
   Arg Ala Ala Ser Leu Thr Ser Val Ala Leu Thr Phe Lys Asp Gln Val
   145 150 155 160
   Asp Asn Lys Thr Leu Glu Pro Glu Tyr Met Lys Lys Asn Pro Ile Cys
   165 170 175
   Met Asp Ala Tyr Lys Tyr Met Phe Asn Thr Cys Arg Ile Pro Val Gln
   180 185 190
   Gly Gly Thr Asp Tyr Pro Glu Thr Tyr Ser Ala Lys Asp Asn Gln Tyr
   195 200 205
   Ile Val Ala Ile Arg Lys Asn Arg Phe Phe Lys Ile Tyr His Asp Val
   210 215 220
   Asn Gly Gln Arg Leu Ser Ala Gly Asp Leu Glu Lys Gln Phe Glu Gln
   225 230 235 240
   Val Tyr Gln Leu Ala Asp Thr Arg Gly Pro Ala Ile Gly Ala Leu Thr
   245 250 255
   Ser Trp Asn Arg Asp Lys Trp Gly Ala Ala Arg Thr Glu Leu Leu Ser
   260 265 270
   Ala Gly Asn Lys Glu Ala Leu Lys Ala Ile Glu Ala Ala Ser Phe Val
   275 280 285
   Val Cys Leu Asp Asp Ala Ala Pro Val Thr Arg Glu Glu Arg Ala His
   290 295 300
   Gln Tyr Trp His Gly Asp Gly Lys Asn Arg Phe Tyr Asp Lys Pro Val
   305 310 315 320
   Gln Phe Ile Val Asn Asn Asn Gly Thr Ser Gly Phe Met Gly Glu His
   325 330 335
   Ser Met Met Asp Gly Thr Gln Thr His Arg Leu Asn Asp Tyr Thr Leu
   340 345 350
   Asp Ala Ile Phe Asn Lys Lys Val Glu Ile Ser Asn Lys Glu Thr Gly
   355 360 365
   Ser Tyr Gly Ala Pro Glu Glu Ile Lys Phe Asn Val Asn Gly Arg Ile
   370 375 380
   Glu Ala Leu Ile Asp Glu Ala Leu Ala Asp Phe Asp Ala Glu Ile Gly
   385 390 395 400
   Ala His Glu Leu Ala Val Phe Asn Tyr Gln Gly Tyr Gly Lys Gly Leu
   405 410 415
   Met Lys Lys Phe Lys Cys Ser Pro Asp Ala Tyr Leu Gln Met Ile Ile
   420 425 430
   Gln Leu Ala Tyr Tyr Lys Met Phe Gly Val Ser Arg Pro Thr Tyr Glu
   435 440 445
   Ser Ala Ala Thr Arg Arg Phe Ala Leu Gly Arg Thr Glu Thr Cys Arg
   450 455 460
   Ser Val Ser Leu Glu Ser Ile Asp Phe Val Lys Thr Met Glu Asp Pro
   465 470 475 480
   Thr Ala Ser Ala Lys Glu Lys Val Glu Ala Ala Arg Lys Ala Ile Ala
   485 490 495
   Ala His Gly Lys Thr Ile Asn Asp Ala Ser Ala Gly Leu Gly Val Asp
   500 505 510
   Arg His Leu Phe Gly Leu Lys Lys Phe Val Lys Ala Gln Asp Ala Leu
               515                 520                 525
   Pro Glu Leu Tyr Gln Asp Pro Met Tyr Ser Tyr Ser Cys Ser Trp Tyr
           530                 535                 540
   Leu Ser Thr Ser Gln Leu Ser Ser Glu Tyr Tyr Asn Gly Tyr Gly Trp
   545                 550                 555                 560
   Ser Gln Val Ile Asp Pro Gly Phe Gly Cys Ala Tyr Met Ile Asn Glu
                   565                 570                 575
   Asn Asn Ile Asn Ile Asn Ile Val Ser Lys Lys Gln Gly Ser Glu Lys
               580                 585                 590
   Leu Arg Phe Tyr Leu Asn Glu Ala Ala Asp Asp Met Ala Ala Val Phe
           595                 600                 605
   Ser Ser Glu Leu Ser Glu Ala Pro Lys Leu
       610                 615
<210> 50
   <211> 3894
   <212> DNA
   <213> Blastobotrys adeninivorans
<220>
   <221> source
   <222> 1..3894
   <223> /mol_type="DNA" /note="synthetic construct" /organism="Blastobotrys adeninivorans"
<220>
   <221> gene
   <222> 1024..2877
   <223> /gene="acyl-carnitine transferase"
<400> 50
   gttgacaggt ttccagtcga atgacaattt ttaattggca aacctgaaat gacctctagg      60
   gtgcagtatg cggcagcacc tgcagtggag tccagcttgg ccaactcccg agataaccgt     120
   cgaggtaacg acccaaggta accggcaacc ccgctctctt acatgctaca aggtgaattt     180
   atacaattgg aacagttcca gctgctcatc aagcctagcc tggagcctgg agcctgatag     240
   cctggaagcc tgatcaacca ggtagtgccg ccgcactggg ttccggatga cgagaaagat     300
   gatttgacaa aggcaacgtg ggtcaatgga ccctgaaaga ccaacggcca acagattgca     360
   cagatcgtcg gggcaaatga cgtcgctaag cccagttccg accgctgttt actagtttaa     420
   ccttggttcc tgtcggcatt tggggtcccc ttttcacggt gcgtcgggca gttaccgagg     480
   ccccgaattg ccgaaaccga ggagacccat atgaagaacc gtcaaagcta gcgaatgctg     540
   ggcctggata ctccagatcc taggtttcca gcaatatatt ttttacttcc tacactaggt     600
   gacccccaaa ccttgtgcca tgccttcatt ggctcaaggc gctggtgata aattatcgtg     660
   cctatctttg tacgcacttc gcgtttgtcc gaggatgact cggggccact gtggggaagg     720
   cggtgaggct tgcaaggacc gctctaacgg agcagagatg aaagagagga aaagccagtc     780
   aattgagtcg gtttgagggc aatttaacgg ttttatcgtg tctatcaggg gtcaattgac     840
   ccaggtccac cctatggtgg tttaatttat tggtgcatca tgcgaaccgg gttaacggcc     900
   tcggtcggca ttgaccgaga ggtgtccgga tggcttgtgt gtgtccccac gatatataaa     960
   accccacaaa tcaccaaaag tgctactttt tcttgtacgt tactattctg aacagggctc 1020
   tctatgttat cccgaatgtc cagaactgca gtaaagacac ccgtttcacg tatggggatg 1080
   attcgtctca actccactca cgcttacgtg gaggacaagt cgcagggacc aatgcttcga 1140
   taccagaact cgctccctcg actgcccgtg cctagtctgg aggagaccgc tgcgcgatac 1200
   ctccgatctg tcaaggccgt ggctacttcg gaccagtacg ctcgaaccga gcaggcggtc 1260
   aagaagtttc ttcagtctga gggccctgct ctccagaagc agctcgaggc ccgtgccgcc 1320
   gaccctaaca tgaagaactg gctggctgag tggtggaaca acggagccta ccttgatgtg 1380
   cgcgaccctg tggtgcccta cgtgtcgtac ttttactccc acaaggacga caagctccga 1440
   aagaagcctg ctgcccgagc tgcttccctg acctcggtgg ccctgacctt caaggaccag 1500
   gttgacaaca agactctgga gcctgagtac atgaagaaga accccatctg catggacgcc 1560
   tacaagtaca tgttcaacac ttgccgtatc cccgtccagg gcggtaccga ctaccctgaa 1620
   acttactcgg ccaaggacaa ccagtacatt gtggccatcc gcaagaaccg attcttcaag 1680
   atttaccacg atgtcaatgg ccagcgattg tctgctggtg acctggagaa gcagtttgag 1740
   caggtttacc agctcgctga cacccgtggt cctgccattg gtgccctgac ctcctggaac 1800
   cgtgacaagt ggggtgctgc ccgcaccgag ctgctgtccg ccggcaacaa ggaggctctt 1860
   aaggccattg aggccgcctc tttcgtggtg tgcctggacg acgctgctcc tgtcactcgt 1920
   gaggagcgcg ctcaccagta ctggcacgga gacggaaaga accgattcta cgacaagcca 1980
   gttcagttta ttgtcaacaa caacggtacc tctggtttca tgggagagca ctccatgatg 2040
   gacggaaccc agactcaccg tctcaacgac tacactcttg atgccatctt caacaagaag 2100
   gttgagattt ctaacaagga gactggctcg tacggtgctc ccgaggagat caagttcaac 2160
   gtcaacggcc gaattgaggc tctcattgac gaggctcttg ccgactttga tgccgagatt 2220
   ggcgcccacg agctggcagt gttcaactac cagggatacg gaaagggact catgaagaag 2280
   ttcaagtgct ctcctgatgc ctacctgcag atgatcattc agctcgctta ctacaagatg 2340
   ttcggcgtgt ctcgacctac ctatgagtct gctgccaccc gacgatttgc tctgggacga 2400
   actgagacct gccgatcggt gtctctcgag tcgattgact ttgtcaagac tatggaggac 2460
   cctactgctt ccgccaagga gaaggttgag gctgcccgaa aggccattgc cgctcacgga 2520
   aagactatca atgatgcctc tgccggactg ggagtcgacc gtcacttgtt tggtctcaag 2580
   aagtttgtca aggctcagga tgctcttccc gagctgtacc aggaccccat gtacagctac 2640
   tcttgctcgt ggtacctgtc tacttctcag ctctcttctg agtactacaa cggatacgga 2700
   tggtctcagg tgattgaccc cggatttgga tgcgcttaca tgattaacga gaacaacatt 2760
   aacattaaca ttgtttctaa gaagcaggga tctgagaagc tgcgattcta cctcaacgag 2820
   gccgctgatg acatggctgc agtcttttct tccgagcttt cggaggctcc caagctgtaa 2880
   actataggtt atagattatg aacataccct ttcacacata tttgattgtc gttatataat 2940
   tgtgactaca gagactgcaa tcatttattt cctgcttaca ggtggagtta gagtcgggtt 3000
   tggggcccaa agttgattgc aatctttcca acgtgctcct ttgactccaa cttttcaaat 3060
   gccttgaaaa caccaccatc gtgaaagttg tactggtggt caacgtgaat acggaggtcc 3120
   ttggagtcca caaatttgat caattgttca gacagctcgt gagatcctac cacaattcct 3180
   cgcacaatca gtcccttgaa cataatgttc aggtagtaag cagccaactg ctgttcggcc 3240
   ttgatagcat tcacaccaat gagggaaatg actcctccag gagcagcggc agtgatgctc 3300
   tggaacagag tgcctgggcc cccgttttca attacaaagt ccactccgcc accagtaatc 3360
   tctgccactg tagcacccca actggggttc tttgcatagt tgatagtatg gtcagggttg 3420
   taatggtcct tgatcttggc caacttttca tctgaagatg aagtgatgat agtttgagca 3480
   ccagcagcct ttgcgattgc cagagcagtg agcgacactc ctccagtgcc tagaatcaga 3540
   accgattgtc ccgggcgcaa ttgcttgcaa ccgtacagag cgttccatac tgtgactccg 3600
   gtacaaacaa gcgaagcctt ctcagcccac gacagaggag agtccttacg gatcttagtt 3660
   actgcctcgc tcgagatggg aacatattct ctcagtgttc cgtcaacctc cccgccgagg 3720
   tccacatgtt gctctctgtt gtagggcccg tacaggtggt tggggctaaa gttggtcaca 3780
   acatggtctc ccacgctgac attggtaaca tcgtcaccca cagcaaccac aataccagca 3840
   gcatcggagc atggaactcg tcccttaaga tcccccagct tcttaccgta cttt 3894
<210> 51
   <211> 3500
   <212> DNA
   <213> Artificial Sequence
<220>
   <221> source
   <222> 1..3500
   <223> /mol_type="DNA" /note="synthetic construct" /organism="Artificial Sequence"
<220>
   <221> gene
   <222> 1126..1842
   <223> /gene="reverse complementary TRP"
<400> 51
   gttgacaggt ttccagtcga atgacaattt ttaattggca aacctgaaat gacctctagg 60
   gtgcagtatg cggcagcacc tgcagtggag tccagcttgg ccaactcccg agataaccgt 120
   cgaggtaacg acccaaggta accggcaacc ccgctctctt acatgctaca aggtgaattt 180
   atacaattgg aacagttcca gctgctcatc aagcctagcc tggagcctgg agcctgatag 240
   cctggaagcc tgatcaacca ggtagtgccg ccgcactggg ttccggatga cgagaaagat 300
   gatttgacaa aggcaacgtg ggtcaatgga ccctgaaaga ccaacggcca acagattgca 360
   cagatcgtcg gggcaaatga cgtcgctaag cccagttccg accgctgttt actagtttaa 420
   ccttggttcc tgtcggcatt tggggtcccc ttttcacggt gcgtcgggca gttaccgagg 480
   ccccgaattg ccgaaaccga ggagacccat atgaagaacc gtcaaagcta gcgaatgctg 540
   ggcctggata ctccagatcc taggtttcca gcaatatatt ttttacttcc tacactaggt 600
   gacccccaaa ccttgtgcca tgccttcatt ggctcaaggc gctggtgata aattatcgtg 660
   cctatctttg tacgcacttc gcgtttgtcc gaggatgact cggggccact gtggggaagg 720
   cggtgaggct tgcaaggacc gctctaacgg agcagagatg aaagagagga aaagccagtc 780
   aattgagtcg gtttgagggc aatttaacgg ttttatcgtg tctatcaggg gtcaattgac 840
   ccaggtccac cctatggtgg tttaatttat tggtgcatca tgcgaaccgg gttaacggcc 900
   tcggtcggca ttgaccgaga ggtgtccgga tggcttgtgt gtgtccccac gatgtcgacc 960
   tgaattcgat cgagtctgta ttgaagctta gcttgtataa atagtaaatt gtcaatcacc 1020
   tcaagtttca aatgagaagt aatatagctg tattctgctg tcagacctat tagtacacat 1080
   tacctgtcgt cagtatcacc tattgatgct gctgctggag tgttaatact tcacagtaac 1140
   ccccttggca gctttgacaa accgctcaat ctcgttgaga tctttgaccc ccgggcttga 1200
   ttcgactcct ccgctgacat caacaccaca ggcaccctcg accgctagag cttgggcaac 1260
   attgtcggct gtcaatcctc cagccataag gaacctcgct cccattcgcg cccgagactg 1320
   gactccgctc cagtccacca tcttgccctc tccgccaacc tcaccgtcta tgagggatag 1380
   gctgtggtat ccagtaaccg gcgaggcata gaaatctgga gtatcggggg tgaaccgacg 1440
   gatcacagga catggaatca gtctacacca ttctaggggc tctgatccgt gaagctgaac 1500
   gaaatccagc gacaattcct gttgcaaccg caggacttct gacagcggct ggttgcggaa 1560
   cacgcctacg attgctggcc gggtccttgt cttggcttca atgatgtggg cgagtgcaga 1620
   gtgagtcgat ccgcccagct cattagtgtc cagctcaaag gatccttgac gtctatatga 1680
   tcgtaccagt cgtgatatct tgcctgctgt ctccatatca acagtgcgcg ctctattagg 1740
   gaccataatc attccaatca tgttggctcc agcatccaaa gcgacctggg ccgcctccac 1800
   ggtcctgatt ccgcacacct tgacgattgg cgacgttgtc attgtacagg tttttgaaag 1860
   ggtttgtgag tggagagtcc cctgggcatg tcgttcctta aaagagtcaa cccaaaatgc 1920
   cggtttcggc ccggggcagc cccacgataa agcattctgc gtatccttcc gagcgggggg 1980
   aggaaaggac agcatgtggt gaaaccgctg gggaacaagt tgacctatct gattagctga 2040
   gccagttgat gaacattctc cgattagtgg attgaagctt ggaaagggcg ccgaaagcaa 2100
   cagagctccg tcccatatcg acaggaggct gcagccgcat tcatgcatga aatttgccag 2160
   tacgaccgag ggaagaagcg attgtggtgc ttatcaaagt ctattatgtt tgcgcgaaga 2220
   ggagcaattg tcattgcagg cagtaactgc gagtgagatt cgcctgcgtt gtcccgttgc 2280
   caatggcttg ctaatggttg tcaatgactg accacaatta cgaggtagcc gctcgatgcg 2340
   gcccttgtct gataagatgt tcagtcttaa gagcatgttc tctctttgtc aattgcaatt 2400
   cgtcgattga aagcgctaag gggtttcctc acaattaata gggactgtat ctagacacga 2460
   tatcgaattc ctgcagcccg ggggatccac tagtcatacc ctttcacaca tatttgattg 2520
   tcgttatata attgtgacta cagagactgc aatcatttat ttcctgctta caggtggagt 2580
   tagagtcggg tttggggccc aaagttgatt gcaatctttc caacgtgctc ctttgactcc 2640
   aacttttcaa atgccttgaa aacaccacca tcgtgaaagt tgtactggtg gtcaacgtga 2700
   atacggaggt ccttggagtc cacaaatttg atcaattgtt cagacagctc gtgagatcct 2760
   accacaattc ctcgcacaat cagtcccttg aacataatgt tcaggtagta agcagccaac 2820
   tgctgttcgg ccttgatagc attcacacca atgagggaaa tgactcctcc aggagcagcg 2880
   gcagtgatgc tctggaacag agtgcctggg cccccgtttt caattacaaa gtccactccg 2940
   ccaccagtaa tctctgccac tgtagcaccc caactggggt tctttgcata gttgatagta 3000
   tggtcagggt tgtaatggtc cttgatcttg gccaactttt catctgaaga tgaagtgatg 3060
   atagtttgag caccagcagc ctttgcgatt gccagagcag tgagcgacac tcctccagtg 3120
   cctagaatca gaaccgattg tcccgggcgc aattgcttgc aaccgtacag agcgttccat 3180
   actgtgactc cggtacaaac aagcgaagcc ttctcagccc acgacagagg agagtcctta 3240
   cggatcttag ttactgcctc gctcgagatg ggaacatatt ctctcagtgt tccgtcaacc 3300
   tccccgccga ggtccacatg ttgctctctg ttgtagggcc cgtacaggtg gttggggcta 3360
   aagttggtca caacatggtc tcccacgctg acattggtaa catcgtcacc cacagcaacc 3420
   acaataccag cagcatcgga gcatggaact cgtcccttaa gatcccccag cttcttaccg 3480
   tactttccgc ggcctgcagg 3500
<210> 52
   <211> 239
   <212> PRT
   <213> Blastobotrys adeninivorans
<220>
   <221> SOURCE
   <222> 1..239
   <223> /mol_type="protein" /note="TRP" /organism="Blastobotrys adeninivorans"
<400> 52
   Met Thr Thr Ser Pro Ile Val Lys Val Cys Gly Ile Arg Thr Val Glu
   1 5 10 15
   Ala Ala Gln Val Ala Leu Asp Ala Gly Ala Asn Met Ile Gly Met Ile
               20                  25                  30
   Met Val Pro Asn Arg Ala Arg Thr Val Asp Met Glu Thr Ala Gly Lys
           35                  40                  45
   Ile Ser Arg Leu Val Arg Ser Tyr Arg Arg Gln Gly Ser Phe Glu Leu
       50                  55                  60
   Asp Thr Asn Glu Leu Gly Gly Ser Thr His Ser Ala Leu Ala His Ile
   65                  70                  75                  80
   Ile Glu Ala Lys Thr Arg Thr Arg Pro Ala Ile Val Gly Val Phe Arg
                   85                  90                  95
   Asn Gln Pro Leu Ser Glu Val Leu Arg Leu Gln Gln Glu Leu Ser Leu
               100                 105                 110
   Asp Phe Val Gln Leu His Gly Ser Glu Pro Leu Glu Trp Cys Arg Leu
           115                 120                 125
   Ile Pro Cys Pro Val Ile Arg Arg Phe Thr Pro Asp Thr Pro Asp Phe
       130                 135                 140
   Tyr Ala Ser Pro Val Thr Gly Tyr His Ser Leu Ser Leu Ile Asp Gly
   145                 150                 155                 160
   Glu Val Gly Gly Glu Gly Lys Met Val Asp Trp Ser Gly Val Gln Ser
                   165                 170                 175
   Arg Ala Arg Met Gly Ala Arg Phe Leu Met Ala Gly Gly Leu Thr Ala
               180                 185                 190
   Asp Asn Val Ala Gln Ala Leu Ala Val Glu Gly Ala Cys Gly Val Asp
           195                 200                 205
   Val Ser Gly Gly Val Glu Ser Ser Pro Gly Val Lys Asp Leu Asn Glu
       210                 215                 220
   Ile Glu Arg Phe Val Lys Ala Ala Lys Gly Val Thr Val Lys Tyr
   225                 230                 235
<210> 53
   <211> 942
   <212> DNA
   <213> Blastobotrys adeninivorans
<220>
   <221> source
   <222> 1..942
   <223> /mol_type="DNA" /note="carnitine-acylcarnitine permease" /organism="Blastobotrys adeninivorans"
<220>
   <221> CDS
   <222> 1..942
   <223> /gene="carnitine-acylcarnitine permease" /transl_table=1
<400> 53
   atgtcagaag acgccaggga tgcgctgagg actctgtccc tggctcctca gtccagaaat      60
   gatcggatcc ctcagtggct gctgccatat cgtcaaccgt tgtgcgcata cgcagcgtca     120
   atgtgggcta ttggagtagg attccctctt gattccataa agacccgcca acaaactcac     180
   aagtaccgct cggtgtggaa ctgtgtggtc gaaactcacc gtaccgaggg cattcggggg     240
   ttctaccgag gcattgtcgc tccactggtg tcgtcgtcgg tggtgagagc tctgtcagtg     300
   tcggtgtaca actacgcgct gccctatact tccaaactag ccttctcggt gtacgcaccg     360
   ccaaaggatc acggccattc ccctgatgct cttgtgcgaa gaatgcccgt ggctttcctg     420
   gcaggggtgt gcgccggtac ctcgtgcaca atgttgggat gtccgtttga atttacaaag     480
   ctagcgtccc agattgacgg gctggttcgt cagcagcctc ctcagtcaac attccaggta     540
   gccaagaagc tggtcaagct ccaaggagtc aaagttctgt actcagggtg gaaataccac     600
   atgctgcgag acgccctggg atcaggaatt tactttaccg tttatgaaac tataaagtct     660
   gcatttgggg acgtgctggg cccaggagct atcgccgctg gaggagcgtt ggcagggtct     720
   tgttcatgga ttctcattta cccattagat acctacaaga gcatgatcca gagagatttg     780
   tatgcgcggg cggtaggggc agacattgtg caagctcgca agcggtccct gctgcaaatg     840
   tattctagac gaatgtaccg tggtctgtca gtgtcccttg tcaggaccag cgtgatggga     900
   atgatgttct ttacatctta tgaatggtta atcaagcacg tt                        942
<210> 54
   <211> 314
   <212> PRT
   <213> Blastobotrys adeninivorans
<220>
   <221> SOURCE
   <222> 1..314
   <223> /mol_type="protein" /note="[CDS]:1..942 from SEQ ID NO 53" /note="This sequence is generated from feature [CDS]:1..942 place d under sequence number 53" /organism="Blastobotrys adeninivorans"
<400> 54
   Met Ser Glu Asp Ala Arg Asp Ala Leu Arg Thr Leu Ser Leu Ala Pro
   1               5                   10                  15
   Gln Ser Arg Asn Asp Arg Ile Pro Gln Trp Leu Leu Pro Tyr Arg Gln
               20                  25                  30
   Pro Leu Cys Ala Tyr Ala Ala Ser Met Trp Ala Ile Gly Val Gly Phe
           35                  40                  45
   Pro Leu Asp Ser Ile Lys Thr Arg Gln Gln Thr His Lys Tyr Arg Ser
       50                  55                  60
   Val Trp Asn Cys Val Val Glu Thr His Arg Thr Glu Gly Ile Arg Gly
   65                  70                  75                  80
   Phe Tyr Arg Gly Ile Val Ala Pro Leu Val Ser Ser Ser Val Val Arg
                   85                  90                  95
   Ala Leu Ser Val Ser Val Tyr Asn Tyr Ala Leu Pro Tyr Thr Ser Lys
               100                 105                 110
   Leu Ala Phe Ser Val Tyr Ala Pro Pro Lys Asp His Gly His Ser Pro
           115                 120                 125
   Asp Ala Leu Val Arg Arg Met Pro Val Ala Phe Leu Ala Gly Val Cys
       130                 135                 140
   Ala Gly Thr Ser Cys Thr Met Leu Gly Cys Pro Phe Glu Phe Thr Lys
   145                 150                 155                 160
   Leu Ala Ser Gln Ile Asp Gly Leu Val Arg Gln Gln Pro Pro Gln Ser
                   165                 170                 175
   Thr Phe Gln Val Ala Lys Lys Leu Val Lys Leu Gln Gly Val Lys Val
               180                 185                 190
   Leu Tyr Ser Gly Trp Lys Tyr His Met Leu Arg Asp Ala Leu Gly Ser
           195                 200                 205
   Gly Ile Tyr Phe Thr Val Tyr Glu Thr Ile Lys Ser Ala Phe Gly Asp
       210                 215                 220
   Val Leu Gly Pro Gly Ala Ile Ala Ala Gly Gly Ala Leu Ala Gly Ser
   225                 230                 235                 240
   Cys Ser Trp Ile Leu Ile Tyr Pro Leu Asp Thr Tyr Lys Ser Met Ile
                   245                 250                 255
   Gln Arg Asp Leu Tyr Ala Arg Ala Val Gly Ala Asp Ile Val Gln Ala
               260                 265                 270
   Arg Lys Arg Ser Leu Leu Gln Met Tyr Ser Arg Arg Met Tyr Arg Gly
           275                 280                 285
   Leu Ser Val Ser Leu Val Arg Thr Ser Val Met Gly Met Met Phe Phe
       290                 295                 300
   Thr Ser Tyr Glu Trp Leu Ile Lys His Val
   305                 310
<210> 55
   <211> 2693
   <212> DNA
   <213> Artificial Sequence
<220>
   <221> source
   <222> 1..2693
   <223> /mol_type="DNA" /note="synthetic construct" /organism="Artificial Sequence"
<220>
   <221> gene
   <222> 971..1912
   <223> /gene="carnitine-acylcarnintine permease"
<400> 55
   tgcttgaagt gaaggactgc gggtttgggc ttgcgtcgga cgagggagac gaggacgatg      60
   agcaggagga gataatttca aattctcctc aactcatgca atggctgacg atgattgtgg     120
   ggtgtggatt agactggatt aaggatcccg atgctcagga tgaaattata tcaatggcta     180
   gcatgcggat tgctgaacgg tgcggacgga atgcccaacc cagtaaagtt cgggagattg     240
   tactggaaaa gtctatggag cccattaaaa tccacgagcc ttcgttggtt agtgataatt     300
   taggactaaa aacatggggt tcggcatatg taatttcaag gagactgctt cgccagcccg     360
   acatgctcaa aggacgagtt ttagagctcg ggtcaggcac agggttgttt ggaatcacgg     420
   cagcaaagct aggatcaaag attgttctga ccgatctaga ggacattgta cccaacctgg     480
   agcgcaacat tgaagaaaac aattgcgagg acaacgcatc agtagctacg ctggactgga     540
   tgcatcctga agattttggt gagcaaaagt ttgacaccgt agttgtggca gaccctattt     600
   attcttccga gcatcccaaa atggtgttca acgtcattga aacttttttg actaaaactg     660
   gccgtctgat tgtccaacta ccgttaagag ccaattttga agacttgcga caagaatttt     720
   actctctatt ggacaatgca cactaccacc gaatccaatt cgacgtggaa gagggacggg     780
   atgaatttgg caccatcaaa tttgaattct caatgtacac tgcatcacac taactttggt     840
   tatagactaa catcaatcta ttacagaaac tcatccatac ctccatatcc atgtatttac      900
   agaagctaat gtatttacaa aagttctatt cgttagtatc taatgtacag caataactcc      960
   tccaatccct aaacgtgctt gattaaccat tcataagatg taaagaacat cattcccatc     1020
   acgctggtcc tgacaaggga cactgacaga ccacggtaca ttcgtctaga atacatttgc     1080
   agcagggacc gcttgcgagc ttgcacaatg tctgccccta ccgcccgcgc atacaaatct     1140
   ctctggatca tgctcttgta ggtatctaat gggtaaatga gaatccatga acaagaccct     1200
   gccaacgctc ctccagcggc gatagctcct gggcccagca cgtccccaaa tgcagacttt     1260
   atagtttcat aaacggtaaa gtaaattcct gatcccaggg cgtctcgcag catgtggtat     1320
   ttccaccctg agtacagaac tttgactcct tggagcttga ccagcttctt ggctacctgg     1380
   aatgttgact gaggaggctg ctgacgaacc agcccgtcaa tctgggacgc tagctttgta     1440
   aattcaaacg gacatcccaa cattgtgcac gaggtaccgg cgcacacccc tgccaggaaa     1500
   gccacgggca ttcttcgcac aagagcatca ggggaatggc cgtgatcctt tggcggtgcg     1560
   tacaccgaga aggctagttt ggaagtatag ggcagcgcgt agttgtacac cgacactgac     1620
   agagctctca ccaccgacga cgacaccagt ggagcgacaa tgcctcggta gaacccccga     1680
   atgccctcgg tacggtgagt ttcgaccaca cagttccaca ccgagcggta cttgtgagtt     1740
   tgttggcggg tctttatgga atcaagaggg aatcctactc caatagccca cattgacgct     1800
   gcgtatgcgc acaacggttg acgatatggc agcagccact gagggatccg atcatttctg     1860
   gactgaggag ccagggacag agtcctcagc gcatccctgg cgtcttctga catgtcacga     1920
   cgaagctact cacagtgaac aaggttggct cacggggatg acgcagatca aggtaaatca     1980
   gcaggttgaa cctcgggcac acggacacac ggacacacgg accgagtctt cgcggcgctc     2040
   cgagtatgcg taggtagaga ggctgttgtg actcaaccgt aacttctcga atctccaatc     2100
   catactacat gatatacaga tattaatggc gtaggactag accagtactg agatgtaact     2160
   gaaatacagc ttaaactgaa atgaatgatg gaatggtatg aaatggtatg aaatggtatg     2220
   aaatagtatg ggctagggag tacccctgag aacatggtgc ttaagcatat tcgaactatt    2280
   tccaaatgct cacgaccccc atagggttac aggaaagcca gattctttta tttgagatga     2340
   acaatacttg acctaactga agcaattgac aaggaattca catcttggga tgaacttcta     2400
   aactccaagc gaaactgggg tttgtatgat tgttaccaat tgatcccaca gatgatctag     2460
   tttctataga ctcaggattt gttatattcc aaactacaga tccaaaatat gtgcgcattc     2520
   atcggtttgg cggctaggtc tggacgttcc cagagcctct tcatggatgc ctgtaccaac     2580
   gttcccagag cctcttgatg gatgcctgta ccaggagtga attcccctat gtttcgagtt     2640
   agagttctat catggcccgg agcaatccca gtgatactag taatagtgca tag            2693
<210> 56
   <211> 3211
   <212> DNA
   <213> Artificial Sequence
<220>
   <221> source
   <222> 1..3211
   <223> /mol_type="DNA" /note="synthetic construct" /organism="Artificial Sequence"
<220>
   <221> gene
   <222> 1136..1852
   <223> /gene="reverse complementary TRP"
<400> 56
<210> 57
   <211> 239
   <212> PRT
   <213> Blastobotrys adeninivorans
<220>
   <221> SOURCE
   <222> 1..239
   <223> /mol_type="protein" /note="TRP" /organism="Blastobotrys adeninivorans"
<400> 57
   Met Thr Thr Ser Pro Ile Val Lys Val Cys Gly Ile Arg Thr Val Glu
   1               5                   10                  15
   Ala Ala Gln Val Ala Leu Asp Ala Gly Ala Asn Met Ile Gly Met Ile
               20                  25                  30
   Met Val Pro Asn Arg Ala Arg Thr Val Asp Met Glu Thr Ala Gly Lys
           35                  40                  45
   Ile Ser Arg Leu Val Arg Ser Tyr Arg Arg Gln Gly Ser Phe Glu Leu
       50                  55                  60
   Asp Thr Asn Glu Leu Gly Gly Ser Thr His Ser Ala Leu Ala His Ile
   65                  70                  75                  80
   Ile Glu Ala Lys Thr Arg Thr Arg Pro Ala Ile Val Gly Val Phe Arg
                   85                  90                  95
   Asn Gln Pro Leu Ser Glu Val Leu Arg Leu Gln Gln Glu Leu Ser Leu
               100                 105                 110
   Asp Phe Val Gln Leu His Gly Ser Glu Pro Leu Glu Trp Cys Arg Leu
           115                 120                 125
   Ile Pro Cys Pro Val Ile Arg Arg Phe Thr Pro Asp Thr Pro Asp Phe
       130                 135                 140
   Tyr Ala Ser Pro Val Thr Gly Tyr His Ser Leu Ser Leu Ile Asp Gly
   145                 150                 155                 160
   Glu Val Gly Gly Glu Gly Lys Met Val Asp Trp Ser Gly Val Gln Ser
                   165                 170                 175
   Arg Ala Arg Met Gly Ala Arg Phe Leu Met Ala Gly Gly Leu Thr Ala
               180                 185                 190
   Asp Asn Val Ala Gln Ala Leu Ala Val Glu Gly Ala Cys Gly Val Asp
           195                 200                 205
   Val Ser Gly Gly Val Glu Ser Ser Pro Gly Val Lys Asp Leu Asn Glu
       210                 215                 220
   Ile Glu Arg Phe Val Lys Ala Ala Lys Gly Val Thr Val Lys Tyr
   225                 230                 235
<210> 58
   <211> 951
   <212> DNA
   <213> Blastobotrys adeninivorans
<220>
   <221> source
   <222> 1..951
   <223> /mol_type="DNA" /note="carnitine-acylcarnitine permease 2" /organism="Blastobotrys adeninivorans"
<220>
   <221> CDS
   <222> 1..951
   <223> /gene="carnitine-acylcarnitine permease 2" /transl-table=1
<400> 58
   atgaacaaaa tgtctgaact gagctctcca gaagacgtgg ctctgaaaga acctcactac      60
   catgagaagg atcagcctgg caagaccgag tcggccgtct ggggtcagac aaagtcgttc     120
   ctggctggag gattcggagg agtgtgcgcg gtgctagtcg gtcacccttt tgatttggtc     180
   aaggtgcgtc tgcagacttc gcagcctggt cagtacggtg gtactctgga cgtggtgaag     240
   aagactctgg ctgctgacgg cttccggggt ctgtaccgag gagttgtcgc tcctttgtac     300
   ggagttactc ctatgtttgc catttccttc tggggatacg acctgggtca gcgaattgtg     360
   acttcggtga ccaagcctga gggctctctg tcaatcggcc agatttctgc tgctggattc     420
   ttgtccgcca tccctaccac tggagtggcc gctccctttg agcgtgtcaa ggtcattctg     480
   cagctccagg gtcagggcaa gacccagggc aagcagttta acggtgctct ggacgtggtc     540
   aagcaccttt acagggaggg aggactgcga tccgtgttca agggatcggt ggctaccctg     600
   gcccgagatg ggcccggatc tgccctttac tttgctactt acgagtacac caaacgacga     660
   cttactcccg agggccagac tatgtctctt ggagccatca ctttggccgg aggtctggcc     720
   ggtaccgcaa tgtggattcc cgtgttcccc attgacacta tcaaatctaa ccttcagtcg     780
   tctgacaagc ctcagtccct tagtgccgtt accaagggaa tctaccgcaa cggaggattc     840
   aaggccttct tccccggtct gggacctgct ctgctgcgat cgttccctgc taatgccgcc     900
   acttttgtgg gagtggaact tgcacactct ttcttccgat ctttcaacct c              951
<210> 59
   <211> 317
   <212> PRT
   <213> Blastobotrys adeninivorans
<220>
   <221> SOURCE
   <222> 1..317
   <223> /mol_type="protein" /note="This sequence is generated from feature [CDS]:1..951 place d under sequence number 58" /note="[CDS]:1..951 from SEQ ID NO 58" /organism="Blastobotrys adeninivorans"
<400> 59
   Met Asn Lys Met Ser Glu Leu Ser Ser Pro Glu Asp Val Ala Leu Lys
   1 5 10 15
   Glu Pro His Tyr His Glu Lys Asp Gln Pro Gly Lys Thr Glu Ser Ala
   20 25 30
   Val Trp Gly Gln Thr Lys Ser Phe Leu Ala Gly Gly Phe Gly Gly Val
   35 40 45
   Cys Ala Val Leu Val Gly His Pro Phe Asp Leu Val Lys Val Arg Leu
   50 55 60
   Gln Thr Ser Gln Pro Gly Gln Tyr Gly Gly Thr Leu Asp Val Val Lys
   65 70 75 80
   Lys Thr Leu Ala Ala Asp Gly Phe Arg Gly Leu Tyr Arg Gly Val Val
   85 90 95
   Ala Pro Leu Tyr Gly Val Thr Pro Met Phe Ala Ile Ser Phe Trp Gly
   100 105 110
   Tyr Asp Leu Gly Gln Arg Ile Val Thr Ser Val Thr Lys Pro Glu Gly
   115 120 125
   Ser Leu Ser Ile Gly Gln Ile Ser Ala Ala Gly Phe Leu Ser Ala Ile
   130 135 140
   Pro Thr Thr Gly Val Ala Ala Pro Phe Glu Arg Val Lys Val Ile Leu
   145 150 155 160
   Gln Leu Gln Gly Gln Gly Lys Thr Gln Gly Lys Gln Phe Asn Gly Ala
   165 170 175
   Leu Asp Val Val Lys His Leu Tyr Arg Glu Gly Gly Leu Arg Ser Val
   180 185 190
   Phe Lys Gly Ser Val Ala Thr Leu Ala Arg Asp Gly Pro Gly Ser Ala
   195 200 205
   Leu Tyr Phe Ala Thr Tyr Glu Tyr Thr Lys Arg Arg Leu Thr Pro Glu
   210 215 220
   Gly Gln Thr Met Ser Leu Gly Ala Ile Thr Leu Ala Gly Gly Leu Ala
   225 230 235 240
   Gly Thr Ala Met Trp Ile Pro Val Phe Pro Ile Asp Thr Ile Lys Ser
   245 250 255
   Asn Leu Gln Ser Ser Asp Lys Pro Gln Ser Leu Ser Ala Val Thr Lys
   260 265 270
   Gly Ile Tyr Arg Asn Gly Gly Phe Lys Ala Phe Phe Pro Gly Leu Gly
   275 280 285
   Pro Ala Leu Leu Arg Ser Phe Pro Ala Asn Ala Ala Thr Phe Val Gly
   290 295 300
   Val Glu Leu Ala His Ser Phe Phe Arg Ser Phe Asn Leu
   305 310 315
<210> 60
   <211> 3914
   <212> DNA
   <213> Blastobotrys adeninivorans
<220>
   <221> source
   <222> 1..3914
   <223> /mol_type="DNA" /note="synthetic construct" /organism="Blastobotrys adeninivorans"
<220>
   <221> gene
   <222> 1665..2615
   <223> /gene="carnintine-acylcarnitine permease"
<400> 60
   aacctggaga ctcagactcg cggtcccaag gcgagctgac gctgcagaga agcctggtat 60
   tattgcagga ggcaactcta ggattacgac tgttgttctc catttctgaa gcgtcaaagg 120
   gcttaaaagg cggtcagatt atatcagtag tacgggaatt ctgcaacaat ggcgcggaat 180
   ttgtagccaa gtttgcaaag cggttgttgg aaaaggtatc atacccattc tacaatattc 240
   tcaatgggtg gatggctcgg ggaatactaa cggaccctca tcaagagttc tttgttcaga 300
   tcaaggatgc tggcagccct tggagtggac gattcacgtt tgaacaagaa ctggttccgt 360
   caatcttgac acccgaaata gcaaagtcgg tgttcgacac gggcaaaacc ttgtactttg 420
   tgcgagaagt gtgtaatgag catgcatggg tggacgaacg agaaaatgcg tccattgtaa 480
   actatgagga tttggactac cgggtagccc aaaactacaa ggaagtggta aagtatttaa 540
   acagtattct cgttgagaga tttcatatgg accttcacct tcaagcgctc aaggactatt 600
   tattgcttgg aaaaggtgat tttgtccagg ttcttgtaga gattgctgct cctgtgctca 660
   gcaagcctgc atccaagtta ttgagacatc acctaacttc tattctggag actgccctcc 720
   gtgggtcttc tgctcagacc gatagcccgg aagttttaaa atatctagac gcccgaatgc 780
   ttgaactggg ccatggagac attggatggg acattttcac cctagattac agagtggaga 840
   gccctctaga cattgttatc ctggacagag ccgctgctac ggaatacctg cgagtattca 900
   actttttgtg gaggatcaag cgcatttcgt acgagtttaa tactggctgg cgtcgactgg 960
   caactgcgta tcgagcgttt ttatcaaagt tcaacactcg aagggccgaa tgggacctgg 1020
   cccggtccac atatcaggag atgattcact ttgtaggaga gctacagtac tatgtttcgt 1080
   acgaagtgat tgaagtgtca tgggccaact tgaagcatca gctgtcacaa gggggaggac 1140
   aattgactgt ggatgatatc atttcagcac accgaaagta tctatccgaa atcacacaaa 1200
   agggcctttt aggtggtggt gcacttatgg gggagcttca tgagacactc aaagtggccc 1260
   tagcattccg agagacaatt gatgccctgt atgaattgtc agtgagaatg catgccctag 1320
   gtacagcacg accatccgat gaagaacgat ttgactctgt gcacactcgc attcaaaacc 1380
   tcagggccat gtttgagaag agcgtgcagc gactggcgta taacctgacc aagctggaca 1440
   atggggagat gagattcttg ggagtgcgac tagactttaa cgggttctat tcccgggccc 1500
   gcgtaaagga acgagagaaa cgagaagcta atgaataatg actataacat ttaatggata 1560
   acatctaatg aatcgtagcg atgaattgta atactactca tagtaatgta atcatctatc 1620
   atcatgcaca ttgatcatat atacctactt tggttgatgg gttagaggtt gaaagatcgg 1680
   aagaaagagt gtgcaagttc cactcccaca aaagtggcgg cattagcagg gaacgatcgc 1740
   agcagagcag gtcccagacc ggggaagaag gccttgaatc ctccgttgcg gtagattccc 1800
   ttggtaacgg cactaaggga ctgaggcttg tcagacgact gaaggttaga tttgatagtg 1860
   tcaatgggga acacgggaat ccacattgcg gtaccggcca gacctccggc caaagtgatg 1920
   gctccaagag acatagtctg gccctcggga gtaagtcgtc gtttggtgta ctcgtaagta 1980
   gcaaagtaaa gggcagatcc gggcccatct cgggccaggg tagccaccga tcccttgaac 2040
   acggatcgca gtcctccctc cctgtaaagg tgcttgacca cgtccagagc accgttaaac 2100
   tgcttgccct gggtcttgcc ctgaccctgg agctgcagaa tgaccttgac acgctcaaag 2160
   ggagcggcca ctccagtggt agggatggcg gacaagaatc cagcagcaga aatctggccg 2220
   attgacagag agccctcagg cttggtcacc gaagtcacaa ttcgctgacc caggtcgtat 2280
   ccccagaagg aaatggcaaa cataggagta actccgtaca aaggagcgac aactcctcgg 2340
   tacagacccc ggaagccgtc agcagccaga gtcttcttca ccacgtccag agtaccaccg 2400
   tactgaccag gctgcgaagt ctgcagacgc accttgacca aatcaaaagg gtgaccgact 2460
   agcaccgcgc acactcctcc gaatcctcca gccaggaacg actttgtctg accccagacg 2520
   gccgactcgg tcttgccagg ctgatccttc tcatggtagt gaggttcttt cagagccacg 2580
   tcttctggag agctcagttc agacattttg ttcatatagt gaccacaaac caatccggac 2640
   ctaccccggg ctttattagt tttcaatccc cggttcgagc cgtacgaccg agagaacccc 2700
   gcctggcctc ggtcgagcgg ctggtgggac agaccgaaat catgacgagc ccagtgaggc 2760
   cgagatgccg agggccaatg gaatgcaaaa tttgctcagg gcccaacgtg attcgccaag 2820
   gagtggattt tggccagctt gtactgataa caagaaggat cattatgaga tgggtttgga 2880
   tggattagat gatgagctcg gggtaaccct gtcgatgcgt gctgcgacca gagttagtgg 2940
   cctcccactt accgcctttt gcggagaaac ccggagaccc tgattcactt atcagatcgt 3000
   cgatagattc acggaaacat gggcatcggc aagtcctcgg gacgtatgtg catcattatg 3060
   cttctgatgt tcactcgatt gaatttcggc agtactgaag ggtacatagt cggagtagat 3120
   agtcggagta gataaggtag tagatagggg tagtagggca gggtagatgg ggtaaggaag 3180
   acgatgtagg gtagagtgtg tatgcctctg ttcaagactg ttggcagtga atgtacctgc 3240
   accggtacta atggcccgtc aagtttcgtt gagggtatca cccattacag aagtgctgtt 3300
   atcaccattg aggggtcaat cagcattcca tttgactcaa ttttcactcc agttacggca 3360
   taccttatat gccatctcaa ttgcacatag cgacatccac catgggctct ataaccggat 3420
   cagtgaaaat tacagtccca tggtgaaagg cctcagggcg ataatgagcc cgtaatgatt 3480
   ccaagccaat tgatgcccta tcatcttcca tctaactatc cattgatata tcgtaaccag 3540
   cccattcatc cgctactcgt ctgccttcca tcttcatctg cggcattgat caatgacgcc 3600
   gataaccagc caattgacca ttactatcac ctgtctccca cctctatccc gtgtacctac 3660
   tactccttaa ccaccatggg ccgactacct cccaccgact cctatctggc tcacggattg 3720
   ctcctcctaa ccaccgcatt gacctctacc tctacttgtc ttaccactcc gttaccccct 3780
   ttccctgtcg ttctcttcgt aacgttcatg gtcatttaag ttgccgatct acaccgttcc 3840
   tggcccgccg cacatgtcat ctacccgttg ggcccggtgc cccttgctgg cgccactgct 3900
   cgtgccacca ttgt 3914
<210> 61
   <211> 3411
   <212> DNA
   <213> Artificial Sequence
<220>
   <221> source
   <222> 1..3411
   <223> /mol_type="DNA" /note="synthetic construct" /organism="Artificial Sequence"
<220>
   <221> CDS
   <222> 1524..2246
   <223> /gene="TRP" /transl_table=11
<400> 61
   aacctggaga ctcagactcg cggtcccaag gcgagctgac gctgcagaga agcctggtat 60
   tattgcagga ggcaactcta ggattacgac tgttgttctc catttctgaa gcgtcaaagg 120
   gcttaaaagg cggtcagatt atatcagtag tacgggaatt ctgcaacaat ggcgcggaat 180
   ttgtagccaa gtttgcaaag cggttgttgg aaaaggtatc atacccattc tacaatattc 240
   tcaatgggtg gatggctcgg ggaatactaa cggaccctca tcaagagttc tttgttcaga 300
   tcaaggatgc tggcagccct tggagtggac gattcacgtt tgaacaagaa ctggttccgt 360
   caatcttgac acccgaaata gcaaagtcgg tgttcgacac gggcaaaacc ttgtactttg 420
   tgcgagaagt gtgtaatgag catgcatggg tggacgaacg agaaaatgcg tccattgtaa 480
   actatgagga tttggactac cgggtagccc aaaactacaa ggaagtggta aagtatttaa 540
   acagtattct cgttgagaga tttcatatgg accttcacct tcaagcgctc aaggactatt 600
   tattgcttgg aaaaggtgat tttgtccagg ttcttgtaga gattgctgct cctgtgctca 660
   gcaagcctgc atccaagtta ttgagacatc acctaacttc tattctggag actgccctcc 720
   gtgggtcttc tgctcagacc gatagcccgg aagttttaaa atatctagac gcccgaatgc 780
   ttgaactggg ccatggagac attggatggg acattttcac cctagattac agagtggaga 840
   gccctctaga cattgttatc ctggacagag ccgctgcact agtggatccc ccgggctgca 900
   ggaattcgat atcgtgtcta gatacagtcc ctattaattg tgaggaaacc ccttagcgct 960
   ttcaatcgac gaattgcaat tgacaaagag agaacatgct cttaagactg aacatcttat 1020
   cagacaaggg ccgcatcgag cggctacctc gtaattgtgg tcagtcattg acaaccatta 1080
   gcaagccatt ggcaacggga caacgcaggc gaatctcact cgcagttact gcctgcaatg 1140
   acaattgctc ctcttcgcgc aaacataata gactttgata agcaccacaa tcgcttcttc 1200
   cctcggtcgt actggcaaat ttcatgcatg aatgcggctg cagcctcctg tcgatatggg 1260
   acggagctct gttgctttcg gcgccctttc caagcttcaa tccactaatc ggagaatgtt 1320
   catcaactgg ctcagctaat cagataggtc aacttgttcc ccagcggttt caccacatgc 1380
   tgtcctttcc tccccccgct cggaaggata cgcagaatgc tttatcgtgg ggctgccccg 1440
   ggccgaaacc ggcattttgg gttgactctt ttaaggaacg acatgcccag gggactctcc 1500
   actcacaaac cctttcaaaa acctgtacaa tgacaacgtc gccaatcgtc aaggtgtgcg 1560
   gaatcaggac cgtggaggcg gcccaggtcg ctttggatgc tggagccaac atgattggaa 1620
   tgattatggt ccctaataga gcgcgcactg ttgatatgga gacagcaggc aagatatcac 1680
   gactggtacg atcatataga cgtcaaggat cctttgagct ggacactaat gagctgggcg 1740
   gatcgactca ctctgcactc gcccacatca ttgaagccaa gacaaggacc cggccagcaa 1800
   tcgtaggcgt gttccgcaac cagccgctgt cagaagtcct gcggttgcaa caggaattgt 1860
   cgctggattt cgttcagctt cacggatcag agcccctaga atggtgtaga ctgattccat 1920
   gtcctgtgat ccgtcggttc acccccgata ctccagattt ctatgcctcg ccggttactg 1980
   gataccacag cctatccctc atagacggtg aggttggcgg agagggcaag atggtggact 2040
   ggagcggagt ccagtctcgg gcgcgaatgg gagcgaggtt ccttatggct ggaggattga 2100
   cagccgacaa tgttgcccaa gctctagcgg tcgagggtgc ctgtggtgtt gatgtcagcg 2160
   gaggagtcga atcaagcccg ggggtcaaag atctcaacga gattgagcgg tttgtcaaag 2220
   ctgccaaggg ggttactgtg aagtattaac actccagcag cagcatcaat aggtgatact 2280
   gacgacaggt aatgtgtact aataggtctg acagcagaat acagctatat tacttctcat 2340
   ttgaaacttg aggtgattga caatttacta tttatacaag ctaagcttca atacagactc 2400
   gatcgaattc aggtcgacgg attagatgat gagctcgggg taaccctgtc gatgcgtgct 2460
   gcgaccagag ttagtggcct cccacttacc gccttttgcg gagaaacccg gagaccctga 2520
   ttcacttatc agatcgtcga tagattcacg gaaacatggg catcggcaag tcctcgggac 2580
   gtatgtgcat cattatgctt ctgatgttca ctcgattgaa tttcggcagt actgaagggt 2640
   acatagtcgg agtagatagt cggagtagat aaggtagtag ataggggtag tagggcaggg 2700
   tagatggggt aaggaagacg atgtagggta gagtgtgtat gcctctgttc aagactgttg 2760
   gcagtgaatg tacctgcacc ggtactaatg gcccgtcaag tttcgttgag ggtatcaccc 2820
   attacagaag tgctgttatc accattgagg ggtcaatcag cattccattt gactcaattt 2880
   tcactccagt tacggcatac cttatatgcc atctcaattg cacatagcga catccaccat 2940
   gggctctata accggatcag tgaaaattac agtcccatgg tgaaaggcct cagggcgata 3000
   atgagcccgt aatgattcca agccaattga tgccctatca tcttccatct aactatccat 3060
   tgatatatcg taaccagccc attcatccgc tactcgtctg ccttccatct tcatctgcgg 3120
   cattgatcaa tgacgccgat aaccagccaa ttgaccatta ctatcacctg tctcccacct 3180
   ctatcccgtg tacctactac tccttaacca ccatgggccg actacctccc accgactcct 3240
   atctggctca cggattgctc ctcctaacca ccgcattgac ctctacctct acttgtctta     3300
   ccactccgtt accccctttc cctgtcgttc tcttcgtaac gttcatggtc atttaagttg      3360
   ccgatctaca ccgttcctgg cccgccgcac atgtcatcta cccgttgggc c              3411
<210> 62
   <211> 241
   <212> PRT
   <213> Artificial Sequence
<220>
   <221> SOURCE
   <222> 1..241
   <223> /mol_type="protein" /note="[CDS]:1524..2246 from SEQ ID NO 61" /note="This sequence is generated from feature [CDS]:1530..2246 p laced under sequence number 61" /note="[CDS]:1530..2246 from SEQ ID NO 61" /organism="Artificial Sequence"
<400> 62
   Cys Thr Met Thr Thr Ser Pro Ile Val Lys Val Cys Gly Ile Arg Thr
   1               5                   10                  15
   Val Glu Ala Ala Gln Val Ala Leu Asp Ala Gly Ala Asn Met Ile Gly
               20                  25                  30
   Met Ile Met Val Pro Asn Arg Ala Arg Thr Val Asp Met Glu Thr Ala
           35                  40                  45
   Gly Lys Ile Ser Arg Leu Val Arg Ser Tyr Arg Arg Gln Gly Ser Phe
       50                  55                  60
   Glu Leu Asp Thr Asn Glu Leu Gly Gly Ser Thr His Ser Ala Leu Ala
   65                  70                  75                  80
   His Ile Ile Glu Ala Lys Thr Arg Thr Arg Pro Ala Ile Val Gly Val
                   85                  90                  95
   Phe Arg Asn Gln Pro Leu Ser Glu Val Leu Arg Leu Gln Gln Glu Leu
               100                 105                 110
   Ser Leu Asp Phe Val Gln Leu His Gly Ser Glu Pro Leu Glu Trp Cys
           115                 120                 125
   Arg Leu Ile Pro Cys Pro Val Ile Arg Arg Phe Thr Pro Asp Thr Pro
       130                 135                 140
   Asp Phe Tyr Ala Ser Pro Val Thr Gly Tyr His Ser Leu Ser Leu Ile
   145                 150                 155                 160
   Asp Gly Glu Val Gly Gly Glu Gly Lys Met Val Asp Trp Ser Gly Val
                   165                 170                 175
   Gln Ser Arg Ala Arg Met Gly Ala Arg Phe Leu Met Ala Gly Gly Leu
               180                 185                 190
   Thr Ala Asp Asn Val Ala Gln Ala Leu Ala Val Glu Gly Ala Cys Gly
           195                 200                 205
   Val Asp Val Ser Gly Gly Val Glu Ser Ser Pro Gly Val Lys Asp Leu
       210                 215                 220
   Asn Glu Ile Glu Arg Phe Val Lys Ala Ala Lys Gly Val Thr Val Lys
   225                 230                 235                 240
   Tyr